(19) Europäisches Patentamt
European Patent Office
Office européen des brevets

(11) EP 4 410 788 B1

(12) EUROPEAN PATENT SPECIFICATION

(45) Date of publication and mention of the grant of the patent:
**04.03.2026 Bulletin 2026/10**

(21) Application number: **22875040.2**

(22) Date of filing: **29.09.2022**

(51) International Patent Classification (IPC):
***C07D 403/12*** (2006.01) ***C07D 403/14*** (2006.01)
***C07D 207/26*** (2006.01) ***C07D 307/02*** (2006.01)
***C07D 309/02*** (2006.01) ***C07D 335/02*** (2006.01)
***A61K 31/38*** (2006.01) ***A61P 31/14*** (2006.01)
***A61P 31/16*** (2006.01) ***A61P 31/18*** (2006.01)

(52) Cooperative Patent Classification (CPC):
(C-Sets available)
**A61K 45/06; A61K 31/40; A61K 31/427; A61P 31/14; A61P 31/16; A61P 31/18; C07D 207/16; C07D 491/113; C07D 495/10**
(Cont.)

(86) International application number:
**PCT/CN2022/122384**

(87) International publication number:
**WO 2023/051657 (06.04.2023 Gazette 2023/14)**

# (54) CYANO COMPOUND, AND PREPARATION METHOD THEREFOR AND USE THEREOF

CYANOVERBINDUNG UND HERSTELLUNGSVERFAHREN DAFÜR UND VERWENDUNG DAVON

COMPOSÉ CYANO, SON PROCÉDÉ DE PRÉPARATION ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2021 CN 202111168232**
**15.08.2022 CN 202210973184**

(43) Date of publication of application:
**07.08.2024 Bulletin 2024/32**

(73) Proprietors:
- **Shanghai Institute of Materia Medica, Chinese Academy of Sciences**
  **Pudong, Shanghai 201203 (CN)**
- **Wuhan Institute Of Virology, Chinese Academy of Sciences**
  **Wuhan, Hubei 430071 (CN)**
- **Hainan Simcere Pharmaceutical Co., Ltd.**
  **Haikou, Hainan 570311 (CN)**

(72) Inventors:
- **JIANG, Xiangrui**
  **Shanghai 201203 (CN)**
- **XU, Yechun**
  **Shanghai 201203 (CN)**
- **ZHANG, Leike**
  **Wuhan, Hubei 430071 (CN)**
- **SU, Haixia**
  **Shanghai 201203 (CN)**
- **ZHANG, Qiumeng**
  **Shanghai 201203 (CN)**
- **ZHAO, Wenfeng**
  **Shanghai 201203 (CN)**
- **SHANG, Weijuan**
  **Wuhan, Hubei 430071 (CN)**
- **SHEN, Jingshan**
  **Shanghai 201203 (CN)**
- **XIAO, Gengfu**
  **Wuhan, Hubei 430071 (CN)**
- **JIANG, Hualiang**
  **Shanghai 201203 (CN)**

(74) Representative: **Novagraaf Group**
**Chemin de l'Echo 3**
**1213 Onex / Geneva (CH)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
**CN-A- 112 778 310** **CN-A- 113 181 339**
**US-B1- 11 123 329** **US-B1- 11 124 497**

(52) Cooperative Patent Classification (CPC): (Cont.)

C-Sets
**A61K 31/40, A61K 2300/00;**
**A61K 31/427, A61K 2300/00**

## Description

The present invention claims the right of priority of the following applications: patent application No. 202111168232.4 entitled "CYANO COMPOUNDS, AND A PREPARATION METHOD AND USE THEREOF" and submitted to the China National Intellectual Property Administration on Thursday, September 30, 2021 and China patent application No. 202210973184.4 entitled "CYANO COMPOUNDS, AND A PREPARATION METHOD AND USE THEREOF" and submitted to the China National Intellectual Property Administration on Monday, August 15, 2022.

## Technical Field

The present invention belongs to the field of medicinal chemistry and chemical synthesis, in particular, the invention relates to cyano compounds, a preparation method therefor and use thereof.

## Background Art

Coronaviruses are single-stranded positive-sense RNA viruses, and some coronaviruses can spread widely among people and cause severe symptoms. There are currently 7 known coronaviruses that can infect humans, namely HCoV-229E, HCoV-OC43, HCoV-NL63, HCoV-HKU1, SARS-CoV, MERS-CoV and SARS-CoV-2. Most of the functional proteins of coronaviruses are encoded by the ORF1ab gene, which is first translated into a polyprotein, and then cleaved into multiple active proteins by 3CL protease and PL protease. Therefore, inhibiting the activity of 3CL protease can effectively inhibit the replication of the viruses. 3CL proteases in different coronaviruses have a high degree of structural homology, so 3CL protease inhibitors have a broad-spectrum anti-coronavirus activity.

In addition to coronaviruses, 3CL protease also plays an important role in the hydrolysis of polyproteins encoded by picornaviruses, and 3CL protease inhibitors can effectively inhibit the replication of picornaviruses. Enterovirus 71 is a picornavirus, and is one of the common viruses that cause hand, foot and mouth disease, and may also cause meningitis, brainstem encephalitis, myocarditis and other diseases. In recent years, enterovirus 71 has repeatedly broken out in infants and young children, and there is still a lack of effective therapeutic drugs in clinical practice.

Therefore, there remains a need for compounds that can inhibit RNA viruses/picornaviruses including coronaviruses/enterovirus 71.

## Summary of the Invention

Based on the crystal structure of 3CL protease, the inventors rationally designed a class of cyano compounds, which can effectively inhibit coronaviruse and/or picornaviruse 3CL protease activity, effectively inhibit 3CL protease activity of various picornaviruses including enterovirus 71 *in vitro,* effectively inhibit the replication of picornaviruses at a cell level and can be used for preparing a medicament for a disease induced by coronaviruses and/or picornaviruses. The inventors have completed the present disclosure on the basis of this.

The primary object of the present invention is to provide a cyano compound represented by general formula I, a racemate, an enantiomer, a diastereoisomer and a pharmaceutically acceptable salt thereof.

The second object of the present invention is to provide a preparation method of such compounds.

The third object of the present invention is to provide a pharmaceutical composition comprising such compounds.

The fourth object of the present invention is the use of such compounds in the preparation of 3CL protease inhibitors.

The fifth object of the present invention is the use of such compounds in the preparation of a medicament for preventing or treating a disease induced by coronaviruses and/or picornaviruses.

In order to accomplish the above objects, the technical solution adopted in the present invention is that:
In one aspect, the present invention provides a cyano compound represented by general formula I, a racemate, an enantiomer, a diastereoisomer and a pharmaceutically acceptable salt thereof.

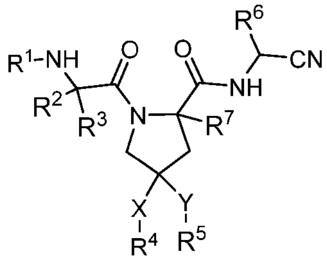

I

In another aspect, the present invention provides a method for preparing the compound of general formula I.

The technical solution of the present invention has at least the following technical effects:

The compound of the present invention has a 3CL protease inhibitory activity, can inhibit the hydrolysis of protein complexes expressed by genes of coronaviruses and picornaviruses, and then inhibits virus replication and development, and can be used for the prevention and treatment of diseases caused by coronavirus or picornavirus infection.

The present invention relates to a cyano compound represented by general formula I, a racemate, an enantiomer, a diastereoisomer or a pharmaceutically acceptable salt thereof:

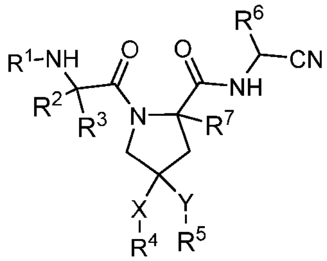

I

wherein

$R^1$ is selected from $-COR^8$ and $-SO_2R^9$;
$R^2$ and $R^3$ are each independently selected from H, D, $C_1$-$C_{10}$ alkyl, adamantyl and $C_3$-$C_7$ cycloalkyl, or, $R^2$ and $R^3$ and the carbon atom attached thereto together form a 3- to 8-membered carbocyclic ring;
X is selected from O, S, $S(=O)_2$ and S=O;
Y is absent or selected from O, S, $S(=O)_2$ and S=O;
$R^4$ is selected from H, $C_1$-$C_{10}$ alkyl, $C_3$-$C_8$ cycloalkyl, $C_6$-$C_{20}$ aryl, $C_1$-$C_{10}$ alkyl substituted $C_6$-$C_{20}$ aryl, $C_1$-$C_{10}$ alkoxy substituted $C_6$-$C_{20}$ aryl and halogenated $C_6$-$C_{20}$ aryl;
$R^5$ is selected from H, $C_1$-$C_{10}$ alkyl and $C_3$-$C_7$ cycloalkyl;
or, $R^4$ and $R^5$ are connected to each other to form $C_2$-$C_6$ alkylene, thereby connecting X and Y;
$R^6$ is selected from

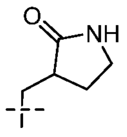 and 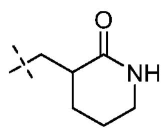 ;

$R^7$ is selected from H and D;
$R^8$ is selected from H, $C_1$-$C_{10}$ alkyl, $C_1$-$C_{10}$ alkoxy, $C_3$-$C_7$ cycloalkyl, halogenated $C_1$-$C_{10}$ alkyl, halogenated $C_3$-$C_7$ cycloalkyl, $-NR^{13}R^{14}$, $C_6$-$C_{20}$ aryl, halogenated $C_6$-$C_{20}$ aryl, $C_1$-$C_{10}$ alkyl substituted $C_6$-$C_{20}$ aryl, halogenated $C_1$-$C_{10}$ alkyl substituted $C_6$-$C_{20}$ aryl, 5- to 20-membered heteroaryl and halogenated 5- to 20-membered heteroaryl;
$R^9$ is selected from $C_1$-$C_{10}$ alkyl, $C_3$-$C_7$ cycloalkyl, halogenated $C_1$-$C_{10}$ alkyl, halogenated $C_3$-$C_7$ cycloalkyl, $-NR^{15}R^{16}$, $C_6$-$C_{20}$ aryl, halogenated $C_6$-$C_{20}$ aryl, $C_1$-$C_{10}$ alkyl substituted $C_6$-$C_{20}$ aryl, halogenated $C_1$-$C_{10}$ alkyl substituted $C_6$-$C_{20}$ aryl, 5- to 20-membered heteroaryl and halogenated 5- to 20-membered heteroaryl;
$R^{13}$ and $R^{14}$ are each independently selected from H and $C_1$-$C_{10}$ alkyl;
$R^{15}$ and $R^{16}$ are each independently selected from H and $C_1$-$C_{10}$ alkyl.

In some embodiments, $R^2$ and $R^3$ are each independently selected from H, D, $C_1$-$C_6$ alkyl, adamantyl and $C_3$-$C_7$ cycloalkyl, or, $R^2$ and $R^3$ and the carbon atom attached thereto together form a 3- to 8-membered carbocyclic ring.

In some embodiments, $R^2$ and $R^3$ are each independently selected from H, isopropyl, tert-butyl, cyclopentyl, and adamantyl, or, $R^2$ and $R^3$ and the carbon atom attached thereto together form cyclopropyl and cyclopentyl.

In some embodiments, one of $R^2$ and $R^3$ is selected from H, and the other is selected from isopropyl, tert-butyl, cyclopentyl, and adamantyl, or, $R^2$ and $R^3$ and the carbon atom attached thereto together form cyclopropyl and cyclopentyl.

In some embodiments, $R^4$ is selected from H, $C_1$-$C_6$ alkyl, $C_3$-$C_8$ cycloalkyl, $C_6$-$C_{10}$ aryl, $C_1$-$C_6$ alkyl substituted $C_6$-$C_{10}$ aryl, $C_1$-$C_6$ alkoxy substituted $C_6$-$C_{10}$ aryl and halogenated $C_6$-$C_{10}$ aryl;

$R^5$ is selected from H, $C_1$-$C_6$ alkyl and $C_3$-$C_7$ cycloalkyl;
or, $R^4$ and $R^5$ are connected to each other to form $C_2$-$C_6$ alkylene, thereby connecting X and Y.

In some embodiments, X is selected from O, S, $S(=O)_2$ and S=O; Y is absent or selected from O, S and S=O; $R^4$ is selected from $C_1$-$C_6$ alkyl and $C_6$-$C_{10}$ aryl, $R^5$ is selected from H; or, $R^4$ and $R^5$ are connected to each other to form $C_2$-$C_6$ alkylene, thereby connecting X and Y.

In some embodiments, $R^4$ and $R^5$ are connected to each other to form $CH_2CH_2$ and $CH_2CH_2CH_2$, thereby connecting X and Y.

In some embodiments, X and Y are each independently selected from O, S and S=O, and $R^4$ and $R^5$ are connected to each other to form $CH_2CH_2$ and $CH_2CH_2CH_2$, thereby connecting X and Y.

In some embodiments, X and Y are each independently selected from O and S, and $R^4$ and $R^5$ are connected to each other to form $CH_2CH_2$, thereby connecting X and Y.

In some embodiments, both X and Y are selected from S, or both X and Y are selected from O, and $R^4$ and $R^5$ are connected to each other to form $CH_2CH_2$, thereby connecting X and Y.

In some embodiments, X is selected from S, Y is absent, $R^4$ is selected from phenyl and isopropyl, and $R^5$ is selected from H.

In some embodiments, $R^6$ is selected from

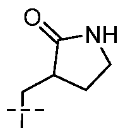

.

In some embodiments, $R^7$ is selected from H.

In some embodiments, $R^8$ is selected from H, $C_1$-$C_6$ alkyl, $C_1$-$C_6$ alkoxy, $C_3$-$C_7$ cycloalkyl, halogenated $C_1$-$C_6$ alkyl, halogenated $C_3$-$C_7$ cycloalkyl, $-NR^{13}R^{14}$, $C_6$-$C_{10}$ aryl, halogenated $C_6$-$C_{10}$ aryl, $C_1$-$C_6$ alkyl substituted $C_6$-$C_{10}$ aryl, halogenated $C_1$-$C_6$ alkyl substituted $C_6$-$C_{10}$ aryl, 5- to 10-membered heteroaryl and halogenated 5- to 10-membered heteroaryl.

In some embodiments, $R^8$ is selected from $C_1$-$C_6$ alkyl, halogenated $C_1$-$C_6$ alkyl, $C_1$-$C_6$ alkoxy, $-NR^{13}R^{14}$, $C_3$-$C_7$ cycloalkyl, halogenated $C_3$-$C_7$ cycloalkyl, phenyl, halophenyl, $C_1$-$C_6$ alkyl substituted phenyl, halogenated $C_1$-$C_6$ alkyl substituted phenyl and 5- to 6-membered heteroaryl.

In some embodiments, $R^9$ is selected from $C_1$-$C_6$ alkyl, $C_3$-$C_7$ cycloalkyl, halogenated $C_1$-$C_6$ alkyl, halogenated $C_3$-$C_7$ cycloalkyl, $-NR^{15}R^{16}$, $C_6$-$C_{10}$ aryl, halogenated $C_6$-$C_{10}$ aryl, $C_1$-$C_6$ alkyl substituted $C_6$-$C_{10}$ aryl, halogenated $C_1$-$C_6$ alkyl substituted $C_6$-$C_{10}$ aryl, 5- to 10-membered heteroaryl and halogenated 5- to 10-membered heteroaryl.

In some embodiments, $R^9$ is selected from $C_1$-$C_6$ alkyl, $C_3$-$C_7$ cycloalkyl, phenyl, $C_1$-$C_6$ alkyl substituted phenyl and halogenated $C_1$-$C_6$ alkyl substituted phenyl.

In some embodiments, $R^{13}$ and $R^{14}$ are each independently selected from H and $C_1$-$C_6$ alkyl.

In some embodiments, $R^{15}$ and $R^{16}$ are each independently selected from H and $C_1$-$C_6$ alkyl.

In some embodiments, $R^8$ is selected from $CH_3$, $CF_3$, $CH_2CF_3$, $CF_2CF_3$, methoxy,

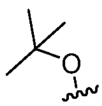 , 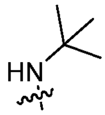 ,

cyclopropyl,

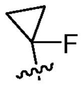
,

phenyl,

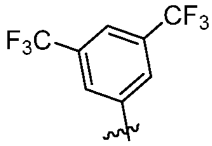
,

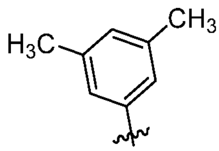
,
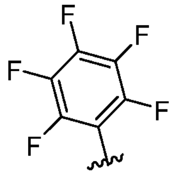

and pyridin-3-yl.

In some embodiments, $R^9$ is selected from $CH_3$, cyclopropyl, phenyl, p-methylphenyl, and p-trifluoromethylphenyl.

In some embodiments, the cyano compound represented by general formula I is selected from the cyano compound represented by general formula IA:

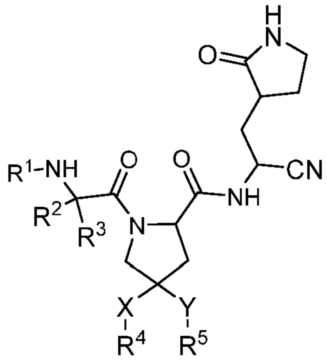

IA

wherein, $R^1$, $R^2$, $R^3$, $R^4$, $R^5$, X and Y are as defined above.

In some embodiments, the cyano compound represented by general formula I is selected from the cyano compound represented by general formula IB:

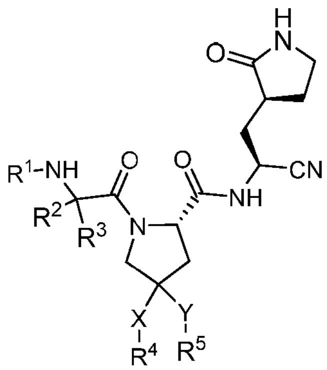

IB

wherein, each substituent is as defined above.

In some embodiments, the cyano compound represented by general formula I is selected from any one of the cyano compound represented by the following general formula:

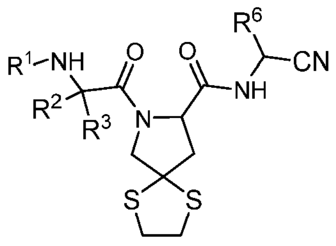

I-1

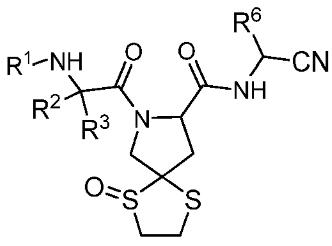

I-2

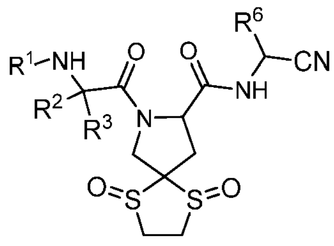

I-3

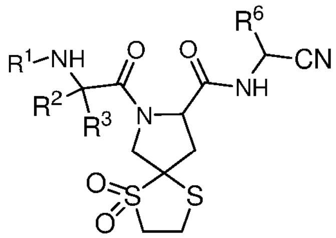

I-4

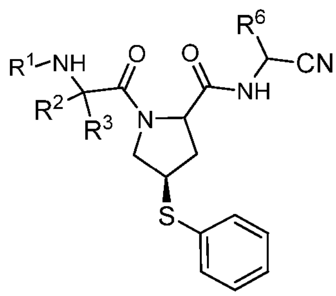

I-5

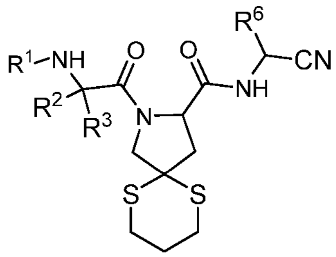

I-6

or

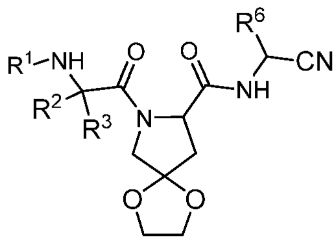

I-7

wherein, $R^1$, $R^2$, $R^3$ and $R^6$ are as defined above.

In some embodiments, the compound represented by general formula I of the present invention is selected from the following compounds:

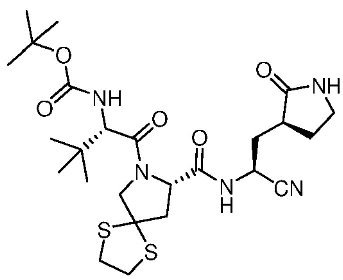

Compound 1

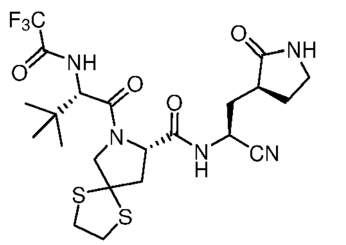

Compound 2

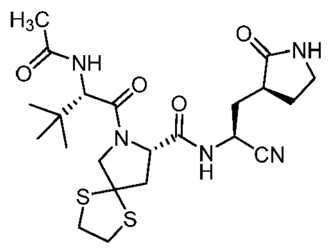

Compound 3

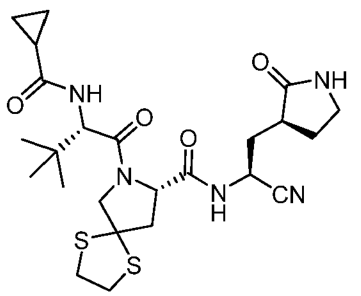

Compound 4

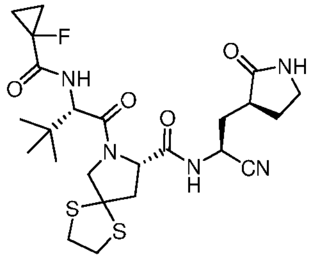

Compound 5

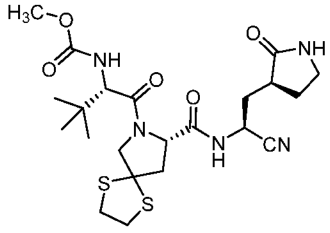

Compound 6

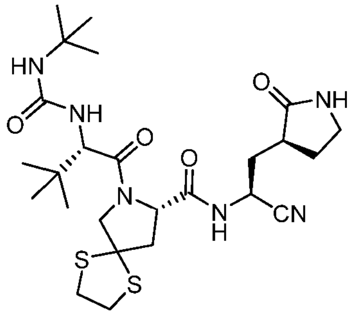

Compound 7

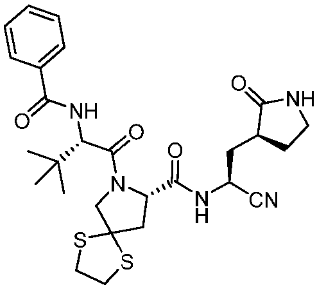

Compound 8

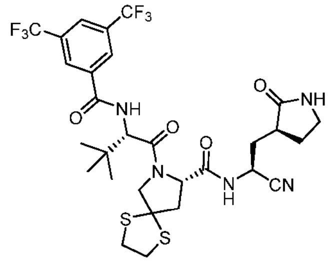

Compound 9

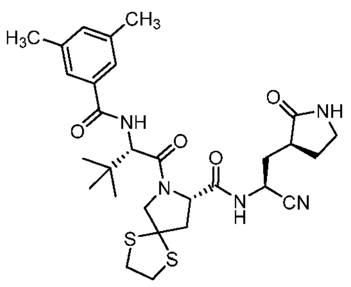

Compound 10

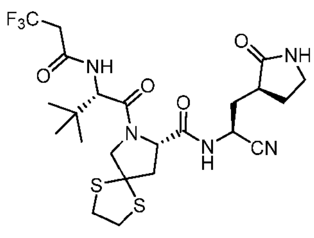

Compound 11

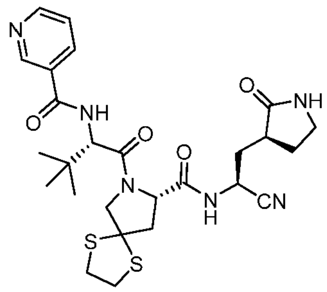

Compound 12

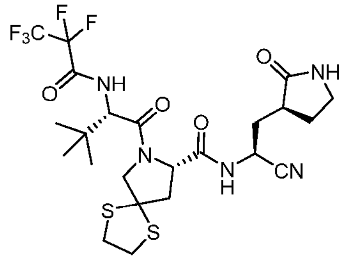

Compound 13

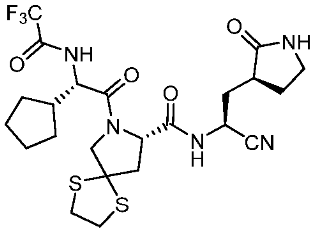

Compound 14

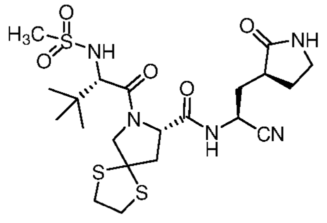

Compound 15

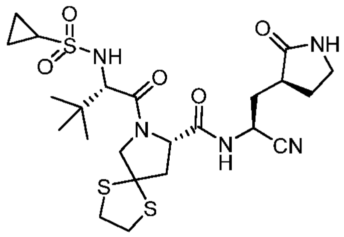

Compound 16

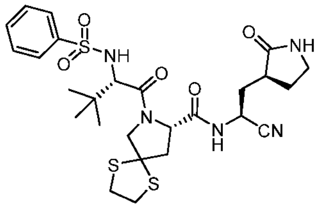

Compound 17

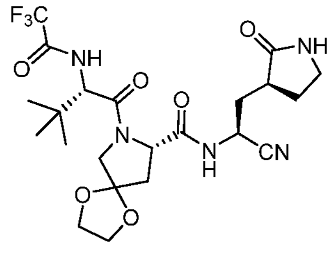

Compound 18

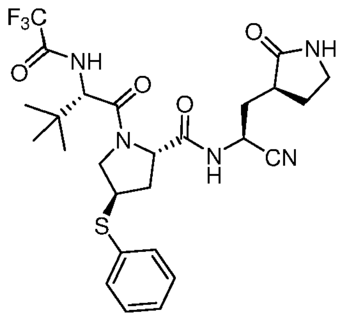

Compound 19

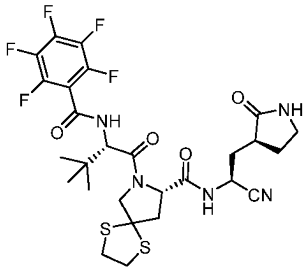

Compound 20

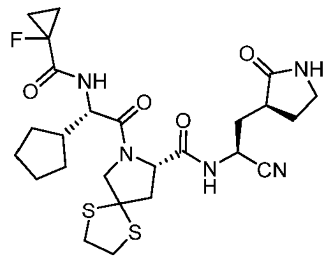

Compound 21

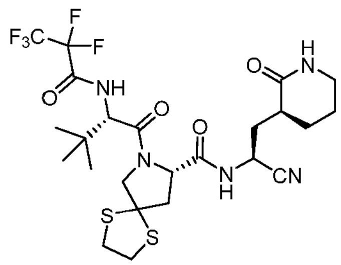

Compound 22

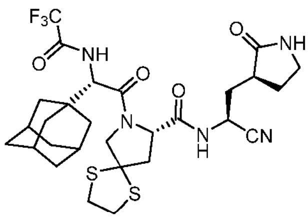

Compound 23

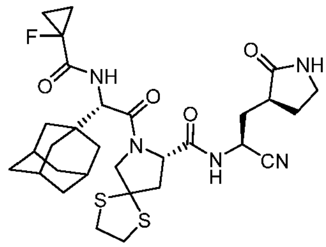

Compound 24

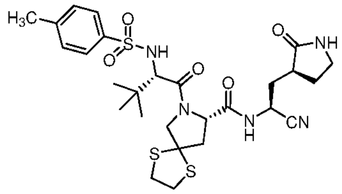

Compound 25

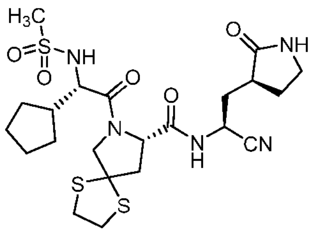

Compound 26

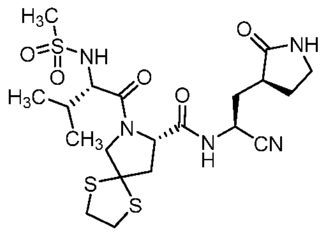

Compound 27

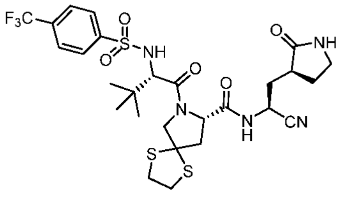

Compound 28

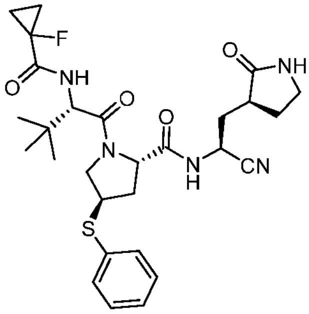

Compound 29

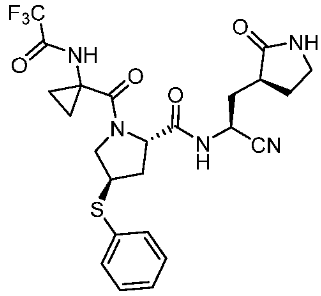

Compound 30

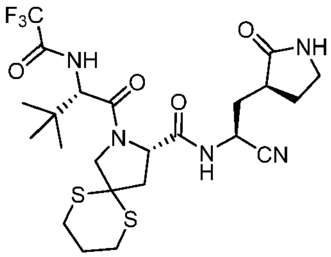

Compound 31

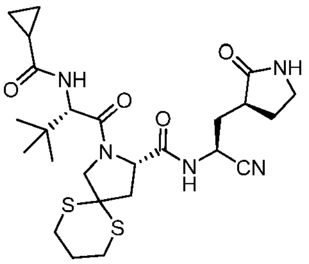

Compound 32

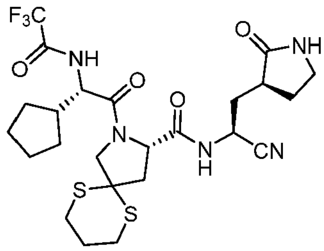

Compound 33

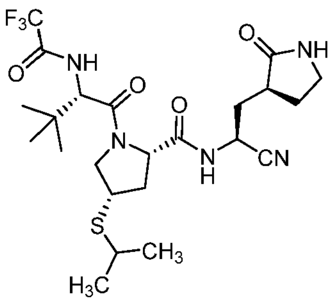

Compound 34

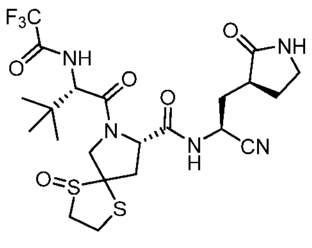

Compound 35

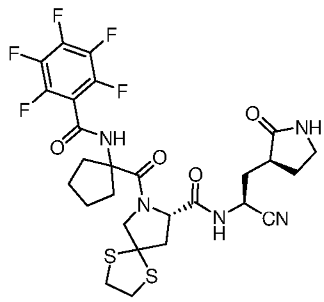

Compound 36

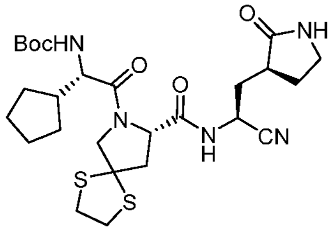

Compound 37

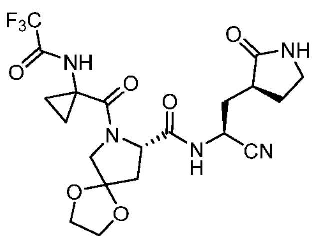

Compound 38

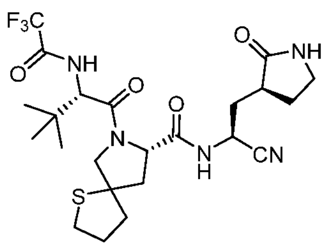

Compound 39

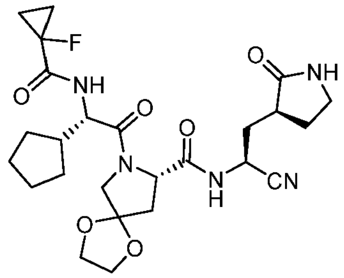

Compound 40

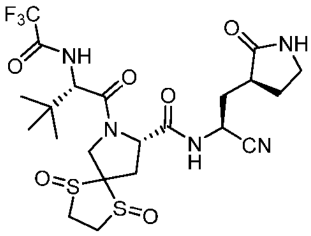

Compound 41

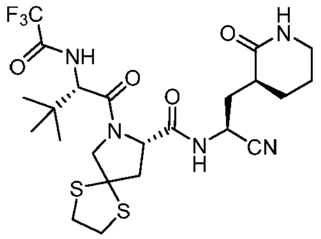

Compound 42

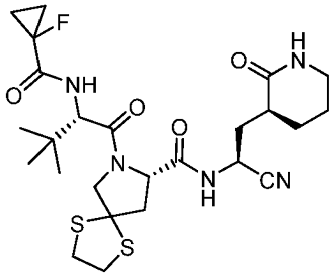

Compound 43

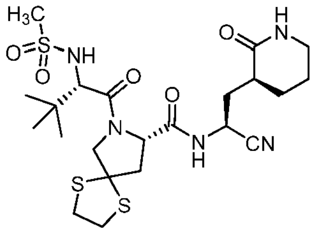

Compound 44

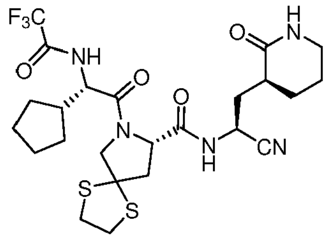

Compound 45

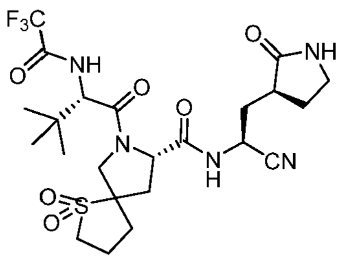

Compound 46

The present invention provides a method for preparing the compound represented by general formula I, and the method is one of following methods:

Method i:

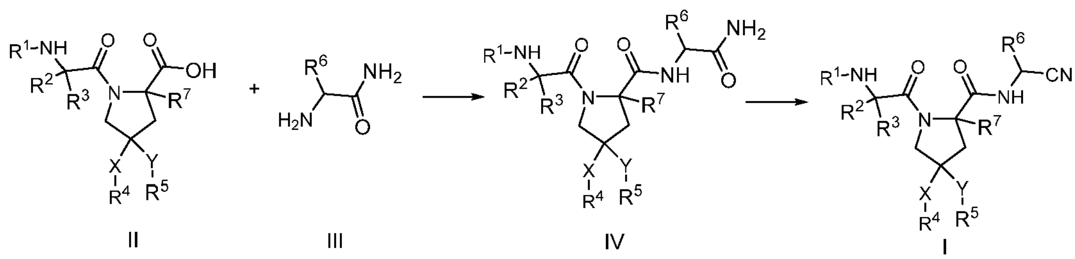

ia) a compound represented by formula IV is obtained via condensation reaction of a compound represented by formula II and a compound represented by formula III;
preferably, the step ia) is that: the compound represented by formula II is reacted with the compound represented by formula III under the action of a condensing agent and a base in a solvent at a temperature of -20°C to 50°C for 0.1-12 h, thereby obtaining the compound represented by formula IV;
wherein, the solvent is one of or a mixture of more than one of the following: tetrahydrofuran, dichloromethane, N,N-dimethylformamide, N,N-dimethylacetamide, ethyl acetate and 1,4-dioxane;
optionally, the condensing agent is one of or a mixture of more than one of the following: 2-(7-azabenzotriazole)-N,N,N',N'-tetramethyluronium hexafluorophosphate, 1-ethyl-(3-dimethylaminopropyl)carbodiimide hydrochloride, 1-hydroxybenzotriazole, 2-hydroxypyridine-N-oxide, 1-propyl phosphoric anhydride, 1H-benzotriazol-1-yloxytripyrrolidinyl hexafluorophosphate, N,N'-carbonyldiimidazole and O-benzotriazole-tetramethyluronium hexafluorophosphate;

optionally, the base is N,N-diisopropylethylamine, triethylamine and N-methylmorpholine;

ib) the compound represented by formula IV is dehydrated to obtain the compound represented by general formula I;

preferably, the step ib) is that: the compound represented by formula IV is reacted with a dehydrating agent in an anhydrous solvent at a temperature of -20°C to 50°C for 1-24 h to obtain the compound represented by general formula I;

wherein, the anhydrous solvent is one of or a mixture of more than one of the following: tetrahydrofuran, dichloromethane, toluene, 1,4-dioxane and pyridine;

the dehydrating agent is trifluoroacetic anhydride and methyl N-(triethylammoniumsulfonyl)carbamate; or

Method ii:

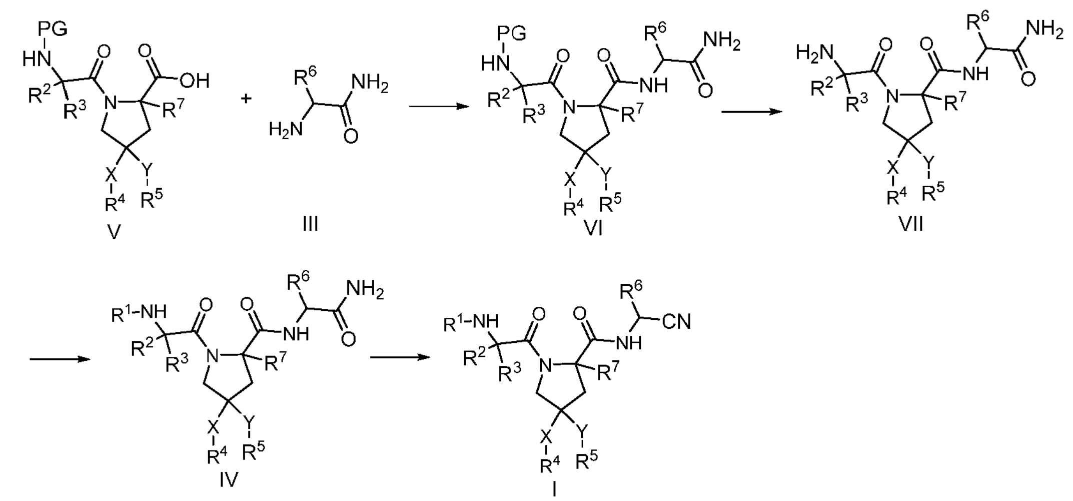

V III VI VII IV I

iia) a compound represented by formula VI is obtained by via condensation reaction of a compound represented by formula V and the compound represented by formula III, wherein PG in the compound represented by formula V is an amino protecting group;

preferably, the step iia) is that: the compound represented by formula V is reacted with the compound represented by formula III under the action of a condensing agent and a base in a solvent at a temperature of -20°C to 50°C for 0.1-12 h, thereby obtaining the compound represented by formula VI,

wherein, the solvent is one of or a mixture of more than one of the following: tetrahydrofuran, dichloromethane, N,N-dimethylformamide, N,N-dimethylacetamide, ethyl acetate and 1,4-dioxane,

the condensing agent is one of or a mixture of more than one of the following: 2-(7-azabenzotriazole)-N,N,N',N'-tetramethyluronium hexafluorophosphate, 1-ethyl-(3-dimethylaminopropyl)carbodiimide hydrochloride, 1-hydroxybenzotriazole, 2-hydroxypyridine-N-oxide, 1-propyl phosphoric anhydride, 1H-benzotriazol-1-yloxytripyrrolidinyl hexafluorophosphate, N,N'-carbonyldiimidazole and O-benzotriazole-tetramethyluronium hexafluorophosphate;

the base is N,N-diisopropylethylamine, triethylamine and N-methylmorpholine;

The amino protecting group PG is tert-butoxycarbonyl, benzyl and p-methoxybenzyl;

iib) the compound represented by formula VI is deprotected to obtain a compound represented by formula VII;

preferably, the step iib) is that: at a temperature of -20°C to 50°C, the compound represented by formula VI is reacted with an organic solution of trifluoroacetic acid or hydrogen chloride or $Pd/C/H_2$ to obtain the compound represented by formula VII;

iic) the compound represented by formula IV is obtained by aminoacylation, sulfonylation or condensation reaction of the compound represented by formula VII;

preferably, the step iic) is that:

at a temperature of -20°C to 50°C, the compound represented by formula VII is subjected to an aminoacylation reaction with acid chloride or acid anhydride under the condition of adding a base to obtain the compound

represented by formula IV; or,

at a temperature of -20°C to 50°C, the compound represented by formula VII is subjected to a sulfonylation reaction with sulfonyl chloride or sulfonic anhydride under the condition of adding a base to obtain the compound represented by formula IV; or,

at a temperature of -20°C to 50°C, the compound represented by formula VII is subjected to a condensation reaction with a carboxyl compound under the condition of a condensing agent and a base to obtain the compound represented by formula IV;

optionally, the base is N,N-diisopropylethylamine, triethylamine and N-methylmorpholine;

optionally, the condensing agent is one of or a mixture of more than one of the following: 2-(7-azabenzotriazole)-N,N,N',N'-tetramethyluronium hexafluorophosphate, 1-ethyl-(3-dimethylaminopropyl)carbodiimide hydrochloride, 1-hydroxybenzotriazole, 2-hydroxypyridine-N-oxide, 1-propyl phosphoric anhydride, 1H-benzotriazol-1-yloxytripyrrolidinyl hexafluorophosphate, N,N'-carbonyldiimidazole and O-benzotriazole-tetramethyluronium hexafluorophosphate,

iid) the compound represented by formula IV is dehydrated to obtain the compound represented by general formula I;

preferably, the step iid) is that: the compound represented by formula IV is reacted with a dehydrating agent in an anhydrous solvent at a temperature of -20°C to 50°C for 1-24 h to obtain the compound represented by general formula I;

wherein, the anhydrous solvent is one of or a mixture of more than one of the following: tetrahydrofuran, dichloromethane, toluene, 1,4-dioxane and pyridine;

optionally, the dehydrating agent is trifluoroacetic anhydride and methyl N-(triethylammoniumsulfonyl) carbamate,

wherein, each substituent is as defined above.

Another aspect of the present invention further provides a pharmaceutical composition, which comprises one or more selected from a cyano compound of general formula I, a racemate, an enantiomer and a diastereomer thereof, and their pharmaceutically acceptable salts. The pharmaceutical composition may further include one or more pharmaceutically acceptable adjuvants, diluents, carriers, excipients or adjuvants.

Another aspect of the present invention further provides a pharmaceutical composition, which comprises a cyano compound of formula I, or a racemate, an enantiomer, a diastereoisomer or a pharmaceutically acceptable salt thereof, and a pharmaceutically acceptable excipient; optionally, the pharmaceutical composition further comprises ritonavir or a pharmaceutically acceptable salt thereof.

Another aspect of the present invention further provides a pharmaceutical combination, which comprises a cyano compound of general formula I, a racemate, an enantiomer, a diastereoisomer or a pharmaceutically acceptable salt thereof, and ritonavir or a pharmaceutically acceptable salt thereof.

Another aspect of the present invention further provides a pharmaceutical composition, which comprises a cyano compound of general formula I or a pharmaceutically acceptable salt thereof, ritonavir or a pharmaceutically acceptable salt thereof, and a pharmaceutically acceptable excipient.

Experiments prove that the compound of the present invention has an inhibitory activity on coronavirus 3CL proteases and picornavirus 3CL proteases.

Therefore, another aspect of the present invention provides a coronavirus 3CL protease inhibitor and/or a picornavirus 3CL protease inhibitor, which comprises one or more selected from a cyano compound of general formula **I,** a racemate, an enantiomer and a diastereomer thereof, and their pharmaceutically acceptable salts, or the above-mentioned pharmaceutical composition.

The present invention further provides the above-mentioned cyano compound, the racemate, the enantiomer, the diastereomer and the pharmaceutically acceptable salt thereof, or their mixture, or use of the pharmaceutical composition in the preparation of a medicament selected from a medicament for inhibiting coronavirus 3CL protease activity, a medicament for preventing and/or treating coronavirus infection, a medicament for inhibiting picornavirus 3CL protease activity, and a medicament for preventing and/or treating picornavirus infection.

The present invention further provides compounds for use in a method of inhibiting 3CL protease, the method comprises administering one or more selected from the cyano compound of general formula I, the racemate, the enantiomer and the diastereomer thereof, and their pharmaceutically acceptable salts of the present invention, or the pharmaceutical composition of the present invention to a subject in need thereof.

The present invention further provides compounds for use in a method of preventing and/or treating a disease or condition, the method comprises administering one or more selected from the cyano compound of general formula I, the racemate, the enantiomer and the diastereomer thereof, and their pharmaceutically acceptable salts of the present invention, or the pharmaceutical composition of the present invention to a subject in need thereof, the disease or condition is a 3CL protease mediated disease or condition, in particular a disease or condition associated with coronavirus infection

and/or picornavirus infection.

The present invention further provides one or more of the cyano compound of general formula I, the racemate, the enantiomer and the diastereomer thereof, and their pharmaceutically acceptable salts described above, use of the pharmaceutical composition thereof in the preparation of a medicament for preventing or treating a related disease caused by coronavirus and/or picornavirus infection.

The present invention further provides compounds for use in a method for preventing or treating a related disease caused by coronavirus and/or picornavirus infection. The method comprises administering to a patient a therapeutically effective amount of a pharmaceutical formulation comprising one or more of the cyano compound of general formula I, the racemate, the enantiomer and the diastereomer thereof, and their pharmaceutically acceptable salts of the present invention.

In some embodiments, the coronavirus is selected from SARS-CoV, MERS-CoV, H229E-CoV, HKU1-CoV, NL63-CoV, OC43-CoV or SARS-CoV-2.

In some embodiments, the related disease caused by the coronavirus infection is selected from respiratory tract infection, pneumonia or a complication thereof.

Definition and Description of Terminology

"$C_1$-$C_{10}$ alkyl" means a straight or branched saturated hydrocarbon group having 1, 2, 3, 4, 5, 6, 7, 8, 9 or 10 carbon atoms. Specific examples of the alkyl include, but are not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, *etc.;* "$C_1$-$C_6$ alkyl" means a straight or branched alkyl containing 1-6 carbon atoms, including but not limited to methyl, ethyl, propyl, isopropyl, butyl, isobutyl, tert-butyl, pentyl, isopentyl, hexyl, *etc.*

"Alkylene" can be understood to mean a straight or branched saturated divalent hydrocarbon group. "$C_2$-$C_6$ alkylene" can be understood to mean a straight or branched saturated divalent hydrocarbon group containing 2-6 carbon atoms, including but not limited to $CH_2CH_2$, $CH_2CH_2CH_2$, $CH_2CH_2CH_2CH_2$, $CH_2CH_2CH_2CH_2CH_2$, $CH_2CH_2CH_2CH_2CH_2CH_2$, *etc.*

"$C_3$-$C_8$ cycloalkyl" means a cyclic alkyl containing 3-8 ring carbon atoms. "$C_3$-$C_7$ cycloalkyl" means a cyclic alkyl containing 3-7 ring carbon atoms, the cycloalkyl in the present disclosure includes but not limited to cyclopropyl, methylcyclopropyl, ethylcyclopropyl, dimethylcyclopropyl, cyclobutyl, methylcyclobutyl, ethylcyclobutyl, cyclopentyl, cyclohexyl, *etc.*

"$C_1$-$C_{10}$ alkoxy" means a straight branched or cyclic alkoxy containing 1-10 carbon atoms. "$C_1$-$C_6$ alkoxy" means a straight, branched or cyclic alkoxy containing 1-6 carbon atoms. The alkoxy in the present disclosure includes but not limited to methoxy, ethoxy, propoxy, isopropoxy, butoxy, isobutoxy, tert-butoxy, pentyloxy, isopentyloxy, cyclopentyloxy, hexyloxy, cyclohexyloxy, *etc.*

"Aryl" refers to an all-carbon monocyclic or fused polycyclic aromatic ring group having a conjugated $\pi$-electron system. The aryl can have 6-20 carbon atoms, 6-14 carbon atoms or 6-12 carbon atoms. "$C_6$-$C_{20}$ aryl" can be understood to mean an aryl with 6-20 carbon atoms. Especially a ring with 6 carbon atoms ("$C_6$ aryl"), such as phenyl; or a ring with 9 carbon atoms ("$C_9$ aryl"), such as indanyl or indenyl; or a ring with 10 carbon atoms ("$C_{10}$ aryl"), such as tetrahydronaphthyl, dihydronaphthyl or naphthyl; or a ring with 13 carbon atoms ("$C_{13}$ aryl"), such as fluorenyl; or a ring with 14 carbon atoms ("$C_{14}$ aryl"), such as anthracenyl. "$C_6$-$C_{10}$ aryl" can be understood to mean an aryl containing 6-10 carbon atoms. Especially a ring with 6 carbon atoms ("$C_6$ aryl"), such as phenyl; or a ring with 9 carbon atoms ("$C_9$ aryl"), such as indanyl or indenyl; or a ring with 10 carbon atoms ("$C_{10}$ aryl"), such as tetrahydronaphthyl, dihydronaphthyl or naphthyl.

"Heteroaryl" is an aromatic ring group containing 5-20 ring atoms, one or more of the ring atoms is a heteroatom selected from N, O or S, and the rest of the ring atoms are carbon. "5- to 20-membered heteroaryl" can be understood to mean a heteroaryl having 5-20 ring atoms, especially 5 or 6 or 9 or 10 or 13 or 14 ring atoms, and containing 1-7 heteroatoms independently selected from N, O and S. "5- to 10-membered heteroaryl" can be understood to mean a heteroaryl having 5-10 ring atoms, especially 5 or 6 or 9 or 10 ring atoms, and containing 1-5 heteroatoms independently selected from N, O and S. "5- to 6-membered heteroaryl" can be understood to mean a heteroaryl having 5 or 6 ring atoms and containing 1-3 heteroatoms independently selected from N, O and S. The heteroaryl described in the present invention can be selected from thienyl, furyl, pyrrolyl, oxazolyl, thiazolyl, imidazolyl, pyrazolyl, isoxazolyl, isothiazolyl, oxadiazolyl, triazolyl, thiadiazolyl, and the like, and the benzo derivatives thereof, such as benzofuryl, benzothienyl, benzothiazolyl, benzoxazolyl, benzisoxazolyl, benzoimidazolyl, benzotriazolyl, indazolyl, indolyl, isoindolyl, and the like; or pyridyl, pyridazinyl, pyrimidinyl, pyrazinyl, triazinyl, and the like, and the benzo derivatives thereof such as quinolyl, quinazolinyl, isoquinolinyl, and the like; or azocinyl, indolizinyl, purinyl, and the like and the benzo derivatives thereof; or cinnolinyl, phthalazinyl, quinazolinyl, quinoxalinyl, naphthyridinyl, pteridinyl, carbazolyl, acridinyl, phenazinyl, phenothiazinyl or phenoxazinyl, and the like.

"Halogen" is selected from fluorine, chlorine, bromine and iodine.

"Halo" includes monohalo, polyhalo or perhalo, that is, one, more or all hydrogen atoms are substituted with halogen.

"Substituted" means that one or more hydrogen atoms on a group are substituted with one or more substituents.

The term "optional" or "optionally" mean that the subsequently described event or circumstance may or may not occur, and the description includes the occurrence and the non-occurrence of the event or circumstance.

"Therapeutically effective amount" means an amount of a compound of the present invention that (i) treats a particular disease, condition, or disorder, (ii) attenuates, ameliorates, or eliminates one or more symptoms of a particular disease, condition, or disorder, or (iii) delays the onset of one or more symptoms of a particular disease, condition, or disorder described herein. The amount of a compound of the present disclosure which constitutes a "therapeutically effective amount" will vary depending on the compound, the conditions and their severity, the manner of administration, and the age of the mammal to be treated, but can be determined routinely by those skilled in the art according to their own knowledge and the present disclosure.

"Pharmaceutically acceptable salt" refers to salts of pharmaceutically acceptable acids or bases, including salts formed between compounds and inorganic or organic acids, and salts formed between compounds and inorganic or organic bases.

The term "pharmaceutical combination" refers to a combination comprising two or more active ingredients or pharmaceutically acceptable salts thereof. In some embodiments of the present invention, the active ingredients in the pharmaceutical combination or the pharmaceutically acceptable salts thereof can be administered simultaneously. In some embodiments of the present invention, the active ingredients in the pharmaceutical combination or the pharmaceutically acceptable salts thereof can also be administered separately or sequentially.

"Pharmaceutical composition" refers to a mixture of one or more of the compounds or salts thereof according to the present disclosure and a pharmaceutically acceptable adjuvant. An object of the pharmaceutical composition is to facilitate administering the compound according to the present disclosure to an organism.

"Pharmaceutically acceptable adjuvant" refers to those adjuvants which have no significant irritating effect on the organism and do not impair the bioactivity and properties of the active compound. Suitable adjuvants are well known to those skilled in the art, and are such as a carbohydrate, a wax, a water-soluble and/or water-swellable polymer, a hydrophilic or hydrophobic material, gelatin, an oil, a solvent, water, and the like.

**Brief Description of the Drawings**

FIG. 1 is a graph showing the inhibitory rate-concentration of compound 2 on the *in vitro* activity of the WIV04 strain of SARS-CoV-2.

FIG. 2 is a graph showing the inhibitory rate-concentration of compound 2 on the *in vitro* activity of the B.1.351 strain of SARS-CoV-2.

FIG. 3 is a graph showing the inhibitory effect of compound 2 on the virus titre in the lungs of mice 2 days after infection (FIG. A) and 4 days after infection (FIG. B) in example 26.

FIG. 4 is a graph showing the body weight changes of mice in example 26.

FIG. 5 is a graph showing the inhibitory effect of compound 2 on the virus titre in the brains of mice 4 days after infection in example 26.

**Detailed Description of Embodiments**

The present disclosure is further described by way of examples below. In the following examples, starting materials are commercially available or can be prepared by methods documented in the literature/organic synthetic methods known in the art.

Example 1

Preparation of compound 1-1:

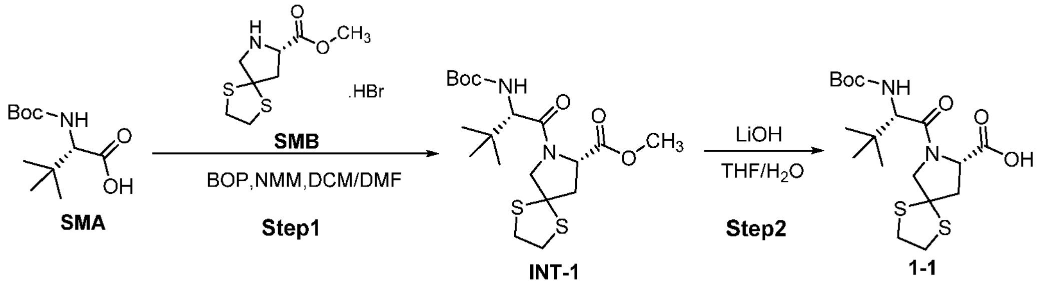


Step 1: The starting material SMA (2.74 g, 11.85 mmol), 35 ml of dichloromethane and 35 ml of DMF were added into a reaction flask and cooled down to 0°C. The starting material SMB (3.56 g, 11.86 mmol), benzotriazole- 1-yloxytris(dimethylamino)phosphonium hexafluorophosphate (BOP, 6.29 g, 14.22 mmol) and N-methylmorpholine (NMM, 3.91 ml, 35.56 mmol) were added and brought to room temperature for reaction for 10 h. After the reaction was completed, an appropriate amount of dichloromethane was added, and the organic phase was washed successively with 1N aqueous hydrochloric acid solution and saturated brine. After the washing, the organic phase was dried over anhydrous sodium sulphate, and the organic phase was concentrated to dryness, and 3.71 g of INT-1 was obtained by column chromatography; ESI-MS: 433.2 m/z $[M+H]^+$; $^1H$ NMR (400 MHz, DMSO-$d_6$): $\delta_H$: 6.75 (d, J = 9.2 Hz, 1H), 4.38 (t, J = 8.2 Hz, 1H), 4.25 (d, J = 10.9 Hz, 1H), 4.11 (d, J = 9.3 Hz, 1H), 3.93 (t, J = 9.3 Hz, 1H), 3.62 (s, 3H), 3.40-3.31 (m, 4H), 2.70 (dd, J = 13.1, 7.9 Hz, 1H), 2.37 (dd, J = 13.2, 8.4 Hz, 1H), 1.37 (s, 9H), 0.94 (s, 9H).

Step 2: INT-1 (3.71 g, 8.58 mmol), 37 ml of THF, 37 ml of purified water and lithium hydroxide monohydrate (0.72 g, 17.16 mmol) were added into a reaction flask, and reacted at room temperature for 2 h. After the reaction was completed, the reaction liquid was adjusted with concentrated hydrochloric acid to pH = 4, and filtered to obtain 3.4 g of compound 1-1; ESI-MS: 419.2 m/z $[M+H]^+$; $^1H$ NMR (400 MHz, DMSO-$d_6$): $\delta_H$:12.68 (s, 1H), 6.71 (d, J = 9.4 Hz, 1H), 4.38-4.19 (m, 2H), 4.11 (d, J = 9.4 Hz, 1H), 3.88 (d, J = 10.9 Hz, 1H), 3.41-3.29 (m, 4H), 2.69 (dd, J = 13.1, 7.9 Hz, 1H), 2.34 (dd, J = 13.2, 8.9 Hz, 1H), 1.38 (s, 9H), 0.94 (s, 9H).

Preparation of compound 1-2:

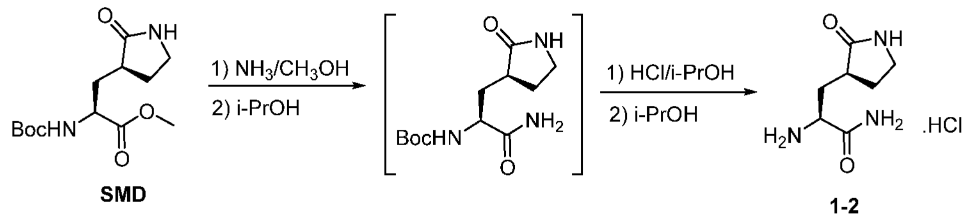


Ammonia-methanol solution (700 ml, 7 mol/L) and starting material SMD (100 g, 0.349 mol) were added into a reaction flask, stirred to dissolve, and reacted for 36 h at a constant temperature of 25 ± 5°C. After the reaction was completed, the reaction liquid was concentrated until the remaining reaction liquid was about 250 ml. 300 ml of isopropanol was added and further concentrated until the remaining reaction liquid was about 250 ml (repeated three times). The resulting reaction liquid was subjected to nitrogen replacement and cooled down to 10 ± 5°C. 500 ml of hydrogen chloride-isopropanol solution (4 mol/L) was added into the reaction kettle. After the addition, the temperature was raised to 25 ± 5°C, and the temperature was kept at 25 ± 5°C for 9 hours. After the reaction was completed, the reaction liquid was concentrated under reduced pressure until the remaining reaction liquid was about 250 ml in volume. 300 ml of isopropanol was added and further concentrated under reduced pressure until the remaining reaction liquid was about 250 ml in volume (repeated twice). 100 ml of isopropanol was added, stirred for 30 ± 5 min and filtered, and the filter cake was rinsed with 50 ml of isopropanol to obtain a wet product; The wet product was dried under vacuum at 45 ± 5°C to obtain 66.7 g of compound 1-2, yield: 92%; $^1H$ NMR (400 MHz, DMSO-$d_6$): $\delta_H$:8.45 (d, J = 5.1 Hz, 3H), 8.25 - 8.04 (m, 1H), 7.95 (s, 1H), 7.67 - 7.49 (m, 1H), 3.85-3.80 (m, 1H), 3.19-3.13 (m, 2H), 2.59 - 2.51 (m, 1H), 2.32-2.27 (m, 1H), 2.05-1.98 (m, 1H), 1.82 - 1.66 (m, 2H); ESI-MS: 172.1 m/z $[M+H]^+$.

Preparation of compound 1:

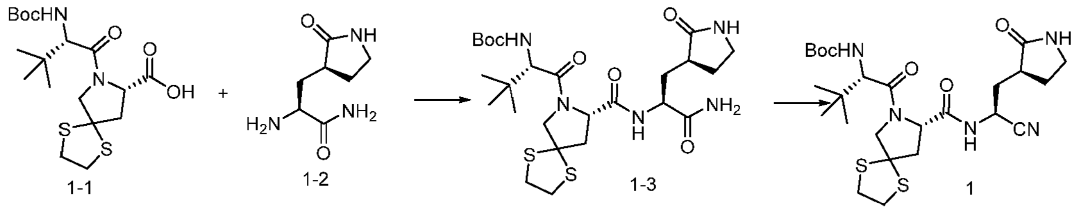

Compound 1-1 (419 mg, 1 mmol) was placed in a two-necked flask, and 5 mL of dichloromethane was added under nitrogen protection, and then 2-(7-azabenzotriazole)-N,N,N',N'-tetramethyluronium hexafluorophosphate (400 mg, 1.1 mmol) was added and stirred at room temperature for 1 h. Compound 1-2 (1 mmol) was dissolved in 1 mL of dichloromethane and added to the above system, then N,N-diisopropylethylamine (0.5 mL, 1 mmol) was added under an ice-water bath, and the ice-water bath was removed. The system was stirred overnight at room temperature. Workup was performed, 50 mL of dichloromethane was added, washed with 1M aqueous hydrochloric acid three times, and washed with saturated aqueous sodium bicarbonate solution three times. The organic phase was washed with saturated saline solution, dried over anhydrous sodium sulphate, and spin-dried by filtration to afford compound 1-3 (a white solid, 469 mg, yield 82%). ESI-MS: m/z 572.3$[M+H]^+$.

Compound 1-3 (114 mg, 0.2 mmol) and Burgess reagent (1.5 eq) were added to a two-necked flask, nitrogen was charged and discharged for three time, then dichloromethane dried over molecular sieves was added and stirred at room temperature overnight. Thin layer chromatography showed that the raw materials was substantially completely reacted. Workup was performed, column chromatography was performed to afford compound 1 (a white solid, 52 mg, yield 47%), ESI-MS: 554.3 m/z $[M+H]^+$.

Example 2

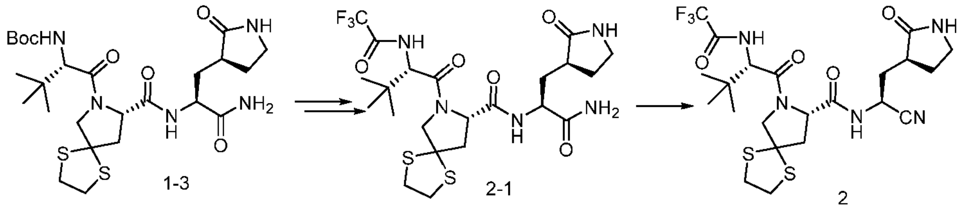

Compound 1-3 (572 mg, 1 mmol) was dissolved in 3 mL of 4M hydrogen chloride/1,4-dioxane solution, or dissolved in 2 mL of dichloromethane, then 2 mL of trifluoroacetic acid was added dropwise and stirred at ambient temperature. Thin layer chromatography showed that the reaction of raw materials was substantially completely, and then the solvent was fully spin-dried. The obtained crude product was dissolved in 2 mL of dichloromethane under nitrogen protection, and triethylamine (3 mmol) was added, then the system was placed in an ice-water bath, and trifluoroacetic anhydride (1.2 mmol) was added dropwise. Thin layer chromatography showed that the raw materials was substantially completely reacted, and then 50 mL of dichloromethane was added and washed three times with 1M aqueous hydrochloric acid solution and three times with saturated aqueous sodium bicarbonate solution. The organic phase was washed with saturated saline solution, dried over anhydrous sodium sulphate, and passed through a column to afford compound 2-1 (a white solid, 265 mg, yield 46%). ESI-MS: m/z 568.3$[M+H]^+$.

Compound 2-1 (113 mg, 0.2 mmol) and Burgess reagent (1.5 eq) were added to a two-necked flask, nitrogen was charged and discharged for three time, then dichloromethane dried over molecular sieves was added and stirred at room temperature overnight. Thin layer chromatography showed that the raw materials was substantially completely reacted. Workup was performed, column chromatography was performed to afford compound 2 (a white solid, 41 mg, yield 47%), $^1H$ NMR (400 MHz, DMSO-$d_6$) $\delta$ 9.46 (d, $J$ = 8.7 Hz, 1H), 9.05 (d, $J$ = 8.6 Hz, 1H), 7.67 (s, 1H), 4.97 (ddd, $J$ = 11.0, 8.5, 5.0 Hz, 1H), 4.53 (d, $J$ = 8.7 Hz, 1H), 4.34 (dd, $J$ = 9.9, 7.1 Hz, 1H), 4.26-4.14 (m, 1H), 3.92 (d, $J$ = 10.9 Hz, 1H), 3.50-3.34 (m, 4H), 3.22-3.11 (m, 1H), 3.06 (td, $J$ = 9.3, 7.1 Hz, 1H), 2.68-2.58 (m, 1H), 2.50-2.43 (m, 1H), 2.31 (dd, $J$ = 13.0, 10.0 Hz, 1H), 2.23-2.07 (m, 2H), 1.71 (tdd, $J$ = 14.9, 10.3, 7.4 Hz, 2H), 0.99 (s, 9H). ESI-MS: 550.3 m/z $[M+H]^+$.

Example 3

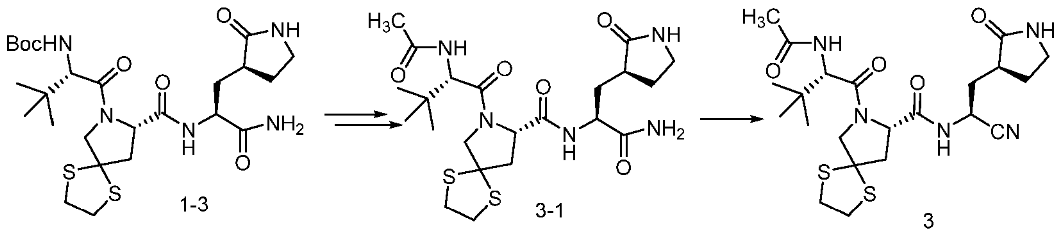

According to the same method in example 2, the difference lies in that: acetic anhydride was used instead of trifluoroacetic anhydride to afford compound 3 (a white solid, 45 mg, yield 45%), ESI-MS: 496.3 m/z [M+H]+.

Example 4

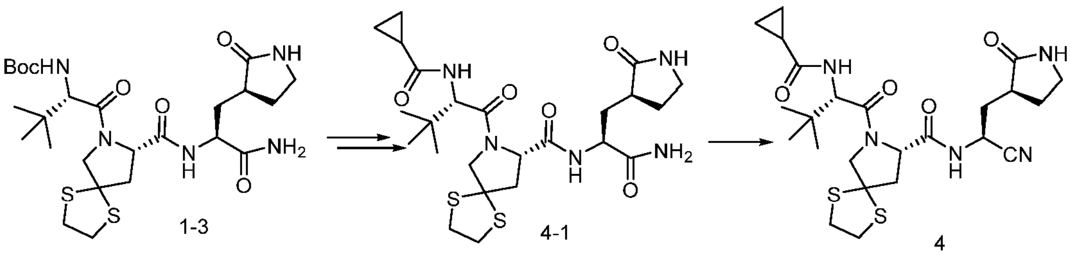

According to the same method in example 2, the difference lies in that: cyclopropane formic anhydride was used instead of trifluoroacetic anhydride to afford compound 4 (a white solid, 32 mg, yield 31%), ESI-MS: 522.3 m/z [M+H]+.

Example 5

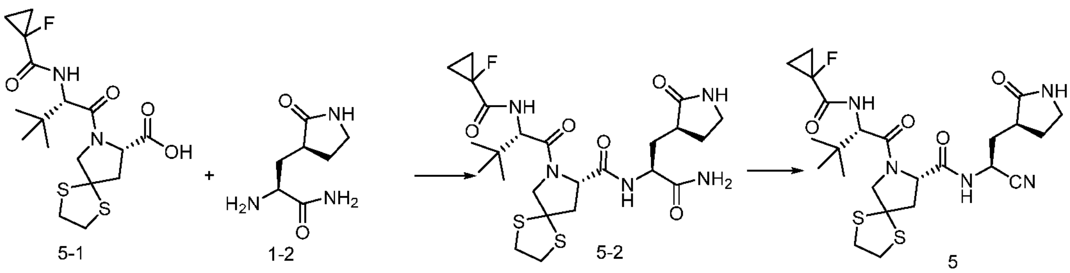

According to the same method in example 1, the difference lies in that: 5-1 was used instead of 1-1 to afford compound 5 (a white solid, 22 mg, yield 20%), $^1$H NMR (400 MHz, DMSO-$d_6$) δ 9.03 (d, *J* = 8.4 Hz, 1H), 7.67 (s, 1H), 7.41 (dd, *J* = 9.1, 2.6 Hz, 1H), 4.96 (ddd, *J* = 10.6, 8.4, 5.4 Hz, 1H), 4.58-4.50 (m, 1H), 4.34 (dd, *J* = 9.8, 7.2 Hz, 1H), 4.26-4.21 (m, 1H), 3.89 (d, *J* = 11.0 Hz, 1H), 3.45-3.33 (m, 4H), 3.22-3.03 (m, 2H), 2.64-2.57 (m, 1H), 2.45 (ddd, *J* = 10.1, 8.5, 4.5 Hz, 1H), 2.31 (dd, *J* = 12.9, 9.8 Hz, 1H), 2.20-2.10 (m, 2H), 1.84-1.67 (m, 2H), 1.39-1.31 (m, 2H), 1.24 (t, *J* = 9.5 Hz, 2H), 0.97 (s, 9H). ESI-MS: 540.2 m/z [M+H] +.

Example 6

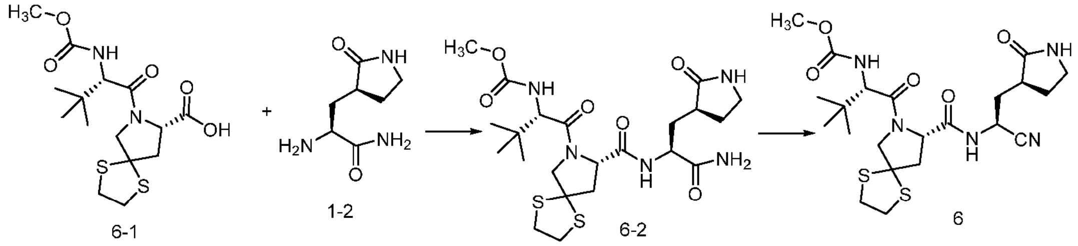

6-1 1-2 6-2 6

According to the same method in example 1, the difference lies in that: 6-1 was used instead of 1-1 to afford compound 6 (a white solid, 38 mg, yield 37%), ESI-MS: 512.2 m/z $[M+H]^+$.

Example 7

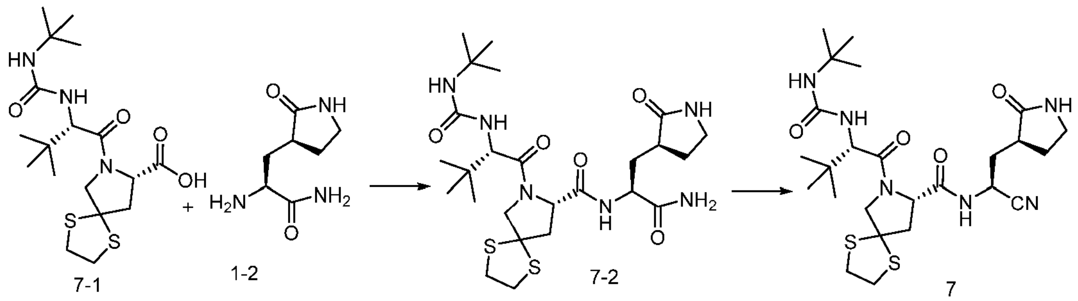

7-1 1-2 7-2 7

According to the same method in example 1, the difference lies in that: 7-1 was used instead of 1-1 to afford compound 7 (a white solid, 49 mg, yield 44%), ESI-MS: 553.3 m/z $[M+H]^+$.

Example 8

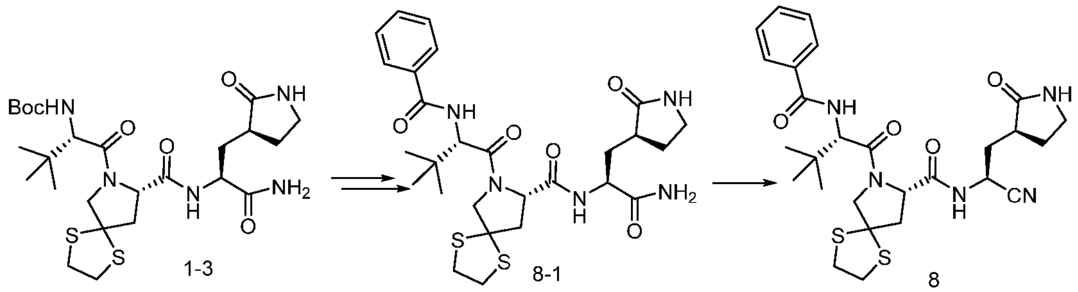

1-3 8-1 8

According to the same method in example 2, the difference lies in that: benzoic anhydride was used instead of trifluoroacetic anhydride to afford compound 8 (a white solid, 44 mg, yield 39%), ESI-MS: 558.3 m/z $[M+H]^+$.

Example 9

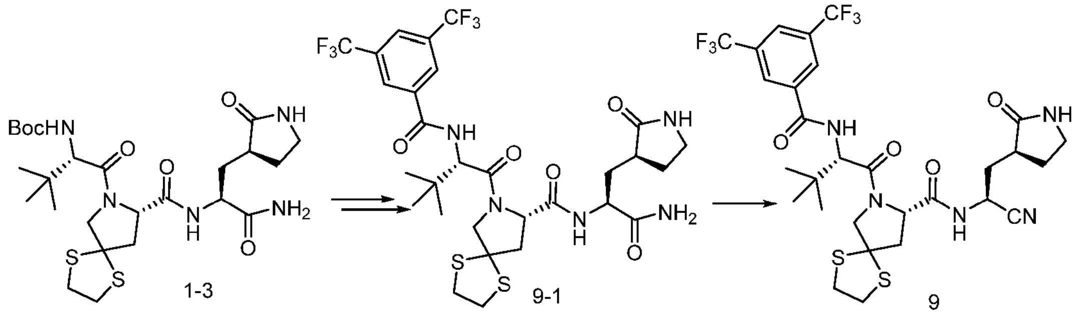

1-3 9-1 9

According to the same method in example 2, the difference lies in that: 3,5-bis(trifluoromethyl)benzoyl chloride was used instead of trifluoroacetic anhydride to afford compound 9 (a white solid, 39 mg, yield 28%), ESI-MS: 694.2 m/z $[M+H]^+$.

Example 10

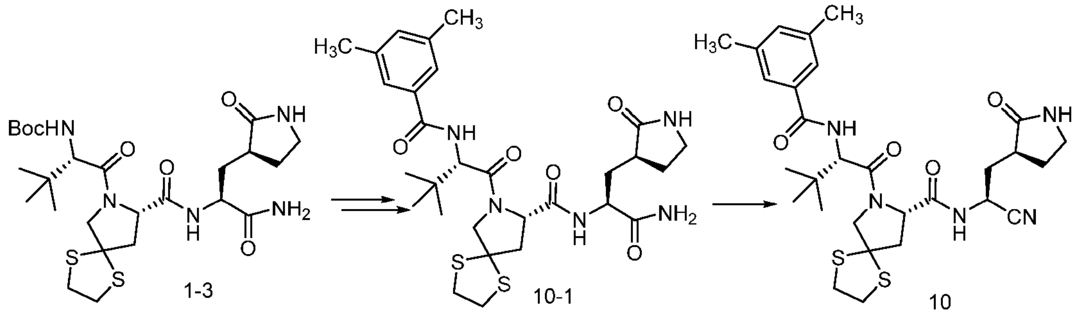

1-3 10-1 10

According to the same method in example 2, the difference lies in that: 3,5-dimethylbenzoyl chloride was used instead of trifluoroacetic anhydride to afford compound 10 (a white solid, 31 mg, yield 26%), ESI-MS: 586.2 m/z $[M+H]^+$.

Example 11

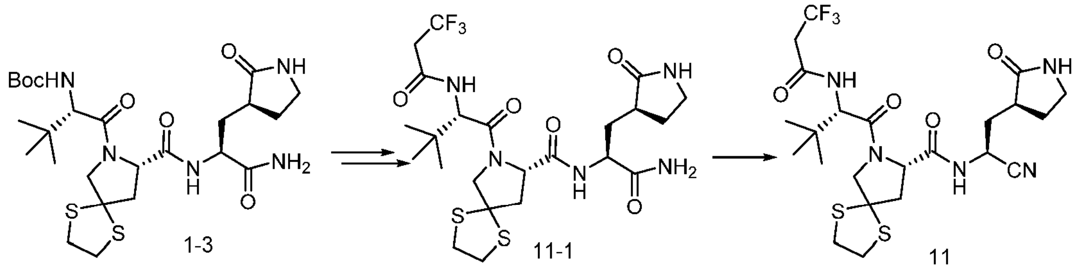

1-3 11-1 11

According to the same method in example 2, the difference lies in that: 3,3,3-trifluoropropionic anhydride was used instead of trifluoroacetic anhydride to afford compound 11 (a white solid, 28 mg, yield 25%), ESI-MS: 564.2 m/z $[M+H]^+$.

Example 12

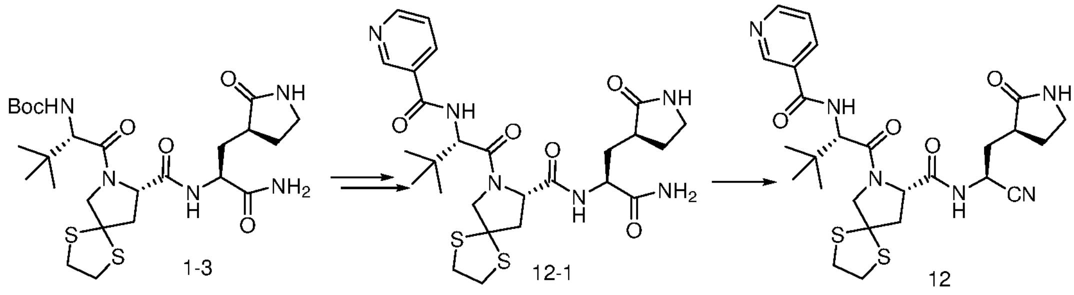

1-3 12-1 12

According to the same method in example 2, the difference lies in that: 3-pyridineformyl chloride was used instead of trifluoroacetic anhydride to afford compound 12 (a white solid, 39 mg, yield 35%), ESI-MS: 559.2 m/z $[M+H]^+$.

Example 13

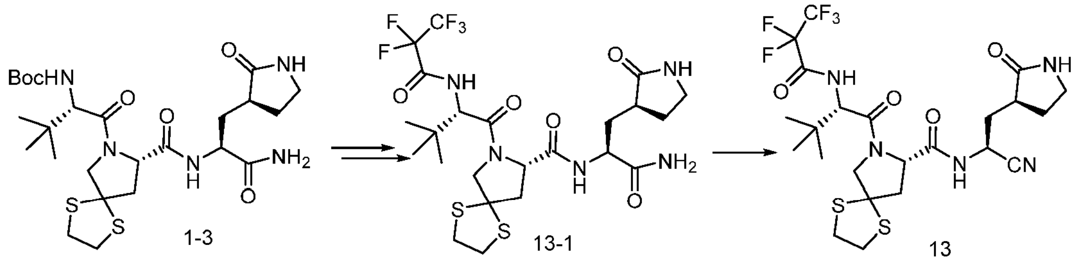

1-3 13-1 13

According to the same method in example 2, the difference lies in that: pentafluoropropionyl chloride was used instead of trifluoroacetic anhydride to afford compound 13 (a white solid, 41 mg, yield 34%), ESI-MS: 600.2 m/z $[M+H]^+$.

Example 14

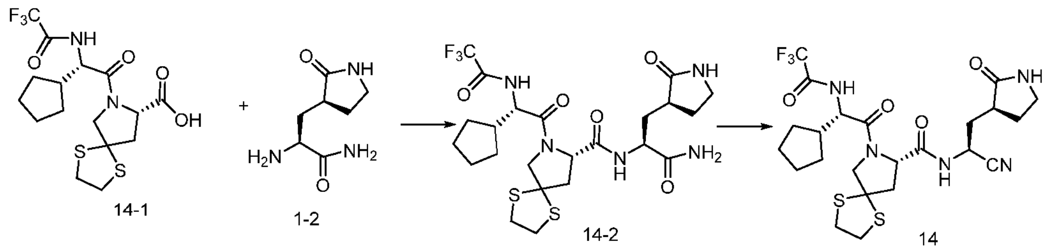

14-1 + 1-2 14-2 14

According to the same method in example 1, the difference lies in that: 14-1 was used instead of 1-1 to afford compound 14 (a white solid, 32 mg, yield 29%), ESI-MS: 550.2 m/z $[M+H]^+$.

Example 15

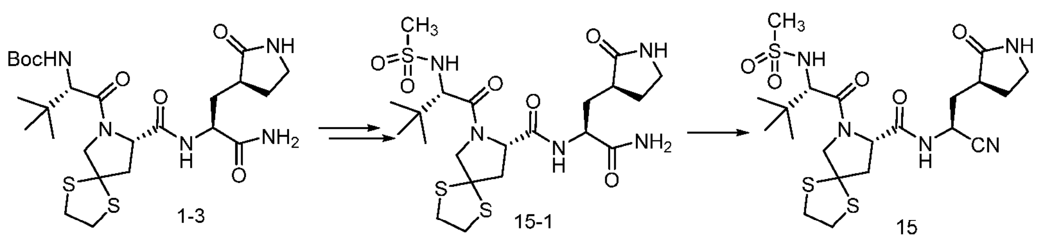

1-3 15-1 15

According to the same method in example 2, the difference lies in that: methanesulfonic anhydride was used instead of trifluoroacetic anhydride to afford compound 15 (a white solid, 38 mg, yield 36%), $^{1}H$ NMR (400 MHz, Methanol-$d_4$) $\delta$ 5.06 (dd, $J$ = 11.5, 4.5 Hz, 1H), 4.46 (dd, $J$ = 10.3, 7.1 Hz, 1H), 4.27 (dd, $J$ = 11.0, 1.6 Hz, 1H), 4.02-3.89 (m, 2H), 3.45 (qt, $J$ = 8.6, 4.7 Hz, 4H), 3.30-3.23 (m, 1H), 2.92 (s, 3H), 2.78 (tdd, $J$ = 10.3, 8.5, 4.0 Hz, 1H), 2.68 (ddd, $J$ = 13.0, 7.2, 1.6 Hz, 1H), 2.51 (dd, $J$ = 13.0, 10.3 Hz, 1H), 2.45-2.27 (m, 2H), 1.94-1.77 (m, 2H), 1.05 (s, 9H). ESI-MS: 532.2 m/z [M+H] $^{+}$.

Example 16

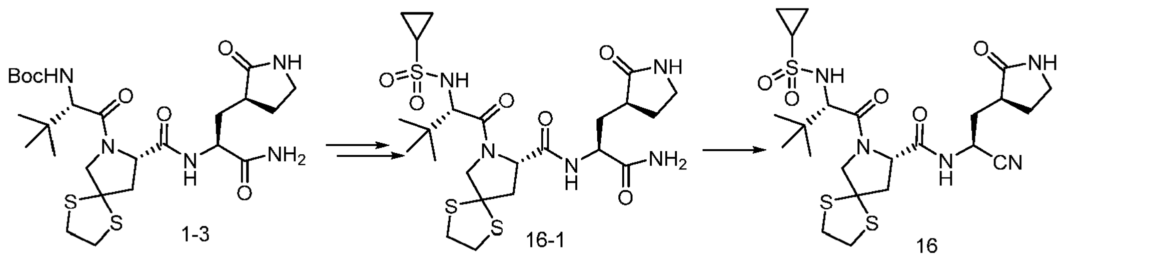

1-3 16-1 16

According to the same method in example 2, the difference lies in that: cyclopropanesulfonyl chloride was used instead of trifluoroacetic anhydride to afford compound 16 (a white solid, 29 mg, yield 26%), $^{1}H$ NMR (400 MHz, Methanol-$d_4$) $\delta$ 5.06 (dd, $J$ = 11.5, 4.5 Hz, 1H), 4.44 (dd, $J$ = 10.3, 7.0 Hz, 1H), 4.28 (dd, $J$ = 11.0, 1.6 Hz, 1H), 4.00-3.94 (m, 2H), 3.52-3.38 (m, 4H), 3.31-3.21 (m, 1H), 2.79 (tdd, $J$ = 10.4, 8.5, 4.1 Hz, 1H), 2.72-2.65 (m, 1H), 2.57 (ddd, $J$ = 7.9, 6.2, 3.9 Hz, 1H), 2.51 (dd, $J$ = 13.0, 10.4 Hz, 1H), 2.43-2.29 (m, 2H), 1.92-1.74 (m, 2H), 1.34-1.29 (m, 1H), 1.14 (qt, $J$ = 6.4, 3.8 Hz, 1H), 1.06 (s, 9H), 1.01 (ddt, $J$ = 7.9, 5.8, 2.7 Hz, 2H), 0.97-0.87 (m, 1H). ESI-MS: 558.2 m/z [M+H] $^{+}$.

Example 17

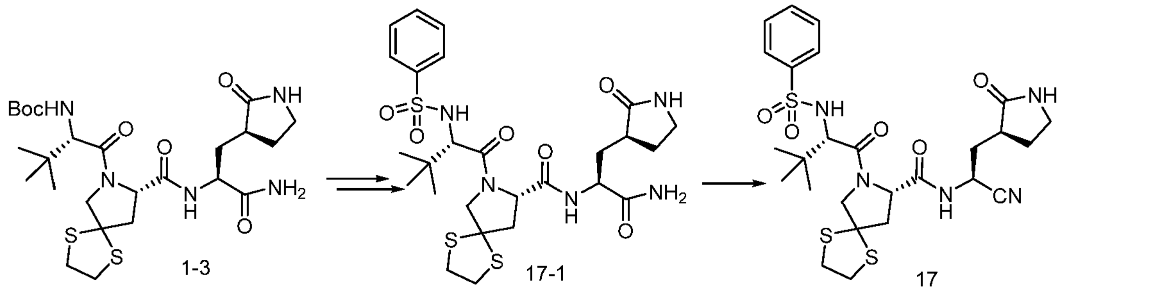

1-3 17-1 17

According to the same method in example 2, the difference lies in that: benzenesulfonyl chloride was used instead of trifluoroacetic anhydride to afford compound 17 (a white solid, 35 mg, yield 29%), ESI-MS: 594.2 m/z [M+H]$^{+}$.

Example 18

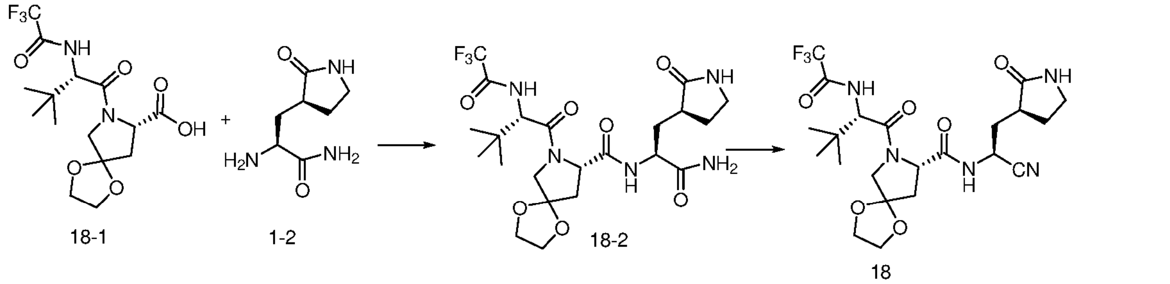

18-1 1-2 18-2 18

According to the same method in example 1, the difference lies in that: 18-1 was used instead of 1-1 to afford compound 18 (a white solid, 44 mg, yield 42%), ESI-MS: 518.2 m/z [M+H]+.

Example 19

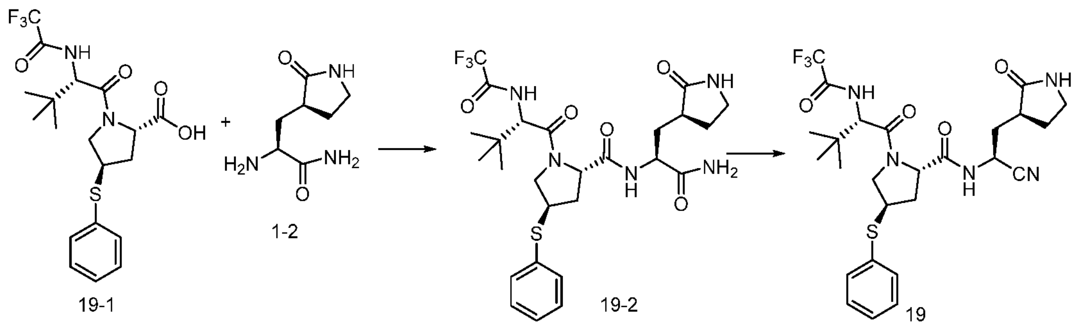

19-1 1-2 19-2 19

According to the same method in example 1, the difference lies in that: 19-1 was used instead of 1-1 to afford compound 19 (a white solid, 41 mg, yield 36%), ESI-MS: 568.2 m/z [M+H]+.

Example 20

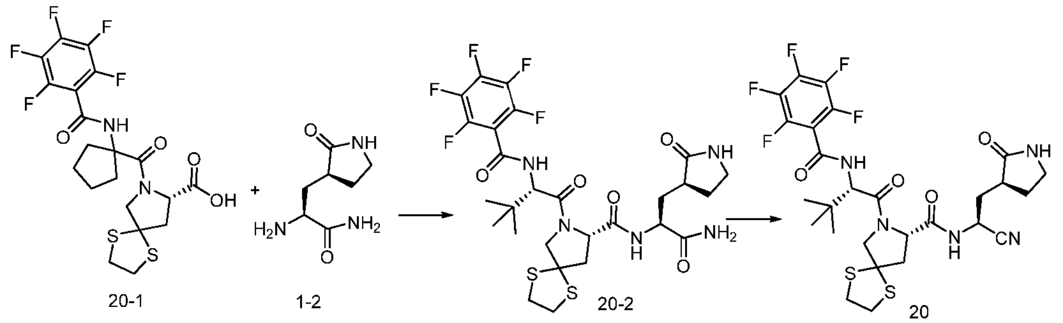

20-1 1-2 20-2 20

According to the same method in example 1, the difference lies in that: 20-1 was used instead of 1-1 to afford compound 20 (a white solid, 45 mg, yield 35%), ESI-MS: 648.2 m/z [M+H]+.

Example 21

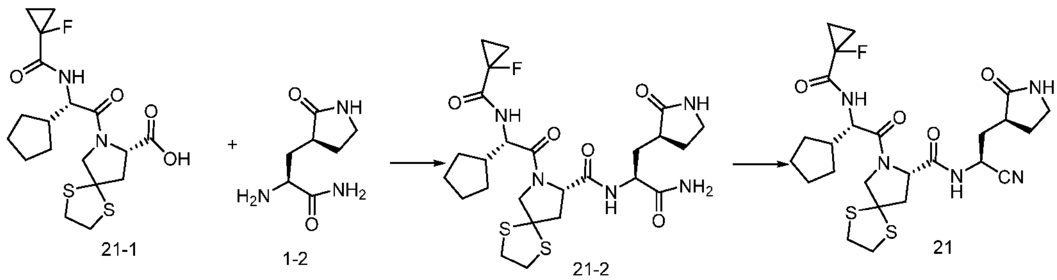

21-1 1-2 21-2 21

According to the same method in example 1, the difference lies in that: 21-1 was used instead of 1-1 to afford compound 21 (a white solid, 34 mg, yield 31%), ESI-MS: 640.2 m/z [M+H]+.

Example 22

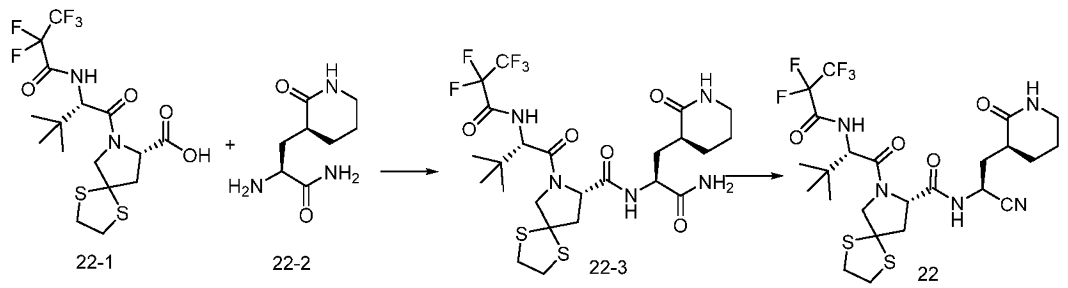

According to the same method in example 1, the difference lies in that: 22-1 was used instead of 1-1 and 22-1 was used instead of 1-2 to afford compound 22 (a white solid, 22 mg, yield 18%), ESI-MS: 614.2 m/z $[M+H]^+$.

Example 23

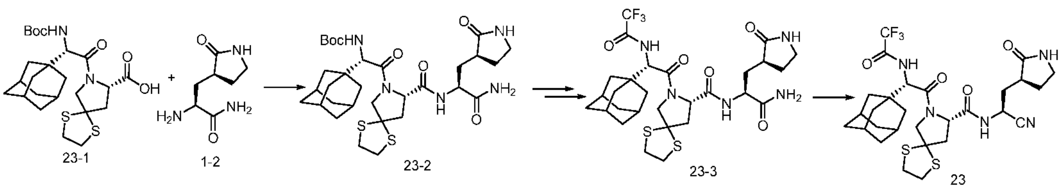

Compound 23-1 (497 mg, 1 mmol) was placed in a two-necked flask, and 5 mL of dichloromethane was added under nitrogen protection, and then 2-(7-azabenzotriazole)-N,N,N',N'-tetramethyluronium hexafluorophosphate (400 mg, 1.1 mmol) was added and stirred at room temperature for 1 h. Compound 1-2 (or a corresponding salt thereof, 1 mmol) was dissolved in 1 mL of dichloromethane and added to the above system, then N,N-diisopropylethylamine (0.5 mL, 1 mmol) was added under an ice-water bath, and the ice-water bath was removed. The system was stirred overnight at room temperature. Workup was performed, 50 mL of dichloromethane was added, washed with 1M aqueous hydrochloric acid three times, and washed with saturated aqueous sodium bicarbonate solution three times. The organic phase was washed with saturated saline solution, dried over anhydrous sodium sulphate, and spin-dried by filtration to afford compound 23-2 (a white solid, 421 mg, yield 65%). ESI-MS: m/z 650.3$[M+H]^+$.

Compound 23-2 (325 mg, 0.5 mmol) was dissolved in 1.5 mL of 4M hydrogen chloride/1,4-dioxane solution, or dissolved in 1 mL of dichloromethane, then 1 mL of trifluoroacetic acid was added dropwise and stirred at ambient temperature. Thin layer chromatography showed that the raw materials was substantially completely reacted, and then the solvent was fully spin-dried. The obtained crude product was dissolved in 2 mL of dichloromethane under nitrogen protection, and triethylamine (1.5 mmol) was added, then the system was placed in an ice-water bath, and trifluoroacetic anhydride (0.6 mmol) was added dropwise. Thin layer chromatography showed that the raw materials was substantially completely reacted, and then 25 mL of dichloromethane was added and washed three times with 1M aqueous hydrochloric acid solution and three times with saturated aqueous sodium bicarbonate solution. The organic phase was washed with saturated saline solution, dried over anhydrous sodium sulphate, and passed through a column to afford compound 23-3 (a white solid, 167 mg, yield 52%). ESI-MS: m/z 646.3$[M+H]^+$.

Compound 23-3 (130 mg, 0.2 mmol) and Burgess reagent (1.5 eq) were added to a two-necked flask, nitrogen was charged and discharged for three time, then dichloromethane dried over molecular sieves was added and stirred at room temperature overnight. Thin layer chromatography showed that the raw materials was substantially completely reacted. Workup was performed, column chromatography was performed to afford compound 23 (a white solid, 37 mg, yield 29%), ESI-MS: 628.2 m/z $[M+H]^+$.

Example 24: Test for inhibitory activity of compounds on SARS-CoV-2 3CLpro

The inhibitory activity of the tested compounds on the enzymatic activity of SARS-CoV-2 $3CL^{pro}$ was evaluated by fluorescence resonance energy transfer method. The volume of the entire enzymatic reaction system is 120 μL, the final concentration of the protease is 30 nM, and the final concentration of the substrate is 20 μM. The buffer of the reaction system includes 50 mM Tris pH 7.3, 1 mM EDTA. The SARS-CoV-2 $3CL^{pro}$ protease and different concentrations of the compounds were added to a 96-well plate, incubated at 30°C for 10 min, and then the substrate was added and the plate

was quickly put into a microplate reader for reading. The excitation and emission light were 320 nM and 405 nM, respectively. The test time was 3.5 min, and the fluorescence value was read every 35 s. In the final result, the readings of the first 2 min were used for fitting to obtain the reaction rate, and compared with the control group (DMSO), the inhibition rate was calculated. The $IC_{50}$ value and the inhibition rate curve were obtained by fitting using the software GraphPad Prism 8.

The experimental results are shown in Table 1. The results show that the compounds of the present invention have potent inhibitory effects on SARS-CoV-2 $3CL^{pro}$. The range of $IC_{50}$ value is that: A means < 0.1 μM, B means 0.1-1 μM, C means 1-10 μM.

Table 1: Inhibitory effects of compounds **1-23** on SARS-CoV-2 $3CL^{pro}$

| Compound number | Compound structural formula | $IC_{50}$ (μM) |
|---|---|---|
| 1 | 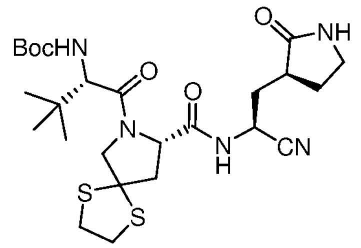 | A |
| 2 | 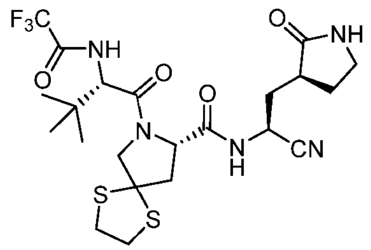 | A |
| 3 | 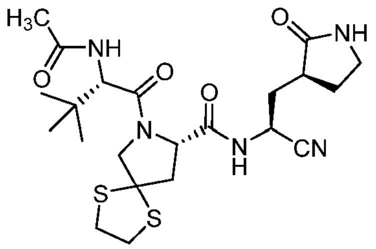 | A |
| 4 | 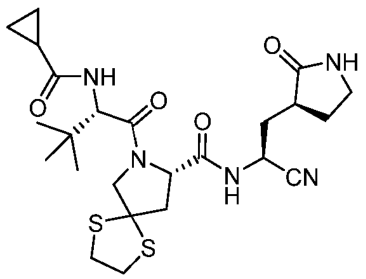 | A |
| 5 | 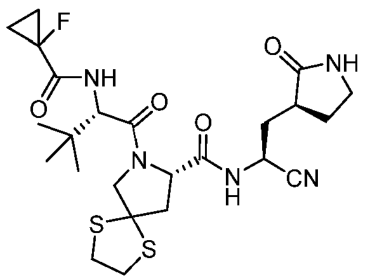 | A |

(continued)

| Compound number | Compound structural formula | IC50 (μM) |
|---|---|---|
| 6 | 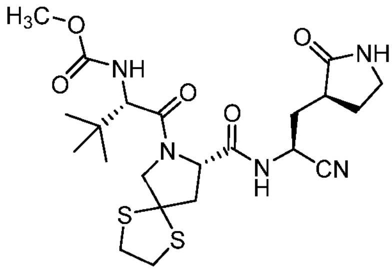 | B |
| 7 | 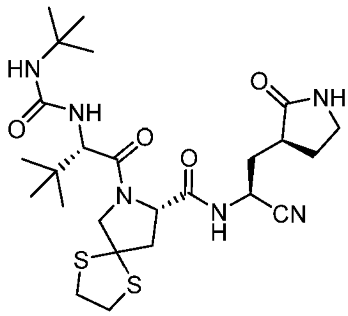 | A |
| 8 | 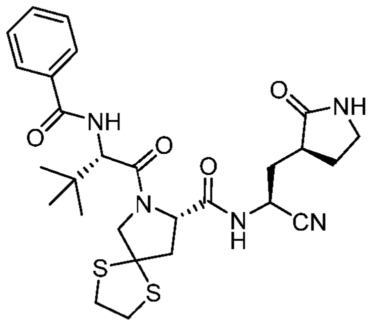 | C |
| 9 | 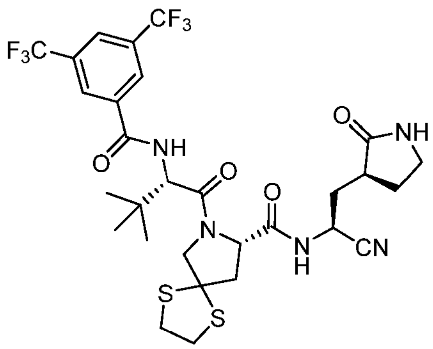 | C |
| 10 | 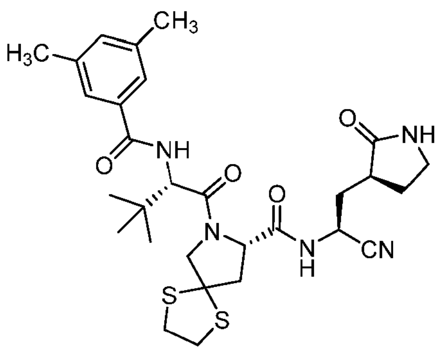 | C |

(continued)

| Compound number | Compound structural formula | $IC_{50}$ (μM) |
|---|---|---|
| 11 | 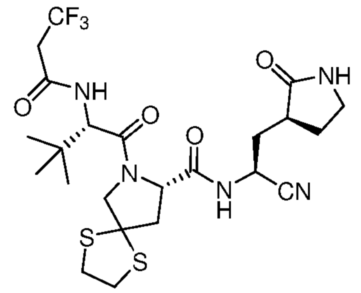 | B |
| 12 | 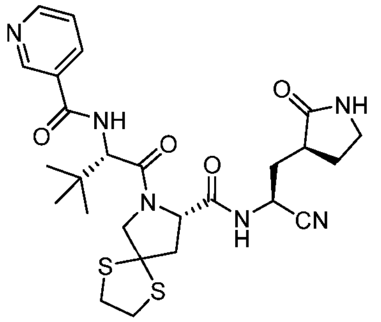 | C |
| 13 | 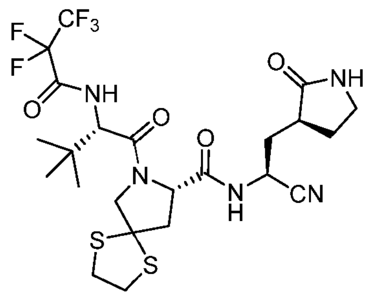 | A |
| 14 | 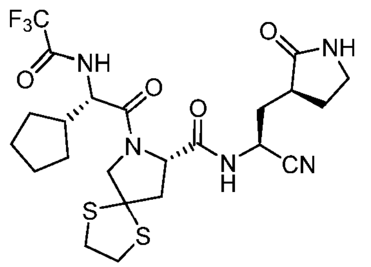 | A |
| 15 | 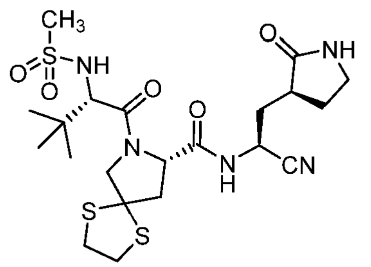 | A |

(continued)

| Compound number | Compound structural formula | $IC_{50}$ (μM) |
|---|---|---|
| 16 | 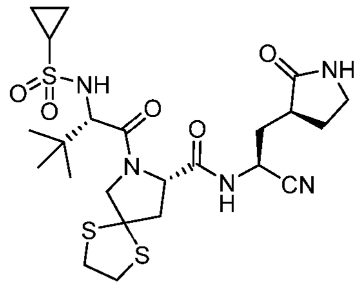 | A |
| 17 | 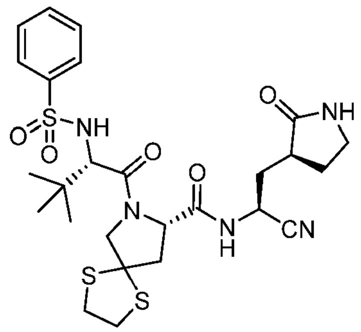 | C |
| 18 | 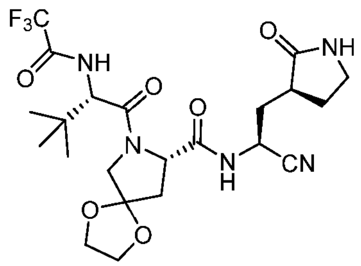 | A |
| 19 | 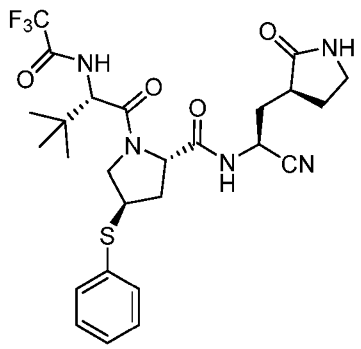 | C |
| 20 | 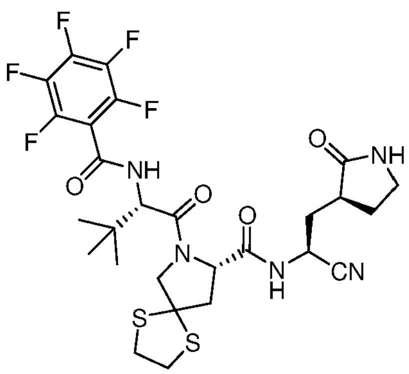 | B |

(continued)

| Compound number | Compound structural formula | $IC_{50}$ (μM) |
|---|---|---|
| 21 | 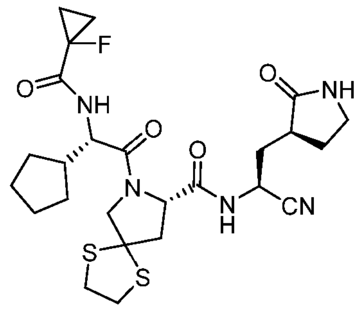 | A |
| 22 | 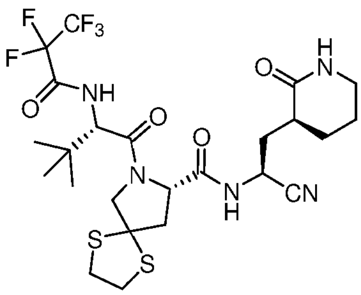 | A |
| 23 | 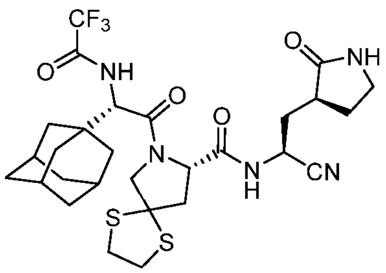 | A |

Example 24-2: Test for inhibitory activity of compound 2 on mutant 3CL protease of SARS-CoV-2 Omicron strain

Experimental principle: The inhibitory effects of the compounds of the invention on the activity of the mutant 3CL protease (P132H) of the Omicron strain were studied by using a method for generate fluorescence resonance energy transfer (FRET) by the reaction of enzyme and substrate.

The experimental materials are shown in the table below:

| Reagent material | Brand | Catalog number: |
|---|---|---|
| 3CL protease-P132H mutant | Shanghai Profession and Creation Co. , Ltd. | / |
| Dabcyl-KTSAVLQSGFRKME-Edans (Coronavirus main protease fluorescent substrate) | Beyotime | P9733 |
| DTT (Dithiothreitol) | Invitrogen | P2325 |
| BSA (Bovine Serum Albumin) | Sigma | V900933 |
| EDTA (ethylenediaminetetraacetic acid) | Invitrogen | AM9260G |
| Tris-HCl (trishydroxymethylaminomethane) | Sangon Biotech | B548127 |

Experimental instrument and equipment:

| Equipment name | Brand | Model |
|---|---|---|
| Echo Nanoliter-Scale Sonic Shifting System | Labcyte | Echo 650 |
| Flexstation 3 microplate reader | MolecMar Devices | FLEX3 |
| Centrifuge | Eppendorf | 5810 |

Experimental steps:

Prepare a reaction buffer containing 20 mM Tris-HCl, 1 mM EDTA, 0.01% BSA, 1 mM DTT, and 100 mM NaCl. The compounds to be tested were diluted to different concentrations in dimethyl sulfoxide (DMSO) with the Echo pipetting system and transferred to a 384-well plate. The mutant 3CL protease was diluted with the reaction buffer, and added to the 384-well plate at an amount of 10 μL/well, centrifuged at 1000 rpm for 1 min, and then incubated at room temperature for 30 minutes. Then the substrate was added at 10 μL/well, and centrifuged at 1000 rpm for 30 s to start the enzyme reaction. In the reaction system, the final concentration of the enzyme was 50 nM, the final concentration of the substrate was 20 μM, and the concentration of the compound ranged from 10000 nM to 0.51 nM. Then the Kinetic Reduction Vmax mode on the Flexstation 3 microplate reader was selected, the fluorescence value at 490 nm wavelength was continuously read every 75 seconds, a total of 35 times, to obtain the reaction rate value (V), and the inhibition rate was calculated, and XLfit software was used to perform four-parameter fitting to obtain the half inhibitory concentration ($IC_{50}$). The inhibition rate calculation method is as follows:

$$\text{Inhibition rate} = (V_{max}-V_{compound})/(V_{max}-V_{min})*100\%$$

wherein $V_{max}$ is the reaction rate value for a well containing only the enzyme and the substrate, $V_{min}$ is the reaction rate value for a well containing only the substrate, and $V_{compound}$ is the reaction rate value for a well containing the compound to be tested, the enzyme and the substrate.

Experimental results: For the 3CL protease with P132H mutation in the SARS-CoV-2 Omicron strain, compound 2 still maintained a significant inhibitory activity ($IC_{50}$ of three independent tests was 0.022 ± 0.00090 μM).

**Table 2: Inhibitory effect of compound 2 on 3CL protease activity of SARS-CoV-2 Omicron strain**

| 3CL protease | Compound 2 $IC_{50}$ (μM) (n = 3*) |
|---|---|
| SARS-CoV-2 (Omicron strain) | 0.022 ± 0.00090 |
| * refers to three independent replicate experiments. | |

Example 24-3: Test for inhibitory activity of compound 2 on 3CL protease of coronavirus from different sources

Experiment purpose: to study the inhibitory effect of compound 2 on the 3CL protease activity derived from six other coronaviruses capable of infecting humans. These six viruses are: SARS-CoV, MERS-CoV, OC43-CoV, H229E-CoV, NL63-CoV, and HKU1-CoV.

Experimental materials:

3CL protease: A recombinant full-length coronavirus 3CL protease was made in house according to the coronavirus genome sequence, the genome GenBank numbers of SARS-CoV, MERS-CoV, H229E-CoV, HKU1-CoV, NL63-CoV and OC43-CoV used therein are AAP13442.1, MT387202.1, AF304460.1, AY597011.2, AY567487.2 and AY903459.1, respectively, and the DNA sequences required for the expression of the 3CL protease proteins of the six coronaviruses were purchased from Nanjing GenScript Biotechnology Co., Ltd.

The 3CL protease substrate was purchased from Nanjing GenScript Biotechnology Co., Ltd.

The chymotrypsin substrate was purchased from GL Biochemical Co., Ltd.

Other reagents are listed in the table below:

| Reagent material | Brand | Catalog number: |
|---|---|---|
| Chymotrypsin derived from bovine pancreas | Sigma | C4129 |
| Tris | Sigma | BCBX3837 |

(continued)

| Reagent material | Brand | Catalog number: |
|---|---|---|
| EDTA | Sigma | 0001434776 |

Experimental steps:

A reaction buffer (containing 50 mM Tris and 1 mM EDTA) was formulated. The compound to be tested was dissolved in DMSO to prepare a 100 mM stock solution, and further 2-fold serially diluted with the reaction buffer, with a total of 11 concentrations. The 3CL protease and different concentrations of the compounds were added to a 96-well plate, incubated at room temperature for 10 minutes, and then the substrate was added and the plate was quickly put into a microplate reader for reading. The volume of the entire enzymatic reaction system was 120 μL, and the final concentrations of SARS-CoV, MERS-CoV, H229E-CoV, HKU1-CoV, NL63-CoV and OC43-CoV proteases were 30 nM, 80 nM, 30 nM, 20 nM, 30 nM and 10 nM respectively, and the final concentration of the substrate was 10 μM. The excitation and emission wavelengths were 340 nm and 490 nm, respectively, when reading. The test time was 10 minutes, and the fluorescence value was read every 1 minute. In the final result, the readings of the first 5 min were used for fitting to obtain the reaction rate, the inhibition rate was calculated and the calculation formula is: Inhibition Rate = 1 - (Reaction Rate of Test Group/Reaction Rate of Control Group).

Experimental results: The results are shown in Table 3. Compound 2 exhibited a good inhibitory effect on 3CL proteases derived from other six coronaviruses, suggesting that compound 2 may have a broad-spectrum anti-coronavirus activity.

**Table 3: Inhibitory effect of compound 2 on 3CL proteases from other coronaviruses**

| 3CL protease source | Compound 2 $IC_{50}$ (μM) |
|---|---|
| HKU1-CoV | 0.0049 |
| OC43-CoV | 0.010 |
| SARS-CoV-1 | 0.024 |
| MERS-CoV | 0.060 |
| H229E-CoV | 0.13 |
| NL63-CoV | 0.85 |

Example 25-1: Inhibitory effect of compound 2 on SARS-CoV-2 WIV04 and B.1.351 strains at cell level

Vero E6 cells were used in the test, and Vero E6 cells (50,000 cells/well) were added to a 48-well plate, a medium containing gradient concentrations of the compounds was added at 100 μL/well, and SARS-CoV-2 was added one hour later, the multiplicity of infection (MOI) was 0.01. After co-incubating for 1 hour, the supernatant was aspirated, the residue was washed and the medium containing gradient concentrations of the compounds was added again at 200 μL/well, and incubated at 37°C for 24 hours. After 24 hours, the cell supernatant was collected, the viral RNA in the supernatant was extracted, and the virus copy number in the supernatant was determined by real-time fluorescent quantitative PCR. The compound inhibition rate was calculated according to the virus copy number, and the $EC_{50}$ of the compound was calculated by prism 6.0.

The test results showed that the compound **2** inhibited the SARS-CoV-2 WIV04 strain with a half effective concentration $EC_{50}$ of 0.57 +/- 0.04 μM, and inhibited the SARS-CoV-2 B.1.351 strain with $EC_{50}$ of 0.73 +/- 0.06 μM, and the $EC_{50}$ curves are shown in FIGs 1 and 2.

Example 25-2: Inhibitory effect of compound 2 on SARS-CoV-2 Vero E6 original strain (WIV04), Delta strain (B.1.617.2), and Omicron strain (B.1.1.529) at cell level

Experiment purpose: In this experiment, the method of determining the virus copy number in the culture supernatant by real-time fluorescent quantitative PCR in Vero E6 cells was used to study the inhibitory effect of compound 2 on SARS-CoV-2 original strain (WIV04 strain), Delta strain (B.1.617.2), and Omicron strain (B.1.1.529) in cell replication. Since Vero E6 cells highly expressed the efflux transporter P-gp, 0.5 μM of a P-gp inhibitor CP-100356 was added and incubated with the compound.

Experimental materials:

Vero E6 was purchased from ATCC (Catalog number: CRL-1586), SARS-CoV-2 original strain (SARS-CoV-2-WIV04 strain), Delta strain (B.1.617.2), Omicron strain ((B.1.1.529) were obtained from the Microbial Bacteria (Virus) Species Collection Centre of Wuhan Institute of Virology, Chinese Academy of Sciences.

Other reagents are listed in the table below:

| Reagent name | Brand | Catalog number: |
|---|---|---|
| TaKaRa MiniBEST Viral RNA/DNA Extraction Kit Ver.5.0 | Takara | 9766 |
| TaKaRa PrimeScript™ RT reagent Kit with gDNA Eraser | Takara | RR047A |
| TaKaRa SYBR® Premix Ex Taq ™ II | Takara | RR820A |
| Fetal Bovine Serum | Gibco | R2768 |
| DMEM medium | Gibco | C11995500BT |
| CCK8 | Beyotime | C0039 |
| Chloroquine Phosphate | SIGMA | C6628-50G |
| Trypsin | BIOSHARP | BL512A |

Experimental instruments:

Biosafety cabinet (AC2-3S1, ESCO, Singapore)
Carbon dioxide incubator (Thermo Scientific HERAcell 150i, Thermo Scientific, USA)
Pure water equipment (ShenYuan SYS ultrapure water machine, Chengdu)
StepOne Plus Real-time PCR system (4376600, ABI, USA)
TC20™ Automated Cell Counter (1450102, BIO-RAD, USA)
T100™ Thermal Cycler (1861096, BIO-RAD, USA)
Centrifuge (Micro21/21R Thermo, Thermo Scientific, USA)

Experimental steps:

The Vero E6 cells were digested with trypsin and placed in a culture medium (90% DMEM, 10% fetal calf serum), inoculated into a 48-well plate at 50,000 cells per well, and cultured overnight. The compound to be tested was dissolved in DMSO to prepare a 40 mM stock solution, and further serially diluted with a medium containing 0.5 μM of Pgp inhibitors to obtain the required concentration. The final concentration of the compound to be tested in the experiment ranged from 1 μM to 0.004 μM. The cell supernatant was removed, the diluted compound (containing Pgp inhibitor 0.5 μM) was added to each well, incubated for 1 h. Different strains of SARS-CoV-2 were added in a biosafety level 3 (BSL-3) laboratory, and the multiplicity of infection (MOI) was 0.01 or 0.001. After incubation for 1 h, the supernatant was removed, the residue was washed with PBS, the diluted compound (containing Pgp inhibitor 0.5 μM) was added at 200 μL/well, and the supernatant was collected 24 or 72 hours after infection. The virus RNA in the supernatant was extracted and real-time fluorescence quantitative PCR was used to determine the virus copy number in the supernatant, the compound inhibition rate was calculated according to the virus copy number, and GraphPad Prism 8 was used to calculate the $IC_{50}$ of the compound.

In the cytotoxicity test, Vero E6 cells were digested and placed in a culture medium (90% DMEM, 10% fetal bovine serum), inoculated into a 96-well plate at 20000 cells per well, and cultured overnight. The compound to be tested was dissolved in DMSO to prepare a 40 mM stock solution, and further serially diluted with a culture medium or a culture medium containing 0.5 μM Pgp inhibitor to obtain the concentration required for the test. The final concentration of the compound to be tested in the experiment ranged from 500 μM to 1.95 μM. The cell supernatant in the 96-well plate was removed, the medium for the compound to be tested (as a monotherapy or containing 0.5 μM of a Pgp inhibitor) was added at 100 μL/well and incubated for 24 h; the CCK8 detection kit was used to determine cell viability, and the inhibition rate and median cytotoxic concentration ($CC_{50}$) were calculated.

Test results: As shown in Table 4, when combined with the P-gp inhibitor CP-100356, compound 2 can dose-dependently inhibit the replication of delta strain in Vero E6 cells with an $IC_{50}$ of 0.040 μM. In the original strain, compound 2 combined with the P-gp inhibitor also exerted a strong inhibitory effect, with an $IC_{50}$ of 0.027 μM. In addition, compound 2 combined with the P-gp inhibitor can significantly inhibit the replication of Omicron strain in Vero E6 cells, with an $IC_{50}$ of 0.12 μM. Compound 2 alone and in combination with the P-gp inhibitor had no obvious cytotoxicity on the proliferation of Vero E6 cells, $CC_{50}$ > 500 μM.

**Table 4: Inhibitory effect of compound 2 combined with P-gp inhibitor on SARS-CoV-2 replication in Vero E6 cells**

| Experiment | Compound 2 + 0.5 μM CP-100356 | |
|---|---|---|
| | $IC_{50}$ (μM) | Select Index SI SI = ($CC_{50}/IC_{50}$) |
| Vero E6 original strain (WIV04) | 0.027 (mean, n = 2) | > 18519 |
| Vero E6 delta strain (B.1.617.2) | 0.040 (mean, n = 2) | > 12500 |
| Vero E6 Omicron strain (B.1.1.529) | 0.12 | > 4167 |
| Vero E6 monotherapy $CC_{50}$ | > 500 μM | |
| Vero E6 combined with P-gp inhibitor $CC_{50}$ | > 500 μM | |

Example 26: *In vivo* antiviral effect of compound 2 on SARS-CoV-2 delta strain in hACE2-K18 transgenic mice
Experiment purpose: This study evaluated the antiviral activity of compound 2 on the SARS-CoV-2 delta strain in K18 transgenic mice stably expressing human angiotensin-converting enzyme 2 (ACE2) (K18-hACE2).

Experimental materials:

7-8 week-old K18-hACE2 transgenic mice were purchased from Jiangsu Jicui Gempharmatech Co., Ltd. The SARS-CoV-2 delta strain virus was obtained from the Microbial Bacteria (Virus) Species Collection Centre of Wuhan Institute of Virology, Chinese Academy of Sciences.
Ritonavir was purchased from Shanghai Desano Inc.
Vero E6 cells were purchased from ATCC (Catalog number: CRL-1586).
Other reagents are listed in the table below:

| Reagent name | Brand | Catalog number: |
|---|---|---|
| Qiagen 74106 RNeasy Mini Kit | Qiagen | 74106 |
| TaKaRa PrimeScript™ RT reagent Kit with gDNA Eraser | Takara | RR047A |
| TaKaRa SYBR® Premix Ex Taq ™ II | Takara | RR820A |
| Fetal Bovine Serum | Gibco | 10099-141C |
| DMEM medium | Gibco | C11995500BT |
| Tissue fixative | BOSTER | AR1068 |
| Aquacide II (sodium methylcellulose) | Millipore | 17851 |
| DMEM medium (powder, high sugar) | Gibco | 12100046 |
| Purple crystal | Sinopharm Group | 71012314 |

Experimental instruments:

Biosafety cabinet (AC2-3S1, ESCO, Singapore)
Carbon dioxide incubator (Thermo Scientific HERAcell 150i, Thermo Scientific, USA)
Pure water equipment (ShenYuan SYS ultrapure water machine, Chengdu)
StepOne Plus Real-time PCR system (4376600, ABI, USA)
TC20™ Automated Cell Counter (1450102, BIO-RAD, USA)
T100™ Thermal Cycler (1861096, BIO-RAD, USA)
Centrifuge (Micro21/21R Thermo, Thermo Scientific, USA)
Tissue grinder (JXFSTPRP-CL, Shanghai Jingxin Industrial Development Co., Ltd.)

Experimental steps: K18-hACE2 transgenic mice were infected with the delta strain of SARS-CoV-2 by intranasal drops on day 0. 2 hours after infection, the vehicle and compound 2 at 50 mg/kg or 200 mg/kg (combined with cytochrome P450 inhibitor ritonavir at 50 mg/kg) were administered by gavage, BID for 2 days (wherein 1 dose was

administrated on day 0, 2 doses on day 1, 1 dose on day 2) or 4 days (wherein 1 dose was administrated on day 0, and 2 doses on the day 1, day 2, and day 3, respectively). The body weight changes of the mice were recorded, and lung and brain tissues were collected at the end point. Among them, the left lung was fixed in formaldehyde, and then used for histopathological examination after embedding, sectioning and H&E staining. The right lung and brain tissue were each divided into two parts. One part was ground and the homogenate was taken to extract RNA which was then reverse-transcribed, and the virus copy number was determined by real-time fluorescent quantitative PCR. The other part was ground and the homogenate was taken to determine the virus titre by plaque test. The plaque test method is as follows: Vero E6 cells were inoculated in a 24-well plate at 12,000 cells per well and cultured overnight. The stock of tissue homogenate was 10-fold serially diluted in DMEM medium for later use. The cell supernatant was removed, add the diluted tissue homogenate was added and incubated for 1 h, then the supernatant was removed, the medium containing 1% sodium methylcellulose and 2% FBS was added, and cultured for 4 days. Then the medium was removed, paraformaldehyde was used for fixation, 1% (w/v) crystal violet was used for staining, and the number of plaques in each well was counted.

Test results: As shown in Table 5, 2 days after infection, compared with the model group (the average virus copy number was 9.19 ± 0.30 log10 copies/g), in the case of being combined with ritonavir, compound 2 at 50 mg/kg and 200 mg /kg significantly reduced the viral load in the lung, with an average copy number of 7.66 ± 0.27 log10 copies/g and 6.79 ± 0.30 log10 copies/g, respectively, wherein the virus copy number decreased by 2.4 log10 copies/g at the dose of 200 mg/kg. 4 days after infection, a sustained inhibitory effect of compound 2 on viral copy number was observed. In terms of virus titre, as shown in FIG. 3, a significant inhibitory effect of compound 2 was observed. 2 days after infection, the virus replication was completely inhibited at a dose of 200 mg/kg, and no titre was detected. Compared with the model group at a dose of 50 mg/kg, the virus titre decreased by more than 3 log10 PFU/g, and compound 2 showed a sustained inhibitory effect on the virus titre 4 days after infection. In terms of body weight, as shown in FIG. 4, 4 days after infection, the body weight of the mice in the model group lost about 10%, while the body weight of the compound 2 administration group did not decrease significantly, indicating that compound 2 did not show obvious toxicity under continuous administration. We further determined the viral load in the brains of the mice, and 2 days after infection, no obvious infection occurred in each group. 4 days after infection, compared with the model group, compound 2 could significantly reduce the virus copy number in the brains of the mice at doses of 50 mg/kg and 200 mg/kg, especially at the dose of 200 mg/kg, the virus copy number was comparable to that of the uninfected normal group. We further determined the virus titre in the brain 4 days after infection, and the results are shown in FIG. 5. Compared with the model group, no virus titre was determined at the two doses of compound 2, showing a strong inhibitory effect of compound 2. In addition, the histopathological analysis of the lung showed that compared with the model group, compound 2 at a dose of 200 mg/kg significantly improved lung damage, including reducing the degree of alveolar atrophy or dilation and the degree of alveolar membrane thickening.

**Table 5 Viral loads in the lungs and brains of mice 2 and 4 days after infection (mean ± SD)**

| Group | Lung tissue (log10 copies/g) | | Brain tissue (log10 copies/mg) | |
|---|---|---|---|---|
| | Day 2 | Day 4 | Day 2 | Day 4 |
| Model group | 9.19 ± 0.30 | 9.35 ± 0.30 | 1.34 ± 0.78 | 7.17 ± 0.27 |
| Compound 2 - 200 mg/kg + Ritonavir - 50 mg/kg | 6.79 ± 0.30 | 7.48 ± 0.63 | 1.04 ± 0.24 | 0.99 ± 0.42 |
| Compound 2 - 50 mg/kg + Ritonavir - 50 mg/kg | 7.66 ± 0.27 | 7.88 ± 0.74 | 0.77 ± 0.20 | 2.88 ± 1.26 |
| Normal group | 5.76 ± 0.32 | 6.27 ± 0.18 | 1.08 ± 0.06 | 1.08 ± 0.06 |

Example 27: Selectivity of compound 2 for kinases

Experiment purpose: The inhibitory activity of compound 2 on 413 kinases was determined on the KinaseProfile experimental platform to study the selectivity of compound 2 on kinases.

Experimental materials:

Full Human Panel [10 uM ATP] KinaseProfiler is a test product provided by Eurofins, Catalog number: 50-005KP10, this product contains 413 kinases.

Experimental steps:

Each kinase selected was tested with the compound using the Eurofins standard KinaseProfiler analysis method and following the relevant standard operating procedures. Protein kinases were detected by radioactive method, while

lipid kinases were detected by HTRF method. The ATP concentration in the experiment was 10 μM. Detailed information on each kinase is available on the Eurofins website at: https://www.eurofinsdiscoveryservices.com/catalogmanagement/viewItem/Full-Human-Panel-10-uM-ATP-KinaseProfiler/50-005KP10.

Experimental results: For the 413 kinases, the inhibition rate of compound 2 was less than 30% at a concentration of 10 μM, showing no significant inhibitory effect, suggesting that compound 2 had excellent selectivity.

Example 28: Selectivity of compound 2 for safety targets

Experiment purpose: The effect of compound 2 on 47 safety-related targets was determined on the Safetyscan experimental platform.

Experimental materials:

Safety47 Panel Dose Response SAFETY scan is a test product provided by Eurofins, Catalog number: 87-1003DR. This product contains 78 tests related to 47 safety targets.

Experimental steps:

For the 78 tests related to the 47 safe targets, the experimental methods used include: cAMP assay, calcium flux assay, hormone nuclear receptor assay, kinase binding assay, enzyme activity assay, neurotransmitter transporter assay, ion channel assay, and transporter assay. Specific methods for each experiment are available on the eurofins website at the following URL: https://www.eurofinsdiscoveryservices.com/catalogmanagement/viewItem/Safety4 7-Panel-Dose-Response-SAFETYscan-DiscoverX/87-1003DR.

Experimental results: For 47 safety-related targets, compound 2 had no significant inhibitory or activating effect at a concentration of 100 μM ($EC_{50}$ were all greater than 100 μM), suggesting that compound 2 had excellent selectivity.

Example 29: Human plasma protein binding assay of compound 2

**Experimental materials**

Human plasma was purchased from BioIVT, anticoagulated with EDTA K2, and stored at -80°C. 96-well equilibrium dialysis plates were purchased from HTDialysis LLC. Equilibrium dialysis membranes were purchased from Gales Ferry.

**Experimental procedure**

An alkaline solution with 14.2 g/L of disodium hydrogen phosphate and 8.77 g/L of sodium chloride was formulated with ultrapure water, and the alkaline solution can be stored at 4°C for 7 days. An acidic solution with 12.0 g/L of sodium dihydrogen phosphate and 8.77 g/L of sodium chloride was formulated with ultrapure water, and the acidic solution can be stored at 4°C for 7 days. The alkaline solution was titrated with an acidic solution to a pH of 7.4, and the buffer can be stored at 4°C for 7 days. The pH value of the buffer was measured on the day of the experiment, and the pH value was adjusted if it exceeded the range of 7.4 ± 0.1.

The dialysis membrane was soaked in ultrapure water for 60 minutes to separate the membrane into two pieces, then soaked in 20% ethanol for 20 minutes, and finally soaked in the buffer used for dialysis for 20 minutes.

The frozen plasma was thawed rapidly at room temperature.

The plasma was centrifuged at 3,220 g for 10 min at 4°C to remove clots, and the supernatant was collected into a new centrifuge tube. The pH of the plasma was measured and recorded.

A 10 mM stock solution of the analyte was formulated in DMSO. 2 μL of stock solution (10 mM) was diluted with 98 μL of DMSO to obtain a working solution (200 μM). 3 μL of the working solution was taken and 597 μL of human plasma was added to obtain a final concentration of 1 μM (0.5% DMSO). The mixture was fully vortexed.

120 μL of plasma sample containing the drug was added to one side of the dialysis membrane, and an equal volume of dialysate (phosphate buffer) was added to the other side thereof. The experiment was performed in parallel duplicate. The dialysis plate was sealed, put into the incubation device, and incubated at 37°C, 5% $CO_2$ and about 100 rpm for 6 hours. After the completion of the incubation, the sealing film was removed and 50 μL was pipetted from the buffer and plasma sides in each well, respectively, into separate wells of a new plate.

50 μL of blank plasma was added to the phosphate buffer sample, and an equal volume of blank phosphate buffer was added to the plasma sample. 300 μL room temperature quencher (containing internal standard acetonitrile (IS, 500 nM labetalol, 100 nM alprazolam and 2 μM ketoprofen)) was added to precipitate proteins. The resulting mixture was

vortexed for 5 minutes and centrifuged at 3220 g for 30 min at 4°C, and 100 μL of the supernatant was transferred to a new plate. According to the LC/MS response signal and peak shape of the analyte, the supernatant was diluted with 100 μL or 200 μL of water and mixed evenly and the sample was analysed using LC/MS.

All calculations were performed by Microsoft Excel. The peak area of the analyte of the buffer side and the plasma side was determined. The formula for calculating the plasma protein binding rate of the analyte and the control drug is as follows: Free rate = (ratio of sample peak area to internal standard peak area of buffer side/ratio of sample peak area to internal standard peak area of plasma side) * 100%, binding rate = 1-free rate, and recovery rate = (ratio of sample peak area to internal standard peak area of buffer side + ratio of sample peak area to internal standard peak area of plasma side)/(ratio of sample peak area to internal standard peak area of initial plasma sample) * 100%. The ratio of sample peak area to internal standard peak area of buffer side represents the free concentration of the compound. The ratio of sample peak area to internal standard peak area of plasma side represents the sum of the free concentration and binding concentration of the compound. The ratio of sample peak area to internal standard peak area of initial plasma sample represents the total concentration of the compound at the beginning of the sample incubation.

**Test results:**

See Table 6. When 1 μM compound 2 was incubated at 37°C for 6 hours, the average free rate was 46.63%, the binding rate was 53.37%, and the recovery rate was 88.02%.

**Table 6 Human plasma protein binding assay results of compound 2**

| Compound | Free rate (F%) | | | Binding rate (%) | Recovery rate (%) |
|---|---|---|---|---|---|
| | Repeat 1 | Repeat 2 | Average value | | |
| Compound 2 | 48.71 | 44.55 | 46.63 | 53.37 | 88.02 |

Example 30: Tissue distribution test of compound 2 by single gavage

Experimental materials:

A total of 60 Balb/c mice (purchased from Shanghai Minchang Biotechnology Co., Ltd.) were used, half male and half male, weighing 18-25 g.

Experimental steps:

The Balb/c mice were administered compound 2 by single gavage at a dose of 100 mg/kg and a volume of 10 mL/kg.

Before administration and 5 min, 0.25, 1.0, 2.0, 3.0, 5.0, 7.0 and 10 h after administration (6 mice at each time point, half male and half male); at the above set time points, 0.2 ml of blood was collected through the retroocular venous plexus, placed in an EDTA-K2 test tube, centrifuged at 11,000 rpm for 5 min, and the plasma was isolated and frozen in a -70°C refrigerator; After the whole blood collection at the time point of 0.25, 1.0, 3.0, and 7.0 h, the lung tissue was dissected immediately and collected. The residual blood and contents on the surface of the tissue were rinsed with cold saline, blotted dry, labelled, and stored at -70°C for testing. The content of compound 2 in plasma and lung tissue was determined by LC/MS-MS, and the lung blood ratio was calculated.

Test results:

After administration of compound 2 by single gavage to Balb/c mice, the ratio of lung tissue exposure to plasma exposure was 0.62, and the exposure of compound 2 in lung tissue was high.

Example 31: Safety pharmacological test of the effect of compound 2 on the cardiovascular system of cynomolgus monkeys by gavage administration

In the cynomolgus monkey repeated dosing toxicity study for 2 weeks, the effect of compound 2 on the cardiovascular system was concomitantly investigated.

Experimental materials:

32 cynomolgus monkeys, half male and half male, aged 2.5-5 years old at the time of administration.

Animal source: Yunnan Yingmao Biotechnology Co., Ltd.; Guangxi Xiongsen Primate Experimental Animal

Breeding and Development Co., Ltd.; Zhongke Lingrui (Zhanjiang) Biotechnology Co., Ltd.

The systolic blood pressure (SBP), diastolic blood pressure (DBP) and mean arterial blood pressure (MBP) of all conscious animals were measured using an intelligent non-invasive sphygmomanometer BP-98E with a Provantis/v10.2.3.1 electronic data acquisition system (PV-02).

Experimental steps: 32 cynomolgus monkeys (Groups 1 and 4, 5 animals/sex/group, Groups 2 and 3, 3 animals/sex/group, 4 groups in total) were randomized and administered compound 2 by nasal feeding (40, 160 and 600 mg/kg/day) or control formulation (98.9% vehicle formulation + 1.1% MTBE, 0 mg/kg/day) twice a day for a total of 14 days, and the animals recovered for 14 days after administration. All animals were included in this study to evaluate the effect of the administration on ECG parameters including heart rate, PR interval, QRS duration, QT interval, and QTcF, and on blood pressure during the pre-dose period, dosing period, and recovery period.

Test results: Under the conditions of this test, cynomolgus monkeys were administered compound 2 (40, 160 and 600 mg/kg/day) by nasal gavage for 14 days, twice a day, and there was no change in the cardiovascular system related to the test product; there was no arrhythmia related to the test product; and there were no changes in ECG parameters or blood pressure related to the test product during the entire test.

## Claims

1. A cyano compound represented by general formula I, or a racemate, an enantiomer, a diastereoisomer or a pharmaceutically acceptable salt thereof:

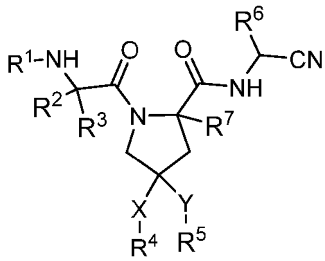

I

**characterized in that**,

$R^1$ is selected from $-COR^8$ and $-SO_2R^9$;
$R^2$ and $R^3$ are each independently selected from H, D, $C_1$-$C_{10}$ alkyl, adamantyl and $C_3$-$C_7$ cycloalkyl, or, $R^2$ and $R^3$ and the carbon atom attached thereto together form a 3- to 8-membered carbocyclic ring;
X is selected from O, S, $S(=O)_2$ and S=O;
Y is absent or selected from O, S, $S(=O)_2$ and S=O;
$R^4$ is selected from H, $C_1$-$C_{10}$ alkyl, $C_3$-$C_8$ cycloalkyl, $C_6$-$C_{20}$ aryl, $C_1$-$C_{10}$ alkyl substituted $C_6$-$C_{20}$ aryl, $C_1$-$C_{10}$ alkoxy substituted $C_6$-$C_{20}$ aryl and halogenated $C_6$-$C_{20}$ aryl;
$R^5$ is selected from H, $C_1$-$C_{10}$ alkyl and $C_3$-$C_7$ cycloalkyl;
or, $R^4$ and $R^5$ are connected to each other to form $C_2$-$C_6$ alkylene, thereby connecting X and Y;
$R^6$ is selected from

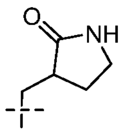

and

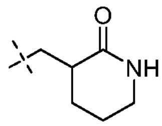

;

$R^7$ is selected from H and D;
$R^8$ is selected from H, $C_1$-$C_{10}$ alkyl, $C_1$-$C_{10}$ alkoxy, $C_3$-$C_7$ cycloalkyl, halogenated $C_1$-$C_{10}$ alkyl, halogenated $C_3$-$C_7$ cycloalkyl, $-NR^{13}R^{14}$, $C_6$-$C_{20}$ aryl, halogenated $C_6$-$C_{20}$ aryl, $C_1$-$C_{10}$ alkyl substituted $C_6$-$C_{20}$ aryl, halogenated $C_1$-$C_{10}$ alkyl substituted $C_6$-$C_{20}$ aryl, 5- to 20-membered heteroaryl and halogenated 5- to 20-membered heteroaryl;
$R^9$ is selected from $C_1$-$C_{10}$ alkyl, $C_3$-$C_7$ cycloalkyl, halogenated $C_1$-$C_{10}$ alkyl, halogenated $C_3$-$C_7$ cycloalkyl, $-NR^{15}R^{16}$, $C_6$-$C_{20}$ aryl, halogenated $C_6$-$C_{20}$ aryl, $C_1$-$C_{10}$ alkyl substituted $C_6$-$C_{20}$ aryl, halogenated $C_1$-$C_{10}$ alkyl

substituted $C_6$-$C_{20}$ aryl, 5- to 20-membered heteroaryl and halogenated 5- to 20-membered heteroaryl;
$R^{13}$ and $R^{14}$ are each independently selected from H and $C_1$-$C_{10}$ alkyl;
$R^{15}$ and $R^{16}$ are each independently selected from H and $C_1$-$C_{10}$ alkyl.

**2.** The cyano compound, or the racemate, the enantiomer, the diastereoisomer or the pharmaceutically acceptable salt thereof according to claim 1, **characterized in that**,

$R^2$ and $R^3$ are each independently selected from H, D, $C_1$-$C_6$ alkyl, adamantyl and $C_3$-$C_7$ cycloalkyl, or, $R^2$ and $R^3$ and the carbon atom attached thereto together form a 3- to 8-membered carbocyclic ring; or
$R^2$ and $R^3$ are each independently selected from H, isopropyl, tert-butyl, cyclopentyl, and adamantyl, or, $R^2$ and $R^3$ and the carbon atom attached thereto together form cyclopropyl and cyclopentyl; or
one of $R^2$ and $R^3$ is selected from H, and the other is selected from isopropyl, tert-butyl, cyclopentyl, and adamantyl, or, $R^2$ and $R^3$ and the carbon atom attached thereto together form cyclopropyl and cyclopentyl.

**3.** The cyano compound, or the racemate, the enantiomer, the diastereoisomer or the pharmaceutically acceptable salt thereof according to claim 1 or 2, **characterized in that**,

$R^4$ is selected from H, $C_1$-$C_6$ alkyl, $C_3$-$C_8$ cycloalkyl, $C_6$-$C_{10}$ aryl, $C_1$-$C_6$ alkyl substituted $C_6$-$C_{10}$ aryl, $C_1$-$C_6$ alkoxy substituted $C_6$-$C_{10}$ aryl and halogenated $C_6$-$C_{10}$ aryl;
$R^5$ is selected from H, $C_1$-$C_6$ alkyl and $C_3$-$C_7$ cycloalkyl;
or, $R^4$ and $R^5$ are connected to each other to form $C_2$-$C_6$ alkylene, thereby connecting X and Y; or, $R^4$ and $R^5$ are connected to each other to form $CH_2CH_2$ and $CH_2CH_2CH_2$, thereby connecting X and Y.

**4.** The cyano compound, or the racemate, the enantiomer, the diastereoisomer or the pharmaceutically acceptable salt thereof according to any one of claims 1-3, **characterized in that**,

$R^8$ is selected from H, $C_1$-$C_6$ alkyl, $C_1$-$C_6$ alkoxy, $C_3$-$C_7$ cycloalkyl, halogenated $C_1$-$C_6$ alkyl, halogenated $C_3$-$C_7$ cycloalkyl, $-NR^{13}R^{14}$, $C_6$-$C_{10}$ aryl, halogenated $C_6$-$C_{10}$ aryl, $C_1$-$C_6$ alkyl substituted $C_6$-$C_{10}$ aryl, halogenated $C_1$-$C_6$ alkyl substituted $C_6$-$C_{10}$ aryl, 5- to 10-membered heteroaryl and halogenated 5- to 10-membered heteroaryl, $R^{13}$ and $R^{14}$ are each independently selected from H and $C_1$-$C_6$ alkyl; or
$R^8$ is selected from $C_1$-$C_6$ alkyl, halogenated $C_1$-$C_6$ alkyl, $C_1$-$C_6$ alkoxy, $-NR^{13}R^{14}$, $C_3$-$C_7$ cycloalkyl, halogenated $C_3$-$C_7$ cycloalkyl, phenyl, halophenyl, $C_1$-$C_6$ alkyl substituted phenyl, halogenated $C_1$-$C_6$ alkyl substituted phenyl and 5- to 6-membered heteroaryl, $R^{13}$ and $R^{14}$ are each independently selected from H and $C_1$-$C_6$ alkyl; or
$R^8$ is selected from $CH_3$, $CF_3$, $CH_2CF_3$, $CF_2CF_3$, methoxy,

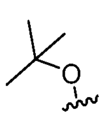,
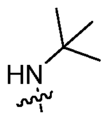,

cyclopropyl,

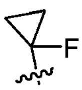,

phenyl,

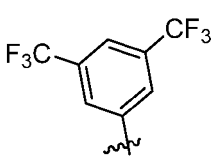,
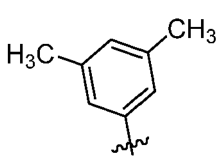,
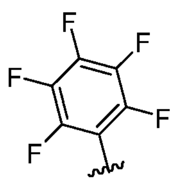

and pyridin-3-yl;
$R^9$ is selected from $C_1$-$C_6$ alkyl, $C_3$-$C_7$ cycloalkyl, halogenated $C_1$-$C_6$ alkyl, halogenated $C_3$-$C_7$ cycloalkyl, $-NR^{15}R^{16}$, $C_6$-$C_{10}$ aryl, halogenated $C_6$-$C_{10}$ aryl, $C_1$-$C_6$ alkyl substituted $C_6$-$C_{10}$ aryl, halogenated $C_1$-$C_6$ alkyl

substituted $C_6$-$C_{10}$ aryl, 5-to 10-membered heteroaryl and halogenated 5- to 10-membered heteroaryl, $R^{15}$ and $R^{16}$ are each independently selected from H and $C_1$-$C_6$ alkyl; or

$R^9$ is selected from $C_1$-$C_6$ alkyl, $C_3$-$C_7$ cycloalkyl, phenyl, $C_1$-$C_6$ alkyl substituted phenyl and halogenated $C_1$-$C_6$ alkyl substituted phenyl; or

$R^9$ is selected from $CH_3$, cyclopropyl, phenyl, p-methylphenyl, and p-trifluoromethylphenyl.

**5.** The cyano compound, or the racemate, the enantiomer, the diastereoisomer or the pharmaceutically acceptable salt thereof according to any one of claims 1-4, **characterized in that**,

X is selected from O, S, $S(=O)_2$ and S=O; Y is absent or selected from O, S and S=O; $R^4$ is selected from $C_1$-$C_6$ alkyl and $C_6$-$C_{10}$ aryl, $R^5$ is selected from H; or, $R^4$ and $R^5$ are connected to each other to form $C_2$-$C_6$ alkylene, thereby connecting X and Y; or

X and Y are each independently selected from O, S and S=O, and $R^4$ and $R^5$ are connected to each other to form $CH_2CH_2$ and $CH_2CH_2CH_2$, thereby connecting X and Y; or

X and Y are each independently selected from O and S, and $R^4$ and $R^5$ are connected to each other to form $CH_2CH_2$, thereby connecting X and Y.

**6.** The cyano compound, or the racemate, the enantiomer, the diastereoisomer or the pharmaceutically acceptable salt thereof according to any one of claims 1-5, **characterized in that**,

the cyano compound represented by general formula I is selected from the cyano compound represented by general formula IA:

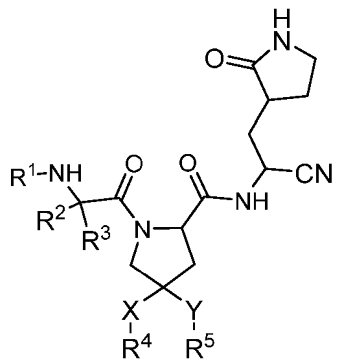

IA

wherein $R^1$, $R^2$, $R^3$, $R^4$, $R^5$, X and Y are defined as described in any one of claims 1-5.

**7.** The cyano compound, or the racemate, the enantiomer, the diastereoisomer or the pharmaceutically acceptable salt thereof according to any one of claims 1-5, **characterized in that**,

the cyano compound represented by general formula I is selected from the cyano compound represented by following general formula:

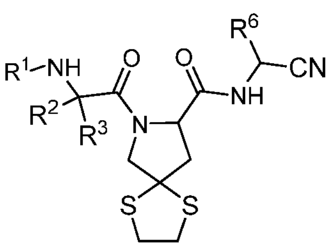

I-1

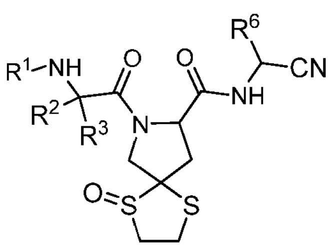

I-2

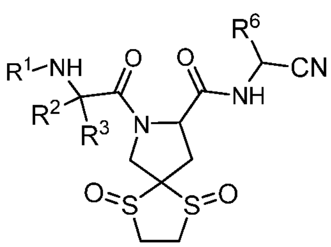

I-3

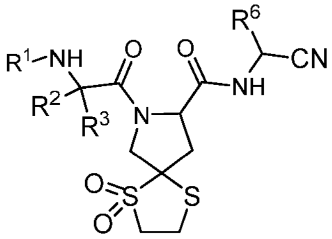

I-4

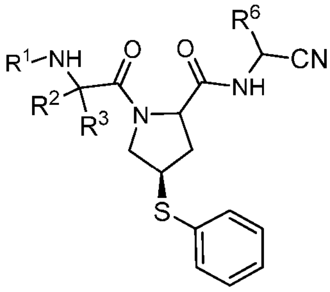

I-5

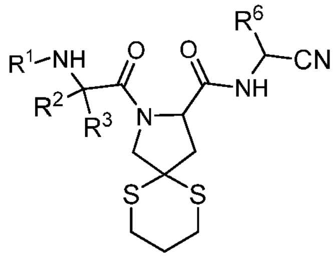

I-6

or

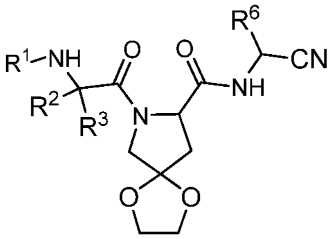

I-7

wherein $R^1$, $R^2$, $R^3$ and $R^6$ are defined as described in any one of claims 1-5.

**8.** The cyano compound, or the racemate, the enantiomer, the diastereoisomer or the pharmaceutically acceptable salt thereof according to claim 1, **characterized in that**, the compound represented by general formula I is selected from the following compounds:

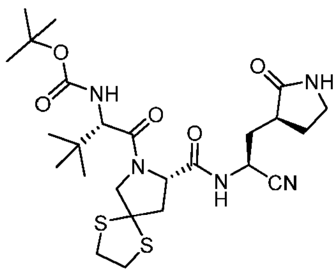

Compound 1

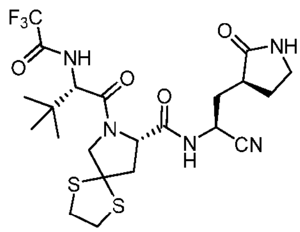

Compound 2

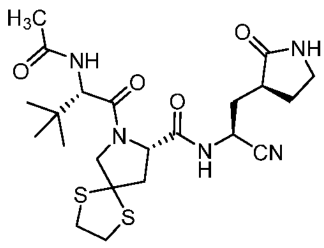

Compound 3

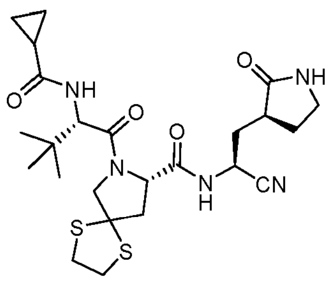

Compound 4

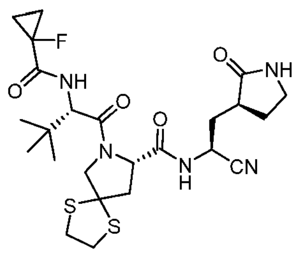

Compound 5

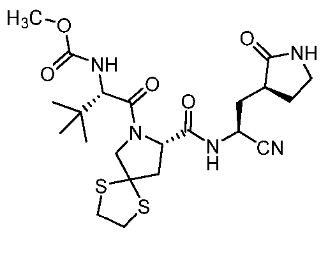

Compound 6

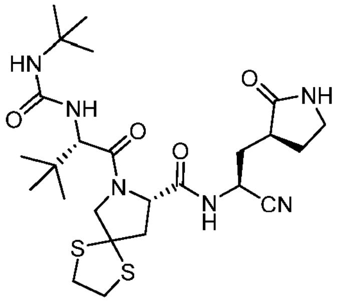

Compound 7

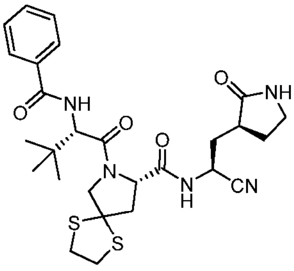

Compound 8

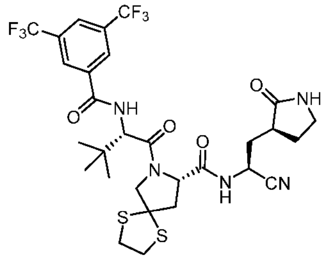

Compound 9

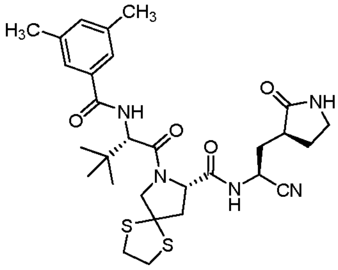

Compound 10

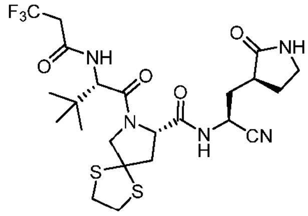

Compound 11

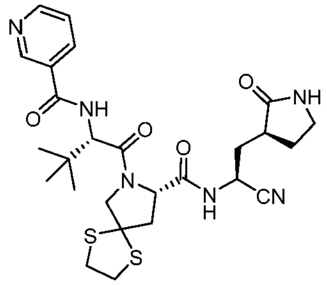

Compound 12

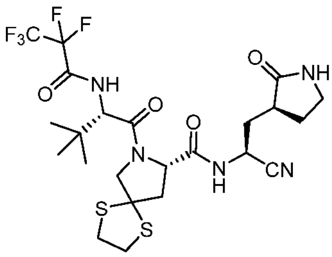

Compound 13

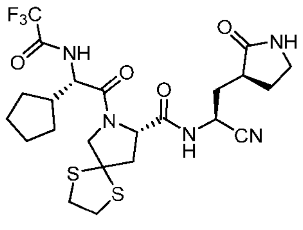

Compound 14

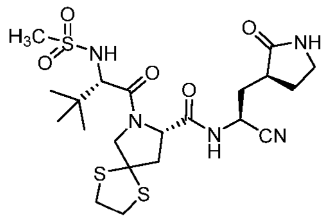

Compound 15

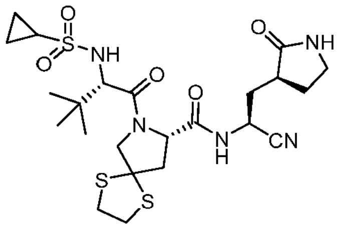

Compound 16

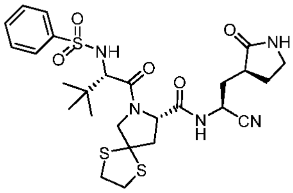

Compound 17

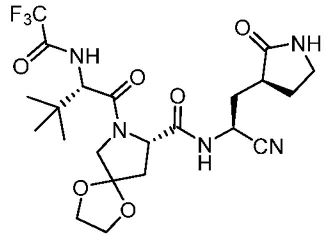

Compound 18

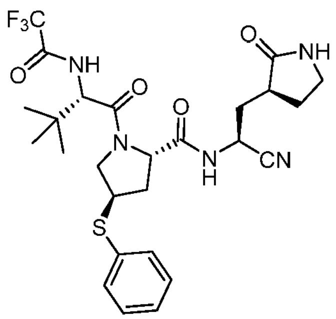

Compound 19

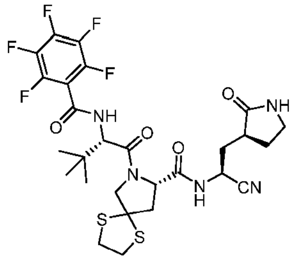

Compound 20

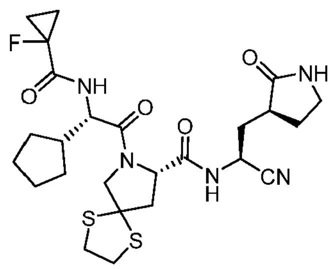

Compound 21

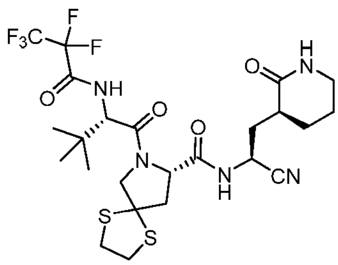

Compound 22

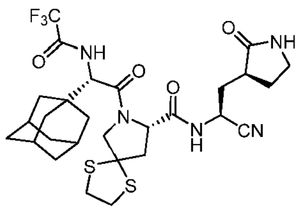

Compound 23

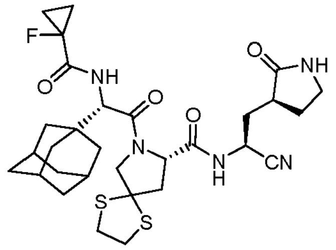

Compound 24

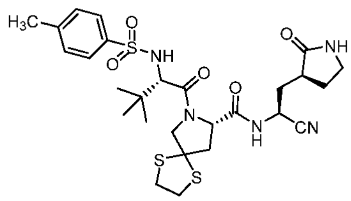

Compound 25

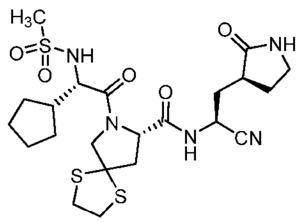

Compound 26

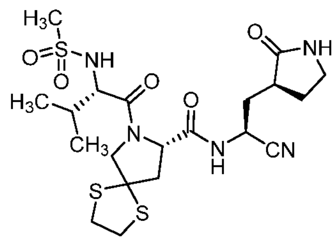

Compound 27

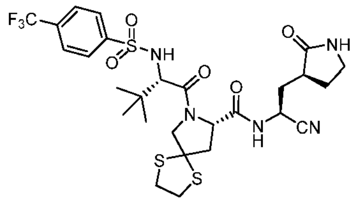

Compound 28

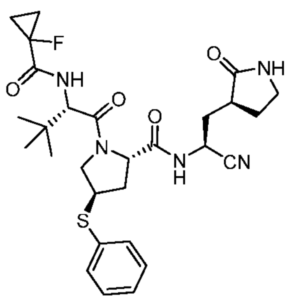

Compound 29

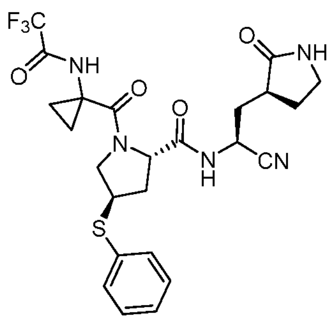

Compound 30

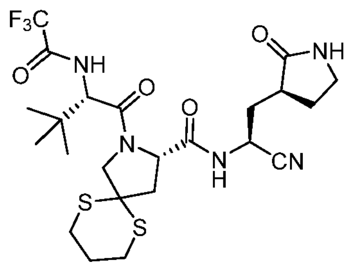

Compound 31

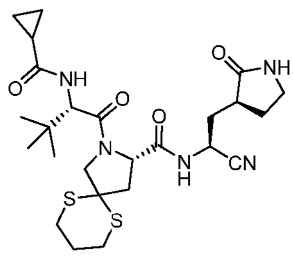

Compound 32

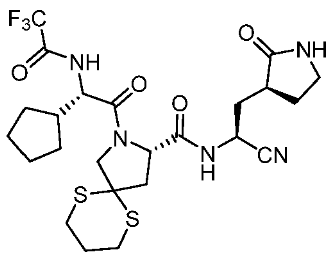

Compound 33

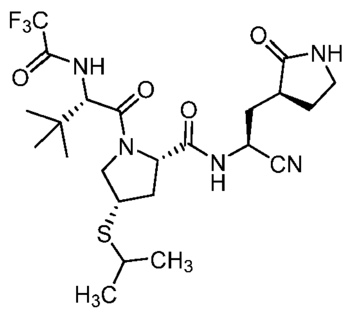

Compound 34

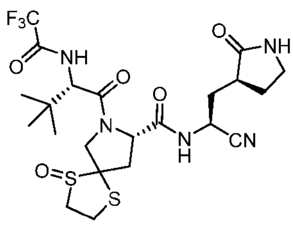

Compound 35

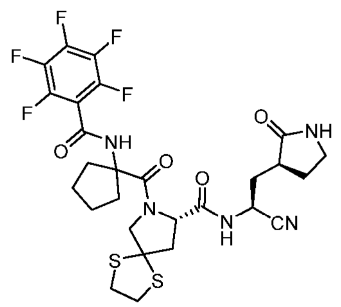

Compound 36

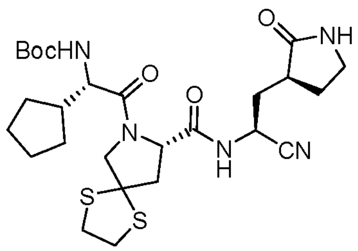

Compound 37

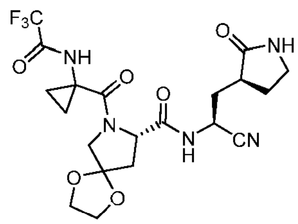

Compound 38

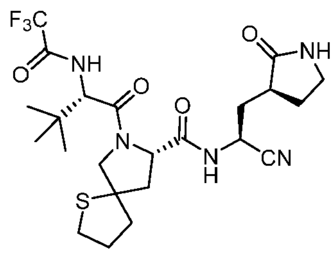

Compound 39

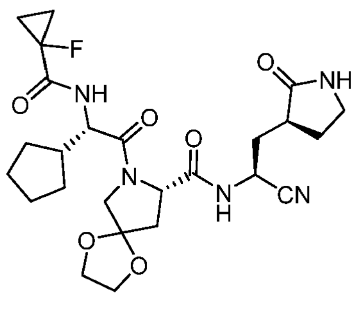

Compound 40

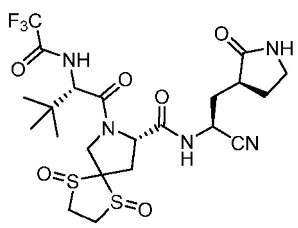

Compound 41

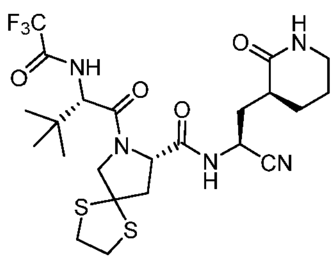

Compound 42

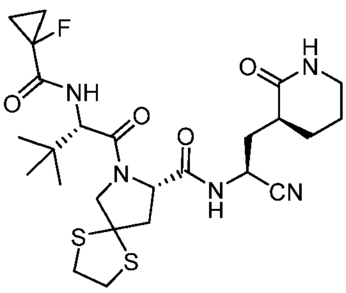

Compound 43

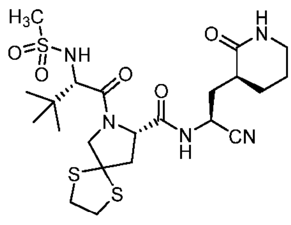

Compound 44

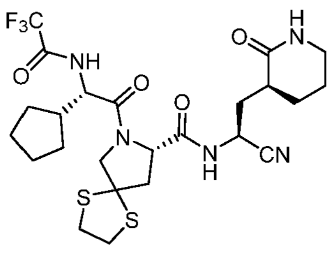

or

Compound 45

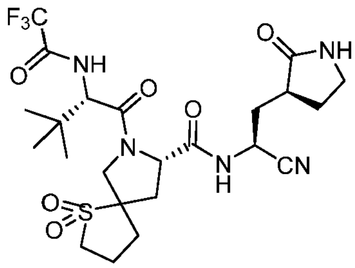

Compound 46

.

**9.** A method for preparing the compound represented by general formula I, **characterized in that** the method is one of the following methods:

Method i:

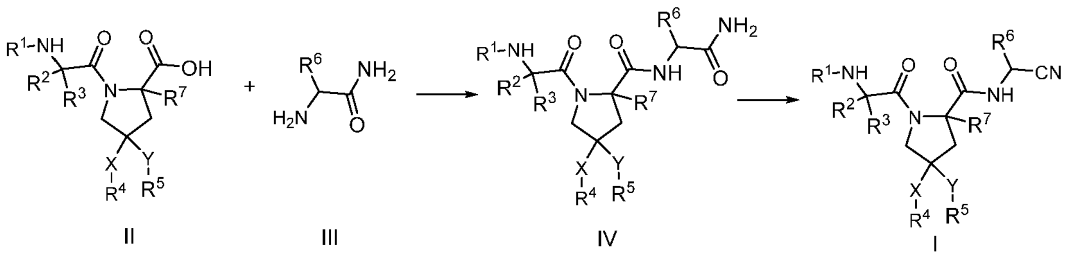

II III IV I

ia) a compound represented by formula IV is obtained via a condensation reaction of a compound represented by formula II and a compound represented by formula III;

preferably, the step ia) is that: the compound represented by formula II is reacted with the compound represented by formula III under the action of a condensing agent and a base in a solvent at a temperature of -20°C to 50°C for 0.1-12 h, thereby obtaining the compound represented by formula IV,

optionally, the solvent is one of or a mixture of more than one of the following: tetrahydrofuran, dichloromethane, N,N-dimethylformamide, N,N-dimethylacetamide, ethyl acetate and 1,4-dioxane,

optionally, the condensing agent is one of or a mixture of more than one of the following: 2-(7-azabenzotriazole)-N,N,N',N'-tetramethyluronium hexafluorophosphate, 1-ethyl-(3-dimethylaminopropyl)carbodiimide hydrochloride, 1-hydroxybenzotriazole, 2-hydroxypyridine-N-oxide, 1-propyl phosphoric anhydride, 1H-benzotriazol-1-yloxytripyrrolidinyl hexafluorophosphate, N,N'-carbonyldiimidazole and O-benzotriazole-tetramethyluronium hexafluorophosphate;

optionally, the base is N,N-diisopropylethylamine, triethylamine and N-methylmorpholine;

ib) the compound represented by formula IV is dehydrated to obtain the compound represented by general formula I;

preferably, the step ib) is that: the compound represented by formula IV is reacted with a dehydrating agent in

an anhydrous solvent at a temperature of -20°C to 50°C for 1-24 h to obtain the compound represented by general formula I;
optionally, the anhydrous solvent is one of or a mixture of more than one of the following: tetrahydrofuran, dichloromethane, toluene, 1,4-dioxane and pyridine;
optionally, the dehydrating agent is trifluoroacetic anhydride or methyl N-(triethylammoniumsulfonyl)carbamate; or

Method ii:

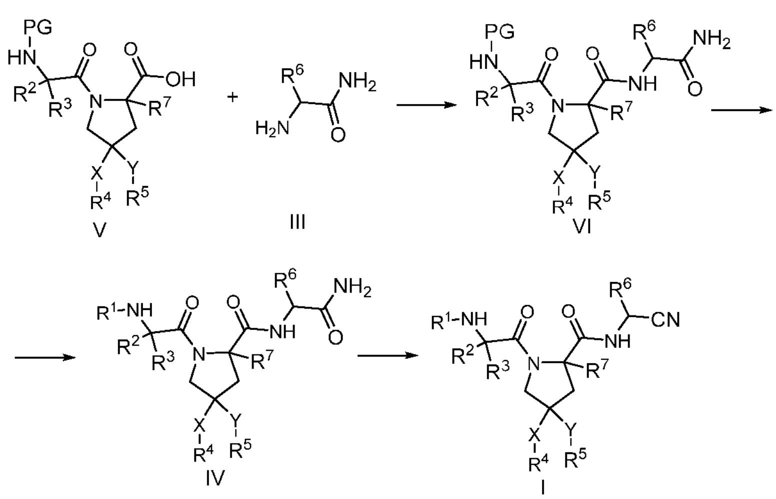

V III VI VII

IV I

iia) a compound represented by formula VI is obtained by via a condensation reaction of a compound represented by formula V and the compound represented by formula III, wherein PG in the compound represented by formula V is an amino protecting group;
preferably, the step iia) is that: the compound represented by formula V is reacted with the compound represented by formula III under the action of a condensing agent and a base in a solvent at a temperature of -20°C to 50°C for 0.1-12 h, thereby obtaining the compound represented by formula VI;
optionally, the solvent is one of or a mixture of more than one of the following: tetrahydrofuran, dichloromethane, N,N-dimethylformamide, N,N-dimethylacetamide, ethyl acetate and 1,4-dioxane,
optionally, the condensing agent is one of or a mixture of more than one of the following: 2-(7-azabenzotriazole)-N,N,N',N'-tetramethyluronium hexafluorophosphate, 1-ethyl-(3-dimethylaminopropyl)carbodiimide hydrochloride, 1-hydroxybenzotriazole, 2-hydroxypyridine-N-oxide, 1-propyl phosphoric anhydride, 1H-benzotriazol-1-yloxytripyrrolidinyl hexafluorophosphate, N,N'-carbonyldiimidazole and O-benzotriazole-tetramethyluronium hexafluorophosphate;
optionally, the base is N,N-diisopropylethylamine, triethylamine and N-methylmorpholine;
optionally, the amino protecting group PG is tert-butoxycarbonyl, benzyl and p-methoxybenzyl;
iib) the compound represented by formula VI is deprotected to obtain a compound represented by formula VII;
preferably, the step iib) is that: at a temperature of -20°C to 50°C, the compound represented by formula VI is reacted with an organic solution of trifluoroacetic acid or hydrogen chloride or $Pd/C/H_2$ to obtain the compound represented by formula VII;
iic) the compound represented by formula IV is obtained by aminoacylation, sulfonylation or a condensation reaction of the compound represented by formula VII;
preferably, the step iic) is that:

at a temperature of -20°C to 50°C, the compound represented by formula VII is subjected to an aminoacylation reaction with acid chloride or acid anhydride under the condition of adding a base to obtain the compound represented by formula IV;
at a temperature of -20°C to 50°C, the compound represented by formula VII is subjected to a sulfonylation reaction with sulfonyl chloride or sulfonic anhydride under the condition of adding a base to obtain the compound represented by formula IV;
at a temperature of -20°C to 50°C, the compound represented by formula VII is subjected to a condensation reaction with a carboxyl compound under the condition of a condensing agent and a

base to obtain the compound represented by formula IV;
optionally, the base is N,N-diisopropylethylamine, triethylamine and N-methylmorpholine;
optionally, the condensing agent is one of or a mixture of more than one of the following: 2-(7-azabenzotriazole)-N,N,N',N'-tetramethyluronium hexafluorophosphate, 1-ethyl-(3-dimethylaminopropyl)carbodiimide hydrochloride, 1-hydroxybenzotriazole, 2-hydroxypyridine-N-oxide, 1-propyl phosphoric anhydride, 1H-benzotriazol-1-yloxytripyrrolidinyl hexafluorophosphate, N,N'-carbonyldiimidazole and O-benzotriazole-tetramethyluronium hexafluorophosphate;
iid) the compound represented by formula IV is dehydrated to obtain the compound represented by general formula I;
preferably, the step iid) is that: the compound represented by formula IV is reacted with a dehydrating agent in an anhydrous solvent at a temperature of -20°C to 50°C for 1-24 h to obtain the compound represented by general formula I;
optionally, the anhydrous solvent is one of or a mixture of more than one of the following: tetrahydrofuran, dichloromethane, toluene, 1,4-dioxane and pyridine;
optionally, the dehydrating agent is trifluoroacetic anhydride or methyl N-(triethylammoniumsulfonyl) carbamate;
wherein $R^1$, $R^2$, $R^3$, $R^4$, $R^5$, $R^6$, $R^7$, X and Y are defined as described in any one of claims 1-8.

**10.** A pharmaceutical composition, **characterized in that** the pharmaceutical composition comprises the cyano compound, or the racemate, the enantiomer, the diastereoisomer or the pharmaceutically acceptable salt thereof according to any one of claims 1-8, and a pharmaceutically acceptable excipient; optionally, the pharmaceutical composition further comprises ritonavir or a pharmaceutically acceptable salt thereof.

**11.** A pharmaceutical combination, **characterized in that** the pharmaceutical composition comprises the cyano compound, or the racemate, the enantiomer, the diastereoisomer or the pharmaceutically acceptable salt thereof according to any one of claims 1-8, and ritonavir or a pharmaceutically acceptable salt thereof.

**12.** A cyano compound, or the racemate, the enantiomer, the diastereoisomer or the pharmaceutically acceptable salt thereof according to any one of claims 1-8 for use as a medicament selected from a medicament for inhibiting coronavirus 3CL protease activity, a medicament for preventing and/or treating coronavirus infection, a medicament for inhibiting picornavirus 3CL protease activity, and a medicament for preventing and/or treating picornavirus infection; optionally, the coronavirus is selected from SARS-CoV, MERS-CoV, H229E-CoV, HKU1-CoV, NL63-CoV, OC43-CoV and SARS-CoV-2.

**13.** A pharmaceutical composition according to claim 10 for use as a medicament selected from a medicament for inhibiting coronavirus 3CL protease activity, a medicament for preventing and/or treating coronavirus infection, a medicament for inhibiting picornavirus 3CL protease activity, and a medicament for preventing and/or treating picornavirus infection; optionally, the coronavirus is selected from SARS-CoV, MERS-CoV, H229E-CoV, HKU1-CoV, NL63-CoV, OC43-CoV and SARS-CoV-2.

**14.** A pharmaceutical combination according to claim 11 fo use as a medicament selected from a medicament for inhibiting coronavirus 3CL protease activity, a medicament for preventing and/or treating coronavirus infection, a medicament for inhibiting picornavirus 3CL protease activity, and a medicament for preventing and/or treating picornavirus infection; optionally, the coronavirus is selected from SARS-CoV, MERS-CoV, H229E-CoV, HKU1-CoV, NL63-CoV, OC43-CoV and SARS-CoV-2.

## Patentansprüche

**1.** Cyanoverbindung, dargestellt durch die allgemeine Formel I, oder ein Racemat, ein Enantiomer, ein Diastereoisomer oder ein pharmazeutisch verträgliches Salz davon:

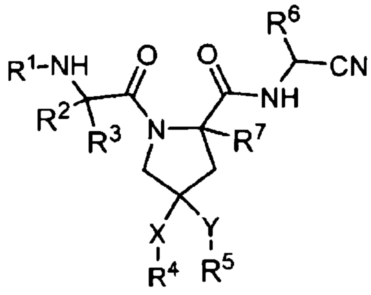

I

**dadurch gekennzeichnet, dass**

$R^1$ ausgewählt ist aus $-COR^8$ und $-SO_2R^9$;
$R^2$ und $R^3$ jeweils unabhängig ausgewählt sind aus H, D, $C_1$-$C_{10}$-Alkyl, Adamantyl und $C_3$-$C_7$-Cycloalkyl, oder $R^2$ und $R^3$ und das daran gebundene Kohlenstoffatom zusammen einen 3- bis 8-gliedrigen carbocyclischen Ring bilden;
X ausgewählt ist aus O, S, $S(=O)_2$ und S=O;
Y fehlt oder ausgewählt ist aus O, S, $S(=O)_2$ und S=O;
$R^4$ ausgewählt ist aus H, $C_1$-$C_{10}$-Alkyl, $C_3$-$C_8$-Cycloalkyl, $C_6$-$C_{20}$-Aryl, $C_1$-$C_{10}$-Alkyl-substituiertem $C_6$-$C_{20}$-Aryl, $C_1$-$C_{10}$-Alkoxy-substituiertem $C_6$-$C_{20}$-Aryl und halogeniertem $C_6$-$C_{20}$-Aryl;
$R^5$ ausgewählt ist aus H, $C_1$-$C_{10}$-Alkyl und $C_3$-$C_7$-Cycloalkyl;
oder $R^4$ und $R^5$ miteinander verbunden sind, um $C_2$-$C_6$-Alkylen zu bilden, wodurch X und Y verbunden werden;
$R^6$ ausgewählt ist aus

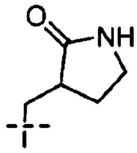 und 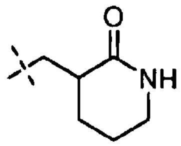 ;

$R^7$ ausgewählt ist aus H und D;
$R^8$ ausgewählt ist aus H, $C_1$-$C_{10}$-Alkyl, $C_1$-$C_{10}$-Alkoxy, $C_3$-$C_7$-Cycloalkyl, halogeniertem $C_1$-$C_{10}$-Alkyl, halogeniertem $C_3$-$C_7$-Cycloalkyl, $-NR^{13}R^{14}$, $C_6$-$C_{20}$-Aryl, halogeniertem $C_6$-$C_{20}$-Aryl, $C_1$-$C_{10}$-Alkyl-substituiertem $C_6$-$C_{20}$-Aryl, halogeniertem $C_1$-$C_{10}$-Alkyl-substituiertem $C_6$-$C_{20}$-Aryl, 5- bis 20-gliedrigem Heteroaryl und halogeniertem 5- bis 20-gliedrigem Heteroaryl;
$R^9$ ausgewählt ist aus $C_1$-$C_{10}$-Alkyl, $C_3$-$C_7$-Cycloalkyl, halogeniertem $C_1$-$C_{10}$-Alkyl, halogeniertem $C_3$-$C_7$-Cycloalkyl, $-NR^{15}R^{16}$, $C_6$-$C_{20}$-Aryl, halogeniertem $C_6$-$C_{20}$-Aryl, $C_1$-$C_{10}$-Alkyl-substituiertem $C_6$-$C_{20}$-Aryl, halogeniertem $C_1$-$C_{10}$-Alkyl-substituiertem $C_6$-$C_{20}$-Aryl, 5- bis 20-gliedrigem Heteroaryl und halogeniertem 5-bis 20-gliedrigem Heteroaryl;
$R^{13}$ und $R^{14}$ jeweils unabhängig voneinander ausgewählt sind aus H und $C_1$-$C_{10}$-Alkyl;
$R^{15}$ und $R^{16}$ jeweils unabhängig ausgewählt sind aus H und $C_1$-$C_{10}$-Alkyl.

**2.** Cyanoverbindung oder Racemat, Enantiomer, Diastereoisomer oder pharmazeutisch verträgliches Salz davon nach Anspruch 1,
**dadurch gekennzeichnet, dass**

$R^2$ und $R^3$ jeweils unabhängig ausgewählt sind aus H, D, $C_1$-$C_6$-Alkyl, Adamantyl und $C_3$-$C_7$-Cycloalkyl, oder $R^2$ und $R^3$ und das daran gebundene Kohlenstoffatom zusammen einen 3- bis 8-gliedrigen carbocyclischen Ring bilden; oder
$R^2$ und $R^3$ jeweils unabhängig voneinander ausgewählt sind aus H, Isopropyl, tert-Butyl, Cyclopentyl und Adamantyl, oder $R^2$ und $R^3$ und das daran gebundene Kohlenstoffatom zusammen Cyclopropyl und Cyclopentyl bilden; oder
eines von $R^2$ und $R^3$ aus H ausgewählt ist, und das andere aus Isopropyl, tert-Butyl, Cyclopentyl und Adamantyl ausgewählt ist, oder $R^2$ und $R^3$ und das daran gebundene Kohlenstoffatom zusammen Cyclopropyl und Cyclopentyl bilden.

**3.** Cyanoverbindung oder Racemat, Enantiomer, Diastereoisomer oder pharmazeutisch verträgliches Salz davon nach Anspruch 1 oder 2,
**dadurch gekennzeichnet, dass**

$R^4$ ausgewählt ist aus H, $C_1$-$C_6$-Alkyl, $C_3$-$C_8$-Cycloalkyl, $C_6$-$C_{10}$-Aryl, $C_1$-$C_6$-Alkyl-substituiertem $C_6$-$C_{10}$-Aryl, $C_1$-$C_6$-Alkoxy-substituiertem $C_6$-$C_{10}$-Aryl und halogeniertem $C_6$-$C_{10}$-Aryl;
$R^5$ ausgewählt ist aus H, $C_1$-$C_6$-Alkyl und $C_3$-$C_7$-Cycloalkyl;
oder $R^4$ und $R^5$ miteinander verbunden sind, um $C_2$-$C_6$-Alkylen zu bilden, wodurch X und Y verbunden werden;
oder $R^4$ und $R^5$ miteinander verbunden sind, um $CH_2CH_2$ und $CH_2CH_2CH_2$ zu bilden, wodurch X und Y verbunden werden.

**4.** Cyanoverbindung oder Racemat, Enantiomer, Diastereoisomer oder pharmazeutisch verträgliches Salz davon nach einem der Ansprüche 1 bis 3, **dadurch gekennzeichnet, dass**

$R^8$ ausgewählt ist aus H, $C_1$-$C_6$-Alkyl, $C_1$-$C_6$-Alkoxy, $C_3$-$C_7$-Cycloalkyl, halogeniertem $C_1$-$C_6$-Alkyl, halogeniertem $C_3$-$C_7$-Cycloalkyl, -$NR^{13}R^{14}$, $C_6$-$C_{10}$-Aryl, halogeniertem $C_6$-$C_{10}$-Aryl, $C_1$-$C_6$-Alkyl-substituiertem $C_6$-$C_{10}$-Aryl, halogeniertem $C_1$-$C_6$-Alkyl-substituiertem $C_6$-$C_{10}$-Aryl, 5- bis 10-gliedrigem Heteroaryl und halogeniertem 5- bis 10-gliedrigem Heteroaryl, $R^{13}$ und $R^{14}$ jeweils unabhängig ausgewählt sind aus H und $C_1$-$C_6$-Alkyl; oder
$R^a$ ausgewählt ist aus $C_1$-$C_6$-Alkyl, halogeniertem $C_1$-$C_6$-Alkyl, $C_1$-$C_6$-Alkoxy, -$NR^{13}R^{14}$, $C_3$-$C_7$-Cycloalkyl, halogeniertem $C_3$-$C_7$-Cycloalkyl, Phenyl, Halophenyl, $C_1$-$C_6$-Alkyl-substituiertem Phenyl, halogeniertem $C_1$-$C_6$-Alkyl-substituiertem Phenyl und 5- bis 6-gliedrigem Heteroaryl, $R^{13}$ und $R^{14}$ jeweils unabhängig ausgewählt sind aus H und $C_1$-$C_6$-Alkyl; oder
$R^8$ ausgewählt ist aus $CH_3$, $CF_3$, $CH_2CF_3$, $CF_2CF_3$, Methoxy,

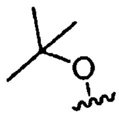
,

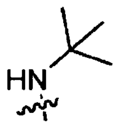
,

Cyclopropyl,

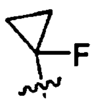
,

Phenyl,

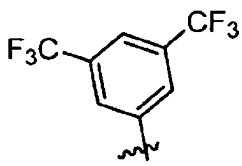
,
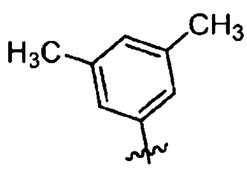
,

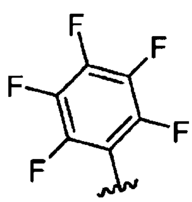

und Pyridin-3-yl;
$R^9$ ausgewählt ist aus $C_1$-$C_6$-Alkyl, $C_3$-$C_7$-Cycloalkyl, halogeniertem $C_1$-$C_6$-Alkyl, halogeniertem $C_3$-$C_7$-Cycloalkyl, -$NR^{15}R^{16}$, $C_6$-$C_{10}$-Aryl, halogeniertem $C_6$-$C_{10}$-Aryl, $C_1$-$C_6$-Alkyl-substituiertem $C_6$-$C_{10}$-Aryl, halogeniertem $C_1$-$C_6$-Alkyl-substituiertem $C_6$-$C_{10}$ Aryl, 5- bis 10-gliedrigem Heteroaryl und halogeniertem 5-bis 10-gliedrigem Heteroaryl, $R^{15}$ und $R^{16}$ jeweils unabhängig voneinander ausgewählt sind aus H und $C_1$-$C_6$-Alkyl; oder
$R^9$ ausgewählt ist aus $C_1$-$C_6$-Alkyl, $C_3$-$C_7$-Cycloalkyl, Phenyl, mit $C_1$-$C_6$-Alkyl substituiertem Phenyl und mit halogeniertem $C_1$-$C_6$-Alkyl substituiertem Phenyl; oder
$R^9$ ausgewählt ist aus $CH_3$, Cyclopropyl, Phenyl, p-Methylphenyl und p-Trifluormethylphenyl.

**5.** Cyanoverbindung oder Racemat, Enantiomer, Diastereoisomer oder pharmazeutisch verträgliches Salz davon nach einem der Ansprüche 1 bis 4, **dadurch gekennzeichnet, dass**

X ausgewählt ist aus O, S, $S(=O)_2$ und S=O; Y fehlt oder ausgewählt ist aus O, S und S=O; $R^4$ ausgewählt ist aus $C_1$-$C_6$-Alkyl und $C_6$-$C_{10}$-Aryl, $R^5$ ausgewählt ist aus H; oder $R^4$ und $R^5$ miteinander verbunden sind, um $C_2$-$C_6$-Alkylen zu bilden, wodurch X und Y verbunden werden; oder
X und Y jeweils unabhängig ausgewählt sind aus O, S und S=O, und $R^4$ und $R^5$ miteinander verbunden sind, um $CH_2CH_2$ und $CH_2CH_2CH_2$ zu bilden, wodurch X und Y verbunden werden; oder
X und Y jeweils unabhängig aus O und S ausgewählt sind, und $R^4$ und $R^5$ miteinander verbunden sind, um $CH_2CH_2$ zu bilden, wodurch X und Y verbunden werden.

**6.** Cyanoverbindung oder Racemat, Enantiomer, Diastereoisomer oder pharmazeutisch verträgliches Salz davon nach einem der Ansprüche 1 bis 5, **dadurch gekennzeichnet, dass**

die durch die allgemeine Formel I dargestellte Cyanoverbindung aus der durch die allgemeine Formel IA dargestellten Cyanoverbindung ausgewählt ist:

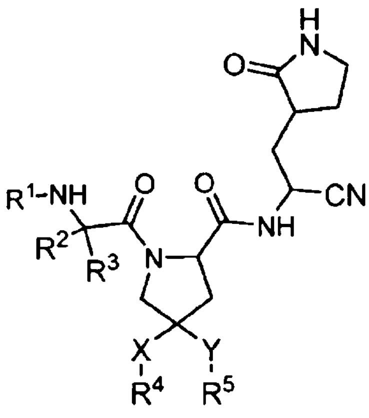

IA

wobei $R^1$, $R^2$, $R^3$, $R^4$, $R^5$, X und Y wie in einem der Ansprüche 1 bis 5 beschrieben definiert sind.

**7.** Cyanoverbindung oder Racemat, Enantiomer, Diastereoisomer oder pharmazeutisch verträgliches Salz davon nach einem der Ansprüche 1 bis 5, **dadurch gekennzeichnet, dass**

die durch die allgemeine Formel I dargestellte Cyanoverbindung aus der durch die folgende allgemeine Formel dargestellten Cyanoverbindung ausgewählt ist:

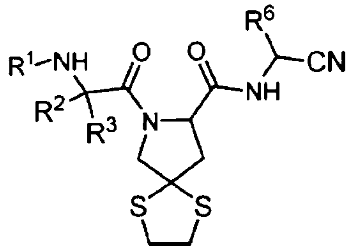

I-1

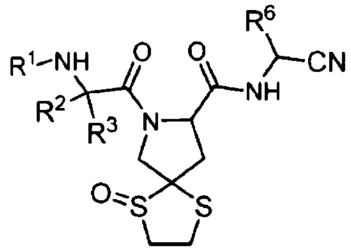

I-2

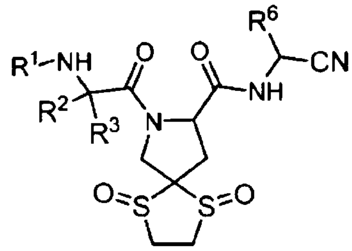

I-3

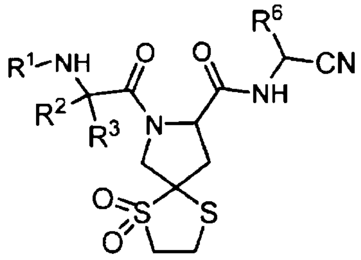

I-4

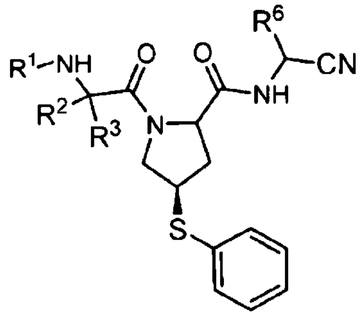

I-5

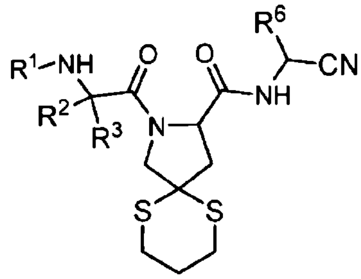

I-6

oder

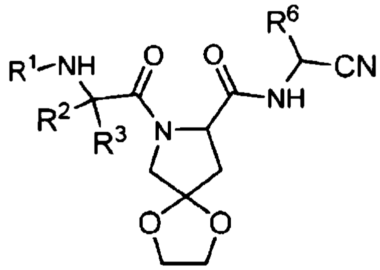

I-7

wobei $R^1$, $R^2$, $R^3$ und $R^6$ wie in einem der Ansprüche 1 bis 5 beschrieben definiert sind.

8. Cyanoverbindung oder Racemat, Enantiomer, Diastereoisomer oder pharmazeutisch verträgliches Salz davon nach Anspruch 1,
**dadurch gekennzeichnet, dass**
die durch die allgemeine Formel I dargestellte Verbindung aus den folgenden Verbindungen ausgewählt ist:

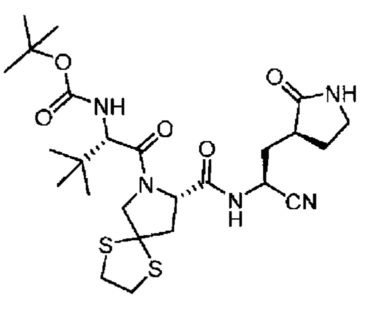

Verbindung 1

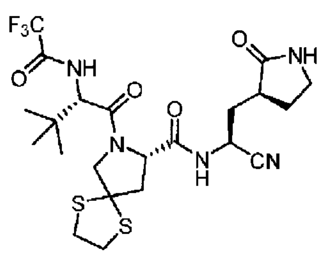

Verbindung 2

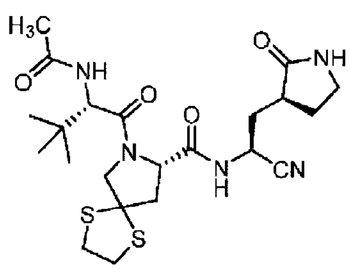

Verbindung 3

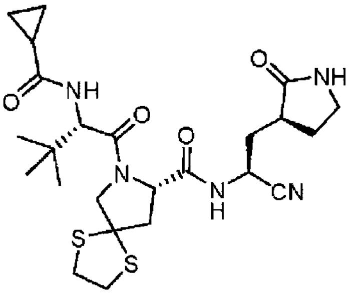

Verbindung 4

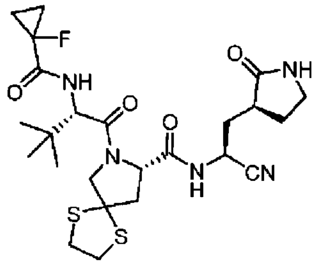

Verbindung 5

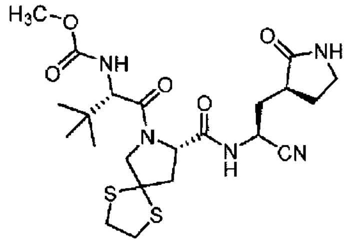

Verbindung 6

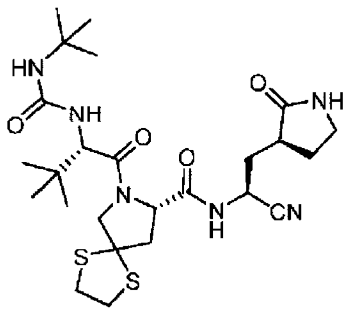

Verbindung 7

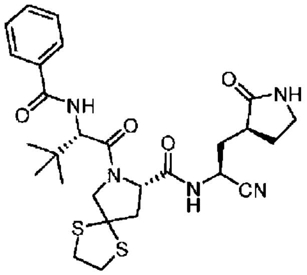

Verbindung 8

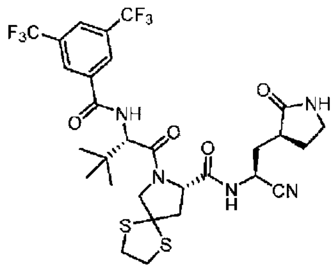

Verbindung 9

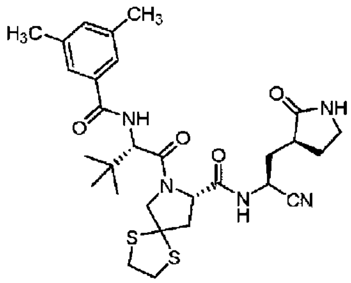

Verbindung 10

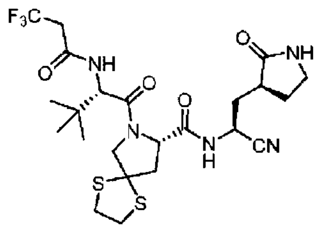

Verbindung 11

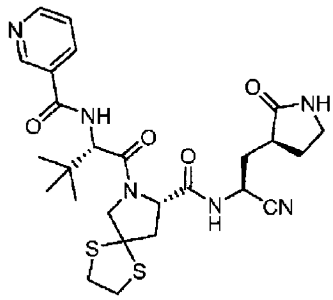

Verbindung 12

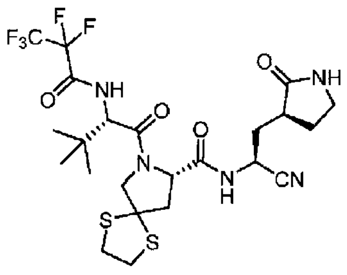

Verbindung 13

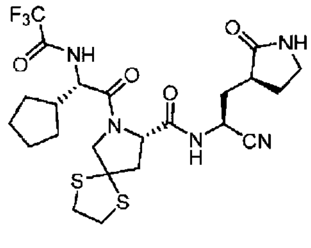

Verbindung 14

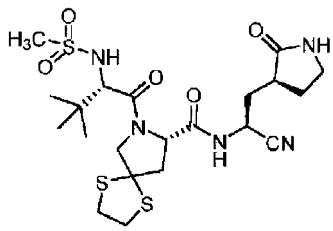

Verbindung 15

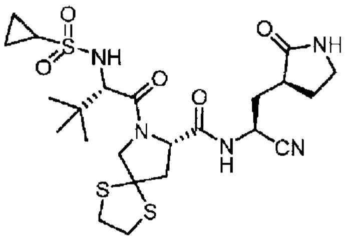

Verbindung 16

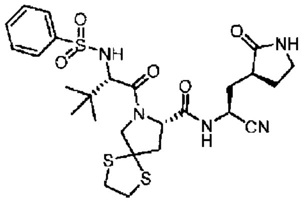

Verbindung 17

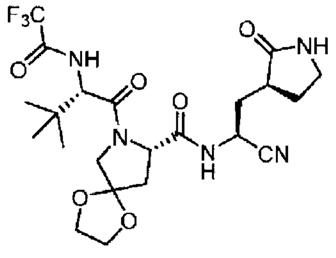

Verbindung 18

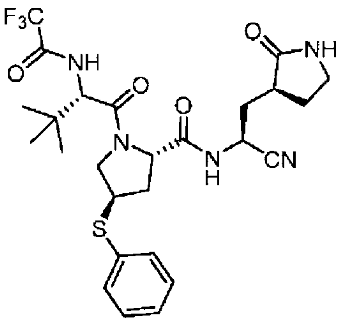

Verbindung 19

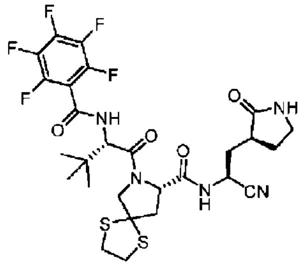

Verbindung 20

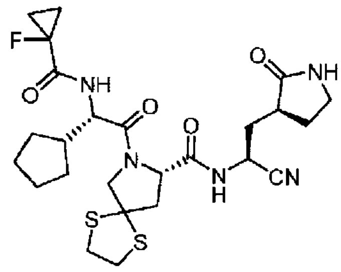

Verbindung 21

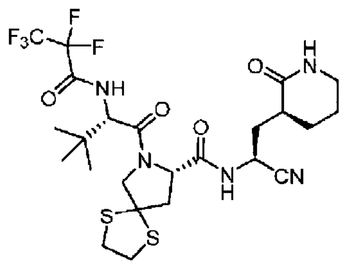

Verbindung 22

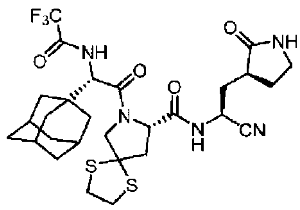

Verbindung 23

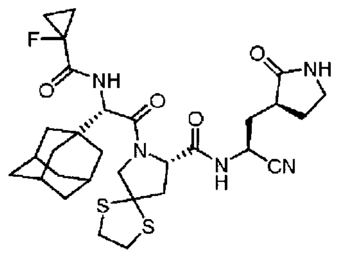

Verbindung 24

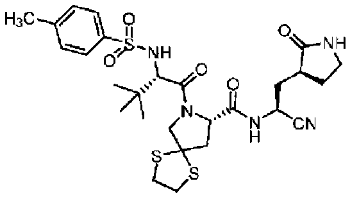

Verbindung 25

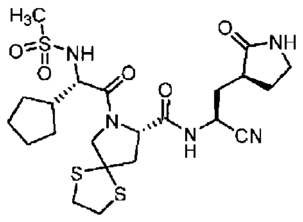

Verbindung 26

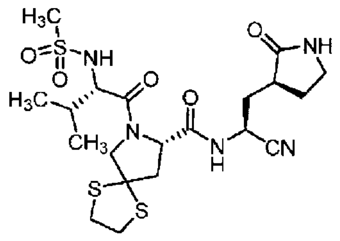

Verbindung 27

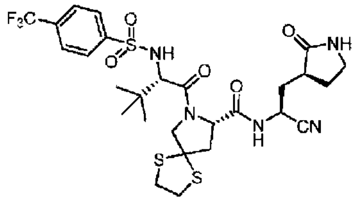

Verbindung 28

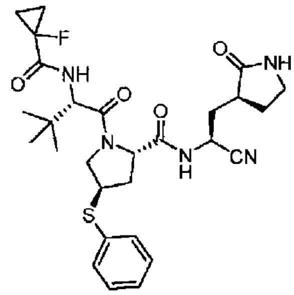

Verbindung 29

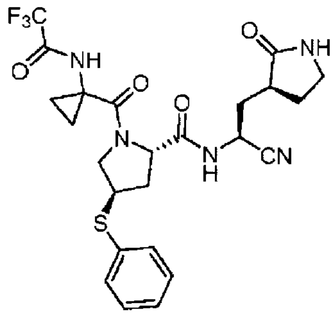

Verbindung 30

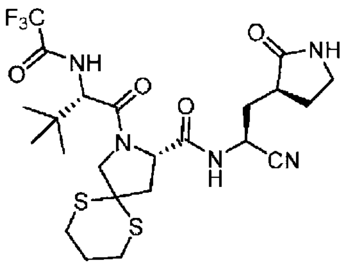

Verbindung 31

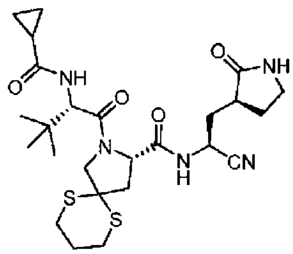

Verbindung 32

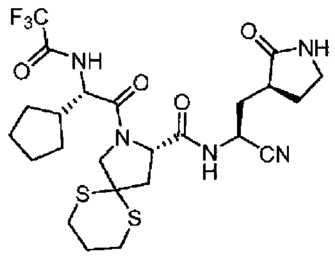

Verbindung 33

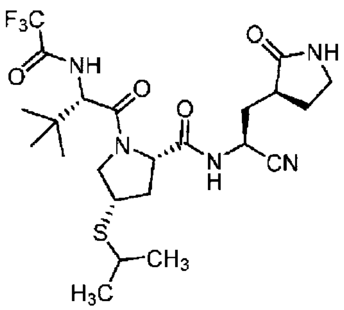

Verbindung 34

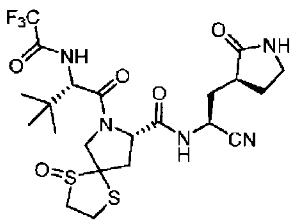

Verbindung 35

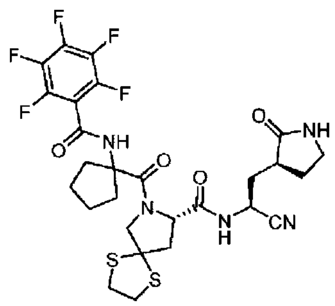

Verbindung 36

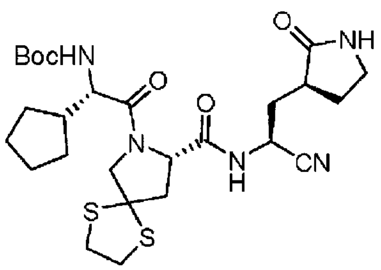

Verbindung 37

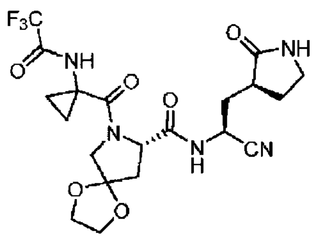

Verbindung 38

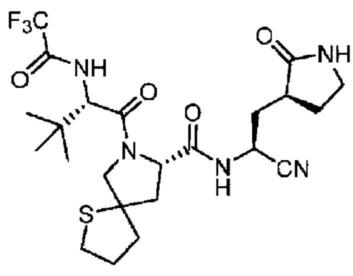

Verbindung 39

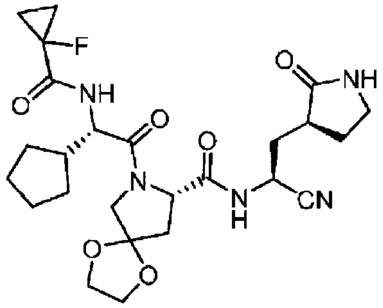

Verbindung 40

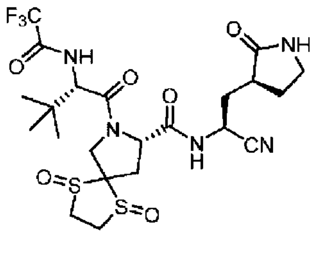

Verbindung 41

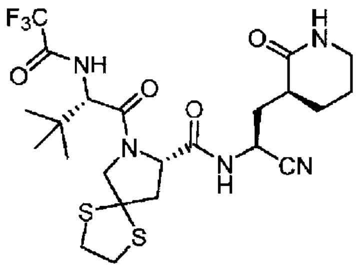

Verbindung 42

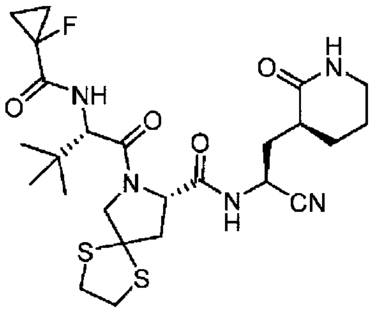

Verbindung 43

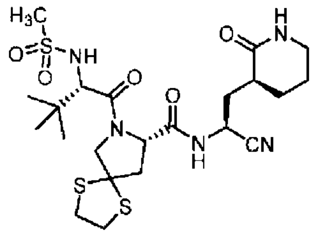

Verbindung 44

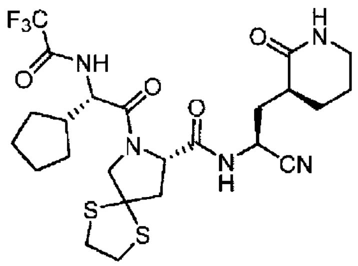

oder

Verbindung 45

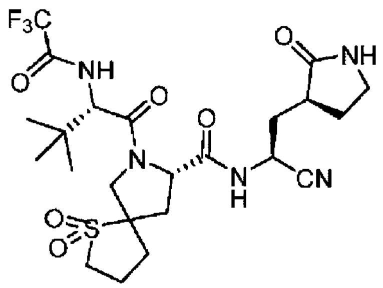

Verbindung 46

**9.** Verfahren zur Herstellung der durch die allgemeine Formel I dargestellten Verbindung, **dadurch gekennzeichnet, dass** das Verfahren eines der folgenden Verfahren ist:

Verfahren i:

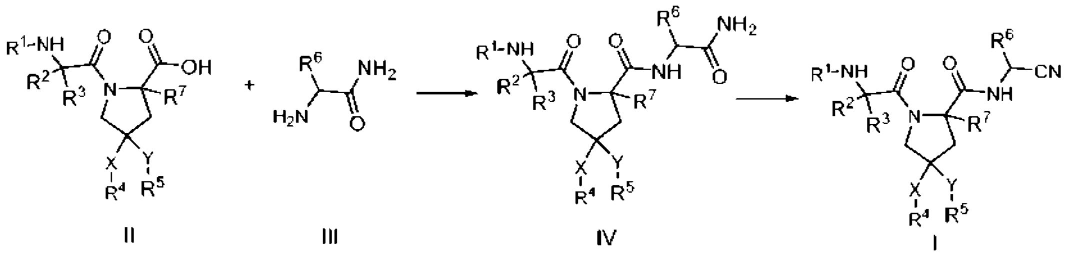

II III IV I

ia) eine durch Formel IV dargestellte Verbindung wird über eine Kondensationsreaktion einer durch Formel II

dargestellten Verbindung und einer durch Formel III dargestellten Verbindung erhalten;

vorzugsweise ist der Schritt ia), dass: die durch Formel II dargestellte Verbindung mit der durch Formel III dargestellten Verbindung unter Einwirken eines Kondensationsmittels und einer Base in einem Lösungsmittel bei einer Temperatur von -20 °C bis 50 °C für 0,1 bis 12 h umgesetzt wird, wodurch die durch Formel IV dargestellte Verbindung erhalten wird,
wahlweise ist das Lösungsmittel eines von oder eine Mischung aus mehr als einem von: Tetrahydrofuran, Dichlormethan, N,N-Dimethylformamid, N,N-Dimethylacetamid, Ethylacetat und 1,4-Dioxan,
wahlweise ist das Kondensierungsmittel eines von oder eine Mischung aus mehr als einem von: 2-(7-Azabenzotriazol)-N,N,N',N'-tetramethyluronium-hexafluorophosphat, 1-Ethyl-(3-dimethylaminopropyl)carbodiimid-hydrochlorid, 1-Hydroxybenzotriazol, 2-Hydroxypyridin-N-oxid, 1-Propylphosphorsäureanhydrid, 1H-Benzotriazol-1-yloxytripyrrolidinyl-hexafluorophosphat, N,N'-Carbonyldiimidazol und O-Benzotriazol-tetramethyluronium-hexafluorophosphat;
wahlweise ist die Base N,N-Diisopropylethylamin, Triethylamin und N-Methylmorpholin;

ib) die durch Formel IV dargestellte Verbindung wird dehydratisiert, um die durch allgemeine Formel I dargestellte Verbindung zu erhalten;

vorzugsweise ist der Schritt ib), dass: die durch Formel IV dargestellte Verbindung mit einem Dehydratisierungsmittel in einem wasserfreien Lösungsmittel bei einer Temperatur von -20 °C bis 50 °C für 1 bis 24 h umgesetzt wird, um die durch allgemeine Formel I dargestellte Verbindung zu erhalten;
wahlweise ist das wasserfreie Lösungsmittel eines von oder eine Mischung aus mehr als einem von: Tetrahydrofuran, Dichlormethan, Toluol, 1,4-Dioxan und Pyridin;
wahlweise ist das Dehydratisierungsmittel Trifluoressigsäureanhydrid oder Methyl-N-(triethylammoniumsulfonyl)carbamat; oder

Verfahren ii:

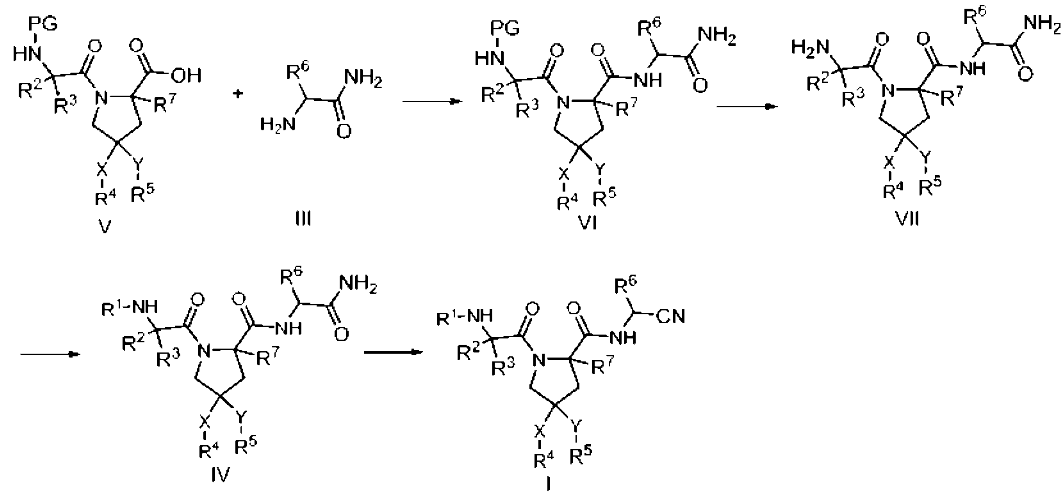

iia) eine durch Formel VI dargestellte Verbindung wird durch eine Kondensationsreaktion einer durch Formel V dargestellten Verbindung und der durch Formel III dargestellten Verbindung erhalten, wobei PG in der durch Formel V dargestellten Verbindung eine Aminoschutzgruppe ist;

vorzugsweise ist der Schritt iia), dass: die durch Formel II dargestellte Verbindung mit der durch Formel III dargestellten Verbindung unter Einwirken eines Kondensationsmittels und einer Base in einem Lösungsmittel bei einer Temperatur von -20 °C bis 50 °C für 0,1 bis 12 h umgesetzt wird, wodurch die durch Formel VI dargestellte Verbindung erhalten wird;
wahlweise ist das Lösungsmittel eines von oder eine Mischung aus mehr als einem von: Tetrahydrofuran, Dichlormethan, N,N-Dimethylformamid, N,N-Dimethylacetamid, Ethylacetat und 1,4-Dioxan,
wahlweise ist das Kondensierungsmittel eines von oder eine Mischung aus mehr als einem von: 2-(7-Azabenzotriazol)-N,N,N',N'-tetramethyluronium-hexafluorophosphat, 1-Ethyl-(3-dimethylaminopropyl)carbodiimid-hydrochlorid, 1-Hydroxybenzotriazol, 2-Hydroxypyridin-N-oxid, 1-Propylphosphorsäureanhydrid, 1H-Benzotriazol-1-yloxytripyrrolidinyl-hexafluorophosphat, N,N'-Carbonyldiimidazol und O-Benzotriazol-tetramethyluronium-hexafluorophosphat;
wahlweise ist die Base N,N-Diisopropylethylamin, Triethylamin und N-Methylmorpholin;
wahlweise ist die Aminoschutzgruppe PG tert-Butoxycarbonyl, Benzyl und p-Methoxybenzyl;

iib) die durch Formel VI dargestellte Verbindung wird entschützt, um eine durch Formel VII dargestellte Verbindung zu erhalten;
vorzugsweise ist der Schritt iib), dass: bei einer Temperatur von -20 °C bis 50 °C die durch Formel VI dargestellte Verbindung mit einer organischen Lösung von Trifluoressigsäure oder Chlorwasserstoff oder $Pd/C/H_2$ umgesetzt wird, um die durch Formel VII dargestellte Verbindung zu erhalten;
iic) die durch Formel IV dargestellte Verbindung wird durch Aminoacylierung, Sulfonylierung oder eine Kondensationsreaktion der durch Formel VII dargestellten Verbindung erhalten;
vorzugsweise ist der Schritt iic), dass:

bei einer Temperatur von -20 °C bis 50 °C die durch Formel VII dargestellte Verbindung einer Aminoacylierungsreaktion mit Säurechlorid oder Säureanhydrid unter der Bedingung der Zugabe einer Base unterzogen wird, um die durch Formel IV dargestellte Verbindung zu erhalten;
bei einer Temperatur von -20 °C bis 50 °C wird die durch Formel VII dargestellte Verbindung einer Sulfonylierungsreaktion mit Sulfonylchlorid oder Sulfonsäureanhydrid unter der Bedingung der Zugabe einer Base unterzogen, um die durch Formel IV dargestellte Verbindung zu erhalten;
bei einer Temperatur von -20 °C bis 50 °C wird die durch Formel VII dargestellte Verbindung unter der Bedingung eines Kondensationsmittels und einer Base einer Kondensationsreaktion mit einer Carboxylverbindung unterzogen, um die durch Formel IV dargestellte Verbindung zu erhalten;
wahlweise ist die Base N,N-Diisopropylethylamin, Triethylamin und N-Methylmorpholin;
wahlweise ist das Kondensierungsmittel eines von oder eine Mischung aus mehr als einem von: 2-(7-Azabenzotriazol)-N,N,N',N'-tetramethyluronium-hexafluorophosphat, 1-Ethyl-(3-dimethylaminopropyl)carbodiimid-hydrochlorid, 1-Hydroxybenzotriazol, 2-Hydroxypyridin-N-oxid, 1-Propylphosphorsäureanhydrid, 1H-Benzotriazol-1-yloxytripyrrolidinyl-hexafluorophosphat, N,N'-Carbonyldiimidazol und O-Benzotriazol-tetramethyluronium-hexafluorophosphat;

iid) die durch Formel IV dargestellte Verbindung wird dehydratisiert, um die durch allgemeine Formel I dargestellte Verbindung zu erhalten;

vorzugsweise ist der Schritt iid), dass: die durch Formel IV dargestellte Verbindung mit einem Dehydratisierungsmittel in einem wasserfreien Lösungsmittel bei einer Temperatur von -20 °C bis 50 °C für 1 bis 24 h umgesetzt wird, um die durch allgemeine Formel I dargestellte Verbindung zu erhalten;
wahlweise ist das wasserfreie Lösungsmittel eines von oder eine Mischung aus mehr als einem von: Tetrahydrofuran, Dichlormethan, Toluol, 1,4-Dioxan und Pyridin;
wahlweise ist das Dehydratisierungsmittel Trifluoressigsäureanhydrid oder Methyl-N-(triethylammoniumsulfonyl)carbamat;
wobei $R^1$, $R^2$, $R^3$, $R^4$, $R^5$, $R^6$, $R^7$, X und Y wie in einem der Ansprüche 1 bis 8 beschrieben definiert sind.

**10.** Pharmazeutische Zusammensetzung, **dadurch gekennzeichnet, dass** die pharmazeutische Zusammensetzung die Cyanoverbindung oder das Racemat, das Enantiomer, das Diastereoisomer oder das pharmazeutisch verträgliche Salz davon nach einem der Ansprüche 1 bis 8 und einen pharmazeutisch verträglichen Hilfsstoff umfasst; wahlweise umfasst die pharmazeutische Zusammensetzung ferner Ritonavir oder ein pharmazeutisch verträgliches Salz davon.

**11.** Pharmazeutische Kombination, **dadurch gekennzeichnet, dass** die pharmazeutische Zusammensetzung die Cyanoverbindung oder das Racemat, das Enantiomer, das Diastereoisomer oder das pharmazeutisch verträgliche Salz davon nach einem der Ansprüche 1 bis 8 und Ritonavir oder ein pharmazeutisch verträgliches Salz davon umfasst.

**12.** Cyanoverbindung oder Racemat, Enantiomer, Diastereoisomer oder pharmazeutisch verträgliches Salz davon nach einem der Ansprüche 1 bis 8 zur Verwendung als Medikament, ausgewählt aus einem Medikament zur Hemmung der 3CL-Protease-Aktivität des Coronavirus, einem Medikament zur Verhinderung und/oder Behandlung einer Coronavirus-Infektion, einem Medikament zur Hemmung der 3CL-Protease-Aktivität des Picornavirus und einem Medikament zur Verhinderung und/oder Behandlung einer Picornavirus-Infektion; wahlweise ist das Coronavirus ausgewählt aus SARS-CoV, MERS-CoV, H229E-CoV, HKU1-CoV, NL63-CoV, OC43-CoV und SARS-CoV-2.

**13.** Pharmazeutische Zusammensetzung nach Anspruch 10 zur Verwendung als Medikament, ausgewählt aus einem Medikament zur Hemmung der 3CL-Protease-Aktivität des Coronavirus, einem Medikament zur Verhinderung und/oder Behandlung einer Coronavirus-Infektion, einem Medikament zur Hemmung der 3CL-Protease-Aktivität des Picornavirus und einem Medikament zur Verhinderung und/oder Behandlung einer Picornavirus-Infektion; wahlweise ist das Coronavirus ausgewählt aus SARS-CoV, MERS-CoV, H229E-CoV, HKU1-CoV, NL63-CoV, OC43-CoV und SARS-CoV-2.

**14.** Pharmazeutische Kombination nach Anspruch 11 zur Verwendung als Medikament, ausgewählt aus einem Medikament zur Hemmung der 3CL-Protease-Aktivität des Coronavirus, einem Medikament zur Verhinderung und/oder Behandlung einer Coronavirus-Infektion, einem Medikament zur Hemmung der 3CL-Protease-Aktivität des Picornavirus und einem Medikament zur Verhinderung und/oder Behandlung einer Picornavirus-Infektion; wahlweise ist das Coronavirus ausgewählt aus SARS-CoV, MERS-CoV, H229E-CoV, HKU1-CoV, NL63-CoV, OC43-CoV und SARS-CoV-2.

## Revendications

**1.** Composé cyano représenté par la formule générale I, ou racémate, énantiomère, diastéréoisomère ou sel pharmaceutiquement acceptable de celui-ci :

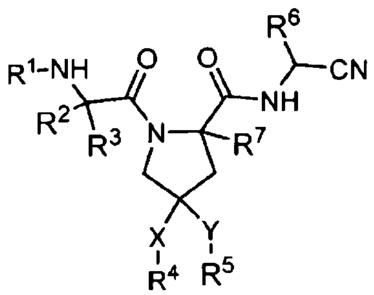

I

**caractérisé en ce que,**

$R^1$ est choisi parmi $-COR^8$ et $-SO_2R^9$ ;
$R^2$ et $R^3$ sont chacun indépendamment choisis parmi H, D, alkyle en $C_1$ à $C_{10}$, adamantyle et cycloalkyle en $C_3$ à $C_7$, ou, $R^2$ et $R^3$ et l'atome de carbone fixé à ceux-ci forment ensemble un noyau carbocyclique de 3 à 8 chaînons ;
X est choisi parmi O, S, $S(=O)_2$ et S=O ;
Y est absent ou choisi parmi O, S, $S(=O)_2$ et S=O ;
$R^4$ est choisi parmi H, alkyle en $C_1$ à $C_{10}$, cycloalkyle en $C_3$ à $C_8$, aryle en $C_6$ à $C_{20}$, aryle en $C_6$ à $C_{20}$ à substitution alkyle en $C_1$ à $C_{10}$, aryle en $C_6$ à $C_{20}$ à substitution alcoxy $C_1$ à $C_{10}$ et aryle en $C_6$ à $C_{20}$ halogéné ;
$R^5$ est choisi parmi H, alkyle en $C_1$ à $C_{10}$ et cycloalkyle en $C_3$ à $C_7$ ;
ou, $R^4$ et $R^5$ sont reliés l'un à l'autre pour former alkylène en $C_2$ à $C_6$, en reliant de ce fait X et Y ;
$R^6$ est choisi parmi

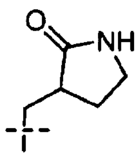 et 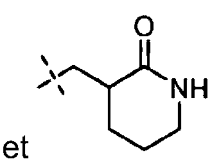 ;

$R^7$ est choisi parmi H et D ;
$R^8$ est choisi parmi H, alkyle en $C_1$ à $C_{10}$, alcoxy en $C_1$ à $C_{10}$, cycloalkyle en $C_3$ à $C_7$, alkyle en $C_1$ à $C_{10}$ halogéné, cycloalkyle en $C_3$ à $C_7$ halogéné, $-NR^{13}R^{14}$, aryle en $C_6$ à $C_{20}$, aryle en $C_6$ à $C_{20}$ halogéné, aryle en $C_6$ à $C_{20}$ à substitution alkyle en $C_1$ à $C_{10}$, aryle en $C_6$ à $C_{20}$ à substitution alkyle en $C_1$ à $C_{10}$ halogéné, hétéroaryle de 5 à 20

chaînons et hétéroaryle de 5 à 20 chaînons halogéné ;
$R^9$ est choisi parmi alkyle en $C_1$ à $C_{10}$, cycloalkyle en $C_3$ à $C_7$, alkyle en $C_1$ à $C_{10}$ halogéné, cycloalkyle en $C_3$ à $C_7$ halogéné, $-NR^{15}R^{16}$, aryle en $C_6$ à $C_{20}$, aryle en $C_6$ à $C_{20}$ halogéné, aryle en $C_6$ à $C_{20}$ à substitution alkyle en $C_1$ à $C_{10}$, aryle en $C_6$ à $C_{20}$ à substitution alkyle en $C_1$ à $C_{10}$ halogéné, hétéroaryle de 5 à 20 chaînons et hétéroaryle de 5 à 20 chaînons halogéné ;
$R^{13}$ et $R^{14}$ sont chacun indépendamment choisis parmi H et alkyle en $C_1$ à $C_{10}$ ;
$R^{15}$ et $R^{16}$ sont chacun indépendamment choisis parmi H et alkyle en $C_1$ à $C_{10}$.

**2.** Composé cyano, ou racémate, énantiomère, diastéréoisomère ou sel pharmaceutiquement acceptable de celui-ci selon la revendication 1, **caractérisé en ce que,**

$R^2$ et $R^3$ sont chacun indépendamment choisis parmi H, D, alkyle en $C_1$ à $C_6$, adamantyle et cycloalkyle en $C_3$ à $C_7$, ou, $R^2$ et $R^3$ et l'atome de carbone fixé à ceux-ci forment ensemble un noyau carbocyclique de 3 à 8 chaînons ; ou
$R^2$ et $R^3$ sont chacun indépendamment choisis parmi H, isopropyle, tert-butyle, cyclopentyle et adamantyle, ou, $R^2$ et $R^3$ et l'atome de carbone fixé à ceux-ci forment ensemble cyclopropyle et cyclopentyle ; ou
l'un de $R^2$ et de $R^3$ est choisi parmi H, et l'autre est choisi parmi isopropyle, tert-butyle, cyclopentyle et adamantyle, ou, $R^2$ et $R^3$ et l'atome de carbone fixé à ceux-ci forment ensemble cyclopropyle et cyclopentyle.

**3.** Composé cyano, ou racémate, énantiomère, diastéréoisomère ou sel pharmaceutiquement acceptable de celui-ci selon la revendication 1 ou 2, **caractérisé en ce que,**

$R^4$ est choisi parmi H, alkyle en $C_1$ à $C_6$, cycloalkyle en $C_3$ à $C_8$, aryle en $C_6$ à $C_{10}$, aryle en $C_6$ à $C_{10}$ à substitution alkyle en $C_1$ à $C_6$, aryle en $C_6$ à $C_{10}$ à substitution alcoxy en $C_1$ à $C_6$ et aryle en $C_6$ à $C_{10}$ halogéné ;
$R^5$ est choisi parmi H, alkyle en $C_1$ à $C_6$ et cycloalkyle en $C_3$ à $C_7$ ;
ou, $R^4$ et $R^5$ sont reliés l'un à l'autre pour former alkylène en $C_2$ à $C_6$, en reliant de ce fait X et Y ; ou, $R^4$ et $R^5$ sont reliés l'un à l'autre pour former $CH_2CH_2$ et $CH_2CH_2CH_2$, en reliant de ce fait X et Y.

**4.** Composé cyano, ou racémate, énantiomère, diastéréoisomère ou sel pharmaceutiquement acceptable de celui-ci selon l'une quelconque des revendications 1 à 3, **caractérisé en ce que,**

$R^8$ est choisi parmi H, alkyle en $C_1$ à $C_6$, alcoxy en $C_1$ à $C_6$, cycloalkyle en $C_3$ à $C_7$, alkyle en $C_1$ à $C_6$ halogéné, cycloalkyle en $C_3$ à $C_7$ halogéné, $-NR^{13}R^{14}$, aryle en $C_6$ à $C_{10}$, aryle en $C_6$ à $C_{10}$ halogéné, aryle en $C_6$ à $C_{10}$ à substitution alkyle en $C_1$ à $C_6$, aryle en $C_6$ à $C_{10}$ à substitution alkyle en $C_1$ à $C_6$ halogéné, hétéroaryle de 5 à 10 chaînons et hétéroaryle de 5 à 10 chaînons halogéné, $R^{13}$ et $R^{14}$ sont chacun indépendamment choisis parmi H et alkyle en $C_1$ à $C_6$ ; ou
$R^8$ est choisi parmi alkyle en $C_1$ à $C_6$, alkyle en $C_1$ à $C_6$ halogéné, alcoxy en $C_1$ à $C_6$, $-NR^{13}R^{14}$, cycloalkyle en $C_3$ à $C_7$, cycloalkyle en $C_3$ à $C_7$ halogéné, phényle, halophényle, phényle à substitution alkyle en $C_1$ à $C_6$, phényle à substitution alkyle en $C_1$ à $C_6$ halogéné et hétéroaryle de 5 à 6 chaînons, $R^{13}$ et $R^{14}$ sont chacun indépendamment choisis parmi H et alkyle en $C_1$ à $C_6$ ; ou
$R^8$ est choisi parmi $CH_3$, $CF_3$, $CH_2CF_3$, $CF_2CF_3$, méthoxy,

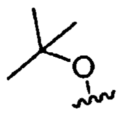,

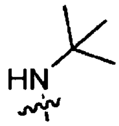,

cyclopropyle,

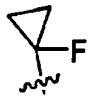

,

phényle,

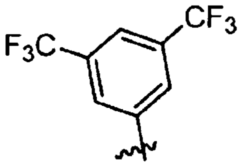

,

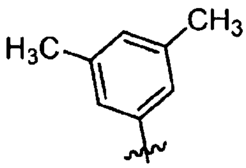

,

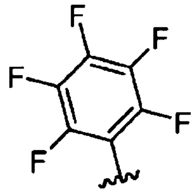

et pyridin-3-yle ;

$R^9$ est choisi parmi alkyle en $C_1$ à $C_6$, cycloalkyle en $C_3$ à $C_7$, alkyle en $C_1$ à $C_6$ halogéné, cycloalkyle en $C_3$ à $C_7$ halogéné, $-NR^{15}R^{16}$, aryle en $C_6$ à $C_{10}$, aryle en $C_6$ à $C_{10}$ halogéné, aryle en $C_6$ à $C_{10}$ à substitution alkyle en $C_1$ à $C_6$, aryle en $C_6$ à $C_{10}$ à substitution alkyle en $C_1$ à $C_6$, hétéroaryle de 5 à 10 chaînons et hétéroaryle de 5 à 10 chaînons halogéné, $R^{15}$ et $R^{16}$ sont chacun indépendamment choisis parmi H et alkyle en $C_1$ à $C_6$; ou

$R^9$ est choisi parmi alkyle en $C_1$ à $C_6$, cycloalkyle en $C_3$ à $C_7$, phényle, phényle à substitution alkyle en $C_1$ à $C_6$ et phényle à substitution alkyle en $C_1$ à $C_6$ halogéné ; ou

$R^9$ est choisi parmi $CH_3$, cyclopropyle, phényle, p-méthylphényle, et p-trifluorométhylphényle.

**5.** Composé cyano, ou racémate, énantiomère, diastéréoisomère ou sel pharmaceutiquement acceptable de celui-ci selon l'une quelconque des revendications 1 à 4, **caractérisé en ce que,**

X est choisi parmi O, S, $S(=O)_2$ et S=O ; Y est absent ou choisi parmi O, S et S=O ; $R^4$ est choisi parmi alkyle en $C_1$ à $C_6$ et aryle en $C_6$ à $C_{10}$, $R^5$ est choisi parmi H ; ou, $R^4$ et $R^5$ sont reliés l'un à l'autre pour former alkylène en $C_2$ à $C_6$, en reliant de ce fait X et Y ; ou

X et Y sont chacun indépendamment choisis parmi O, S et S=O, et $R^4$ et $R^5$ sont reliés l'un à l'autre pour former $CH_2CH_2$ et $CH_2CH_2CH_2$, en reliant de ce fait X et Y ; ou

X et Y sont chacun indépendamment choisis parmi O et S, et $R^4$ et $R^5$ sont reliés l'un à l'autre pour former $CH_2CH_2$, en reliant de ce fait X et Y.

**6.** Composé cyano, ou racémate, énantiomère, diastéréoisomère ou sel pharmaceutiquement acceptable de celui-ci selon l'une quelconque des revendications 1 à 5, **caractérisé en ce que,**

le composé cyano représenté par la formule générale I est choisi parmi le composé cyano représenté par la formule générale IA :

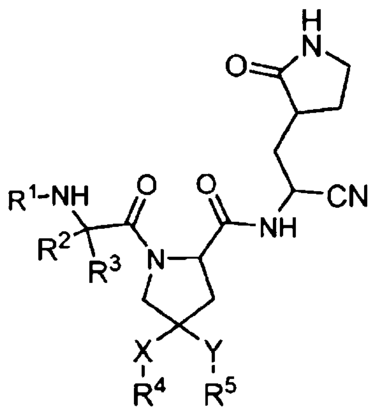

IA

dans laquelle $R^1$, $R^2$, $R^3$, $R^4$, $R^5$, X et Y sont définis comme décrit dans l'une quelconque des revendications 1 à 5.

7. Composé cyano, ou racémate, énantiomère, diastéréoisomère ou sel pharmaceutiquement acceptable de celui-ci selon l'une quelconque des revendications 1 à 5, **caractérisé en ce que,**

le composé cyano représenté par la formule générale I est choisi parmi le composé cyano représenté par la formule générale suivante :

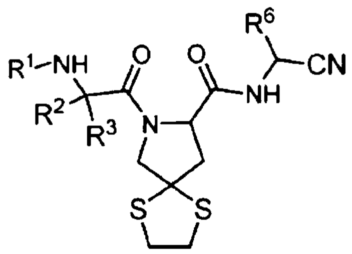

I-1

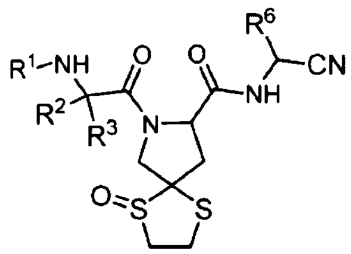

I-2

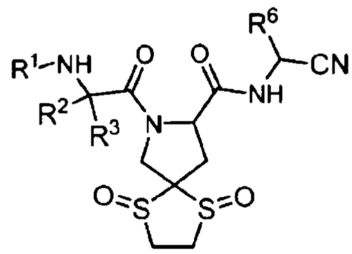

I-3

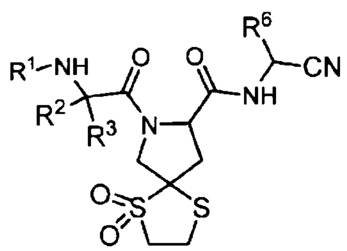

I-4

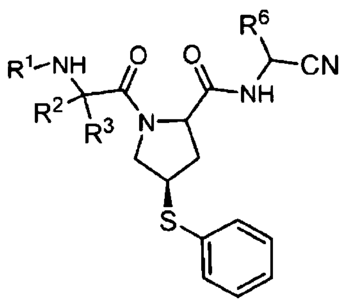

I-5

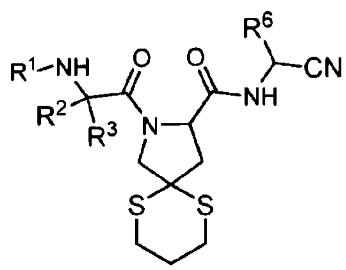

I-6

ou

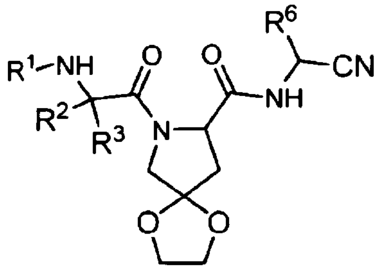

I-7

dans laquelle $R^1$, $R^2$, $R^3$ et $R^6$ sont définis comme décrit dans l'une quelconque des revendications 1 à 5.

**8.** Composé cyano, ou racémate, énantiomère, diastéréoisomère ou sel pharmaceutiquement acceptable de celui-ci selon la revendication 1, **caractérisé en ce que,**
le composé représenté par la formule générale I est choisi parmi les composés suivants :

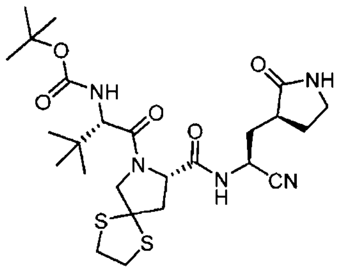

Composé 1

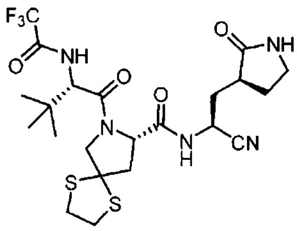

Composé 2

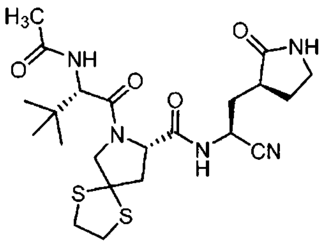

Composé 3

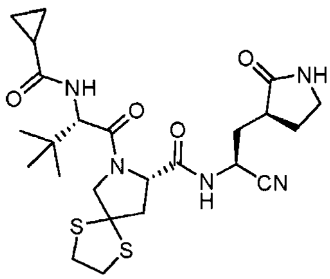

Composé 4

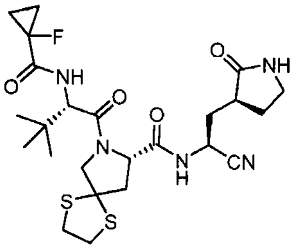

Composé 5

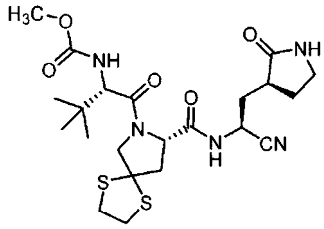

Composé 6

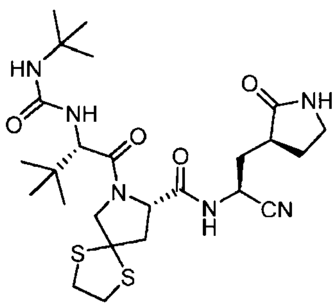

Composé 7

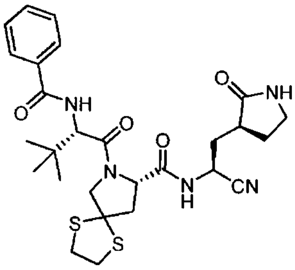

Composé 8

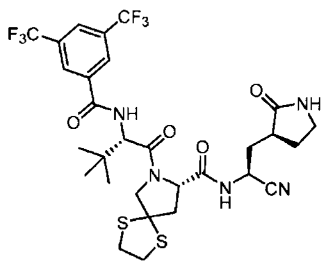

Composé 9

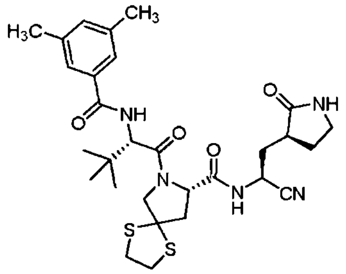

Composé 10

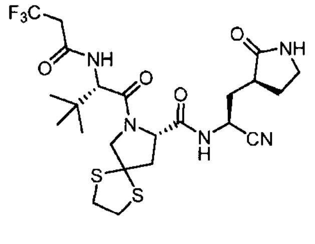

Composé 11

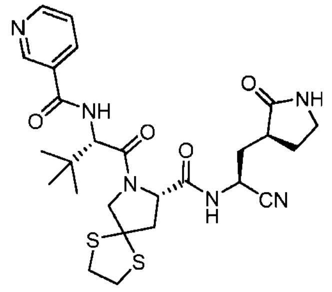

Composé 12

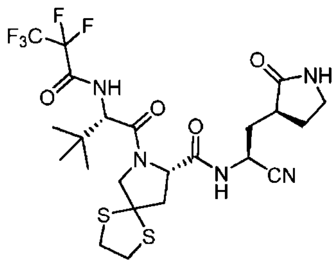

Composé 13

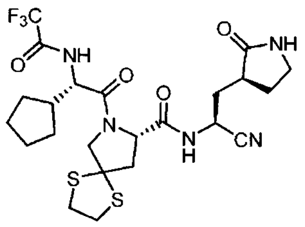

Composé 14

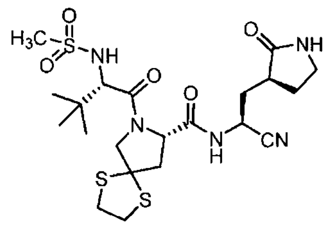

Composé 15

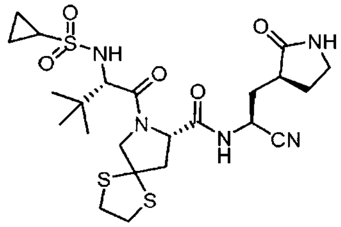

Composé 16

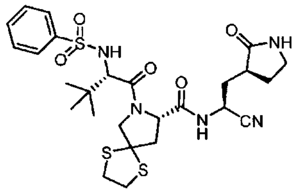

Composé 17

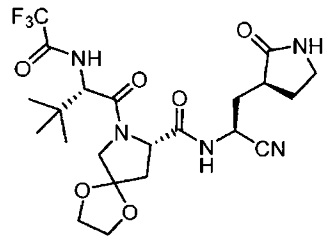

Composé 18

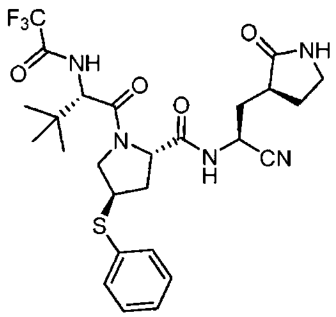

Composé 19

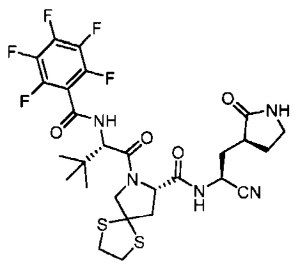

Composé 20

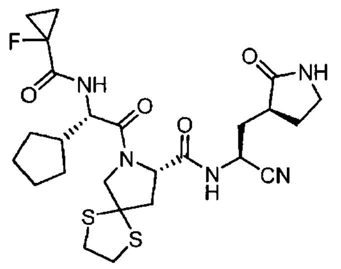

Composé 21

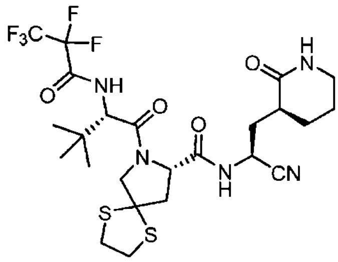

Composé 22

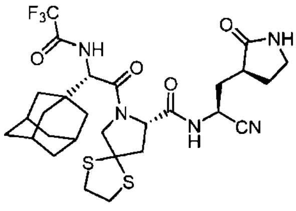

Composé 23

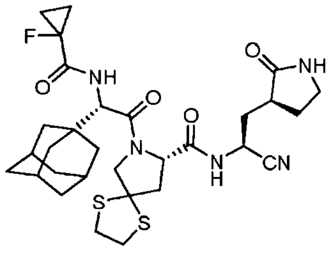

Composé 24

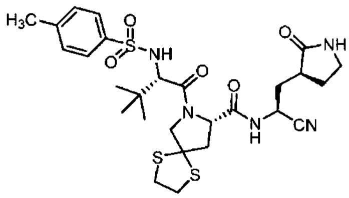

Composé 25

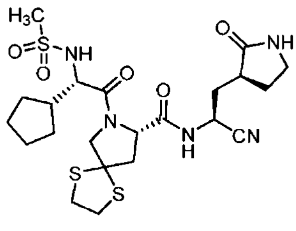

Composé 26

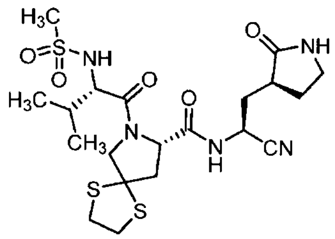

Composé 27

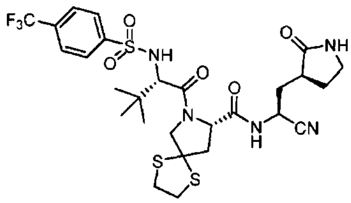

Composé 28

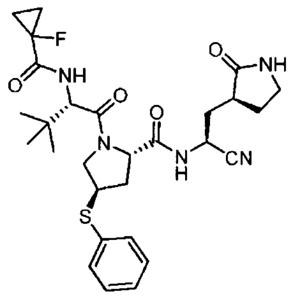

Composé 29

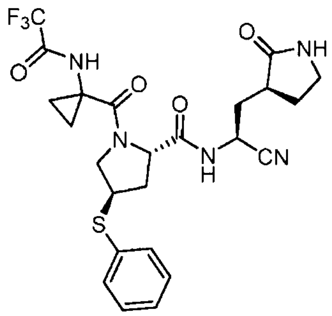

Composé 30

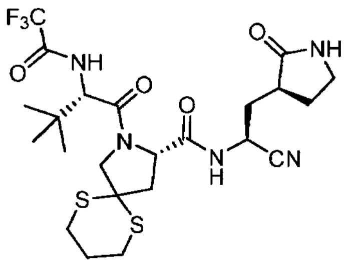

Composé 31

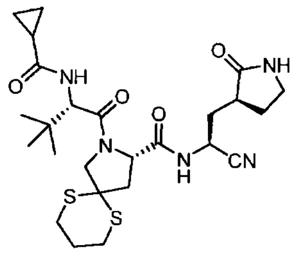

Composé 32

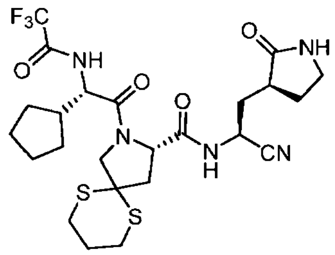

Composé 33

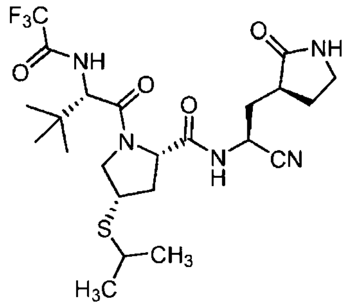

Composé 34

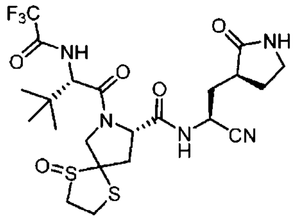

Composé 35

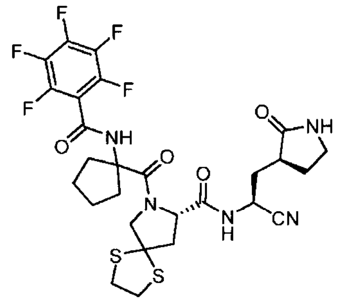

Composé 36

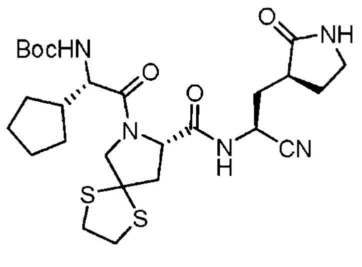

Composé 37

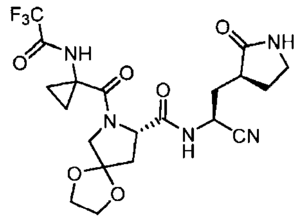

Composé 38

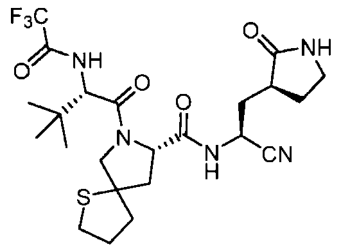

Composé 39

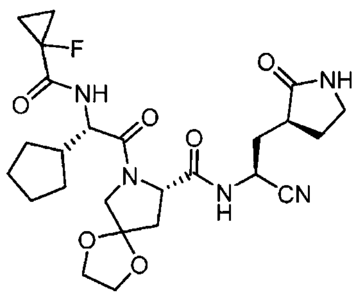

Composé 40

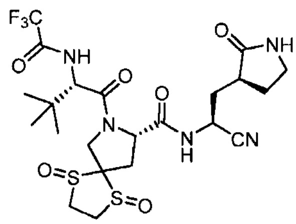

Composé 41

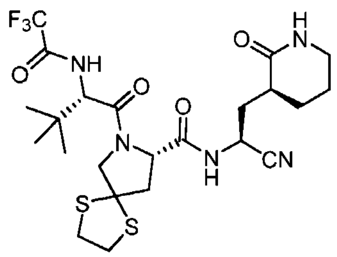

Composé 42

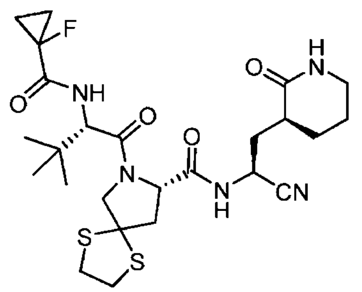

Composé 43

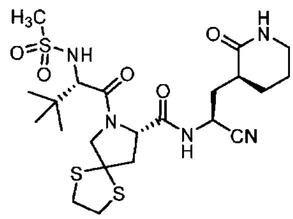

Composé 44

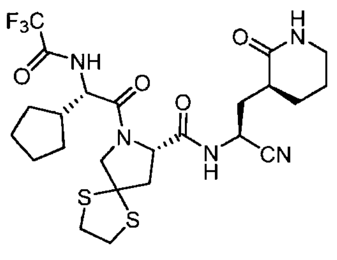

ou

Composé 45

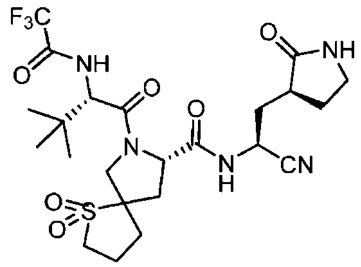

Composé 46

.

**9.** Procédé permettant de préparer le composé représenté par la formule générale I, **caractérisé en ce que** le procédé est l'un des procédés suivants :

Procédé i :

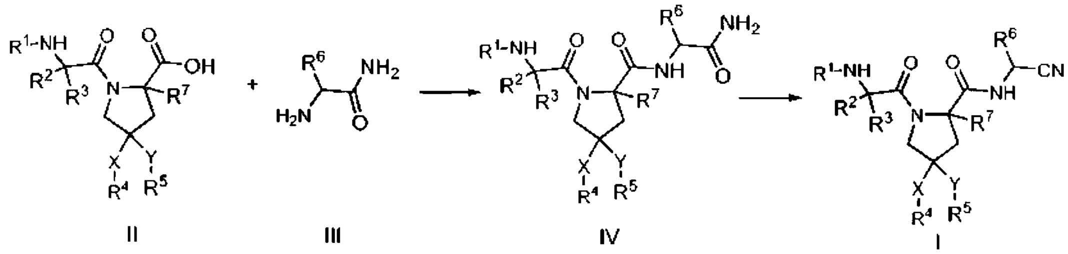

II III IV I

ia) un composé représenté par la formule IV est obtenu par l'intermédiaire d'une réaction de condensation d'un composé représenté par la formule II et d'un composé représenté par la formule III;

de préférence, l'étape ia) est que : le composé représenté par la formule II est mis en réaction avec le composé représenté par la formule III sous l'action d'un agent de condensation et d'une base dans un solvant à une température de - 20 °C à 50 °C pendant 0,1 à 12 h, en obtenant de ce fait le composé représenté par la formule IV,
facultativement, le solvant est l'un ou un mélange de plus d'un des suivants : tétrahydrofurane, dichlorométhane, N,N-diméthylformamide, N,N-diméthylacétamide, acétate d'éthyle et 1,4-dioxane,
facultativement, l'agent de condensation est l'un ou un mélange de plus d'un des suivants : hexafluorophosphate de 2-(7-azabenzotriazole)-N,N,N',N'-tétraméthyluronium, chlorhydrate de 1-éthyl-(3-diméthylaminopropyl)carbodiimide, 1-hydroxybenzotriazole, 2-hydroxypyridine-N-oxyde, anhydride 1-propyl-phosphorique, hexafluorophosphate de 1H-benzotriazol-1-yloxytripyrrolidinyle, N,N'-carbonyldiimidazole et hexafluorophosphate de O-benzotriazole-tétraméthyluronium ;
facultativement, la base est N,N-di-isopropyléthylamine, triéthylamine et N-méthylmorpholine ;

ib) le composé représenté par la formule IV est déshydraté pour obtenir le composé représenté par la formule générale I ;

de préférence, l'étape ib) est que : le composé représenté par la formule IV est mis en réaction avec un agent déshydratant dans un solvant anhydre à une température de -20 °C à 50 °C pendant 1 à 24 h pour obtenir le composé représenté par la formule générale I ;
facultativement, le solvant anhydre est l'un ou un mélange de plus d'un des suivants : tétrahydrofurane, dichlorométhane, toluène, 1,4-dioxane et pyridine ;
facultativement, l'agent déshydratant est anhydride trifluoroacétique ou N-(triéthylammoniumsulfonyl) carbamate de méthyle ; ou

Procédé ii :

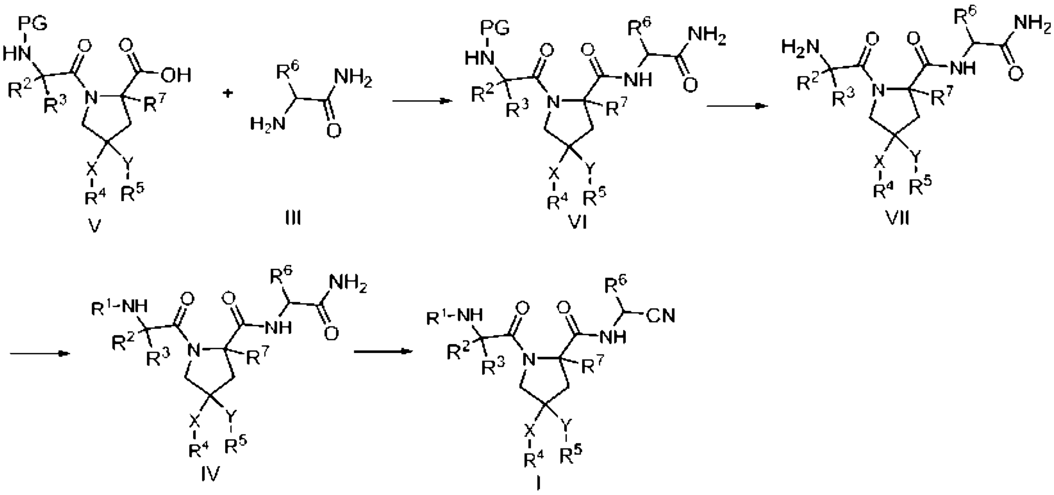

V III VI VII IV I

iia) un composé représenté par la formule VI est obtenu par l'intermédiaire d'une réaction de condensation d'un composé représenté par la formule V et du composé représenté par la formule III, dans lequel PG dans le composé représenté par la formule V est un groupe de protection d'amino ;

de préférence, l'étape iia) est que : le composé représenté par la formule V est mis en réaction avec le composé représenté par la formule III sous l'action d'un agent de condensation et d'une base dans un solvant à une température de -20 °C à 50 °C pendant 0,1 à 12 h, en obtenant de ce fait le composé représenté par la formule VI ;
facultativement, le solvant est l'un ou un mélange de plus d'un des suivants : tétrahydrofurane, dichlorométhane, N,N-diméthylformamide, N,N-diméthylacétamide, acétate d'éthyle et 1,4-dioxane,
facultativement, l'agent de condensation est l'un ou un mélange de plus d'un des suivants : hexafluorophosphate de 2-(7-azabenzotriazole)-N,N,N',N'-tétraméthyluronium, chlorhydrate de 1-éthyl-(3-diméthylaminopropyl)carbodiimide, 1-hydroxybenzotriazole, 2-hydroxypyridine-N-oxyde, anhydride 1-propyl-phosphorique, hexafluorophosphate de 1H-benzotriazol-1-yloxytripyrrolidinyle, N,N'-carbonyldiimidazole et hexafluorophosphate de O-benzotriazole-tétraméthyluronium ;
facultativement, la base est N,N-di-isopropyléthylamine, triéthylamine et N-méthylmorpholine ;
facultativement, le groupe de protection d'amino PG est tert-butoxycarbonyle, benzyle et p-méthoxybenzyle ;

iib) le composé représenté par la formule VI est déprotégé pour obtenir un composé représenté par la formule VII ;
de préférence, l'étape iib) est que : à une température de -20 °C à 50 °C, le composé représenté par la formule VI est mis en réaction avec une solution organique d'acide trifluoroacétique ou de chlorure d'hydrogène ou de $Pd/C/H_2$ pour obtenir le composé représenté par la formule VII ;
iic) le composé représenté par la formule IV est obtenu par aminoacylation, sulfonylation ou une réaction de condensation du composé représenté par la formule VII ;
de préférence, l'étape iic) est que :

à une température de -20 °C à 50 °C, le composé représenté par la formule VII est soumis à une réaction d'aminoacylation avec un chlorure d'acide ou un anhydride d'acide dans la condition d'ajout d'une base pour obtenir le composé représenté par la formule IV ;
à une température de -20 °C à 50 °C, le composé représenté par la formule VII est soumis à une réaction de sulfonylation avec un chlorure de sulfonyle ou un anhydride sulfonique dans la condition d'ajout d'une base pour obtenir le composé représenté par la formule IV ;
à une température de -20 °C à 50 °C, le composé représenté par la formule VII est soumis à une réaction de condensation avec un composé carboxyle dans la condition d'un agent de condensation et d'une base pour obtenir le composé représenté par la formule IV ;
facultativement, la base est N,N-di-isopropyléthylamine, triéthylamine et N-méthylmorpholine ;
facultativement, l'agent de condensation est l'un ou un mélange de plus d'un des suivants : hexafluorophosphate de 2-(7-azabenzotriazole)-N,N,N',N'-tétraméthyluronium, chlorhydrate de 1-éthyl-(3-diméthylaminopropyl)carbodiimide, 1-hydroxybenzotriazole, 2-hydroxypyridine-N-oxyde, anhydride 1-propyl-phosphorique, hexafluorophosphate de 1H-benzotriazol-1-yloxytripyrrolidinyle, N,N'-carbonyldiimidazole et hexafluorophosphate de O-benzotriazole-tétraméthyluronium ;

iid) le composé représenté par la formule IV est déshydraté pour obtenir le composé représenté par la formule générale I ;

de préférence, l'étape iid) est que : le composé représenté par la formule IV est mis en réaction avec un agent déshydratant dans un solvant anhydre à une température de -20 °C à 50 °C pendant 1 à 24 h pour obtenir le composé représenté par la formule générale I ;
facultativement, le solvant anhydre est l'un ou un mélange de plus d'un des suivants : tétrahydrofurane, dichlorométhane, toluène, 1,4-dioxane et pyridine ;
facultativement, l'agent déshydratant est anhydride trifluoroacétique ou N-(triéthylammoniumsulfonyl) carbamate de méthyle ;
dans laquelle $R^1$, $R^2$, $R^3$, $R^4$, $R^5$, $R^6$, $R^7$, X et Y sont tels que définis comme décrit dans l'une quelconque des revendications 1 à 8.

**10.** Composition pharmaceutique, **caractérisée en ce que** la composition pharmaceutique comprend le composé cyano, ou le racémate, l'énantiomère, le diastéréoisomère ou le sel pharmaceutiquement acceptable de celui-ci selon l'une quelconque des revendications 1 à 8, et un excipient pharmaceutiquement acceptable ; facultativement, la composition pharmaceutique comprend en outre du ritonavir ou un sel pharmaceutiquement acceptable de celui-ci.

**11.** Combinaison pharmaceutique, **caractérisée en ce que** la composition pharmaceutique comprend le composé cyano, ou le racémate, l'énantiomère, le diastéréoisomère ou le sel pharmaceutiquement acceptable de celui-ci selon l'une quelconque des revendications 1 à 8, et du ritonavir ou un sel pharmaceutiquement acceptable de celui-ci.

**12.** Composé cyano, ou racémate, énantiomère, diastéréoisomère ou sel pharmaceutiquement acceptable de celui-ci selon l'une quelconque des revendications 1 à 8 pour une utilisation en tant que médicament choisi parmi un médicament permettant d'inhiber une activité de protéase 3CL de coronavirus, un médicament permettant de prévenir et/ou de traiter une infection par coronavirus, un médicament permettant d'inhiber une activité de protéase 3CL de picornavirus, et un médicament permettant de prévenir et/ou de traiter une infection par picornavirus ; facultativement, le coronavirus est choisi parmi SARS-CoV, MERS-CoV, H229E-CoV, HKU1-CoV, NL63-CoV, OC43-CoV et SARS-CoV-2.

**13.** Composition pharmaceutique selon la revendication 10 pour une utilisation en tant que médicament choisi parmi un médicament permettant d'inhiber une activité de protéase 3CL de coronavirus, un médicament permettant de prévenir et/ou de traiter une infection par coronavirus, un médicament permettant d'inhiber une activité de protéase 3CL de picornavirus et un médicament permettant de prévenir et/ou de traiter une infection par picornavirus ; facultativement, le coronavirus est choisi parmi SARS-CoV, MERS-CoV, H229E-CoV, HKU1-CoV, NL63-CoV, OC43-CoV et SARS-CoV-2.

**14.** Combinaison pharmaceutique selon la revendication 11 pour une utilisation en tant que médicament choisi parmi un médicament permettant d'inhiber une activité de protéase 3CL de coronavirus, un médicament permettant de prévenir et/ou de traiter une infection par coronavirus, un médicament permettant d'inhiber une activité de protéase 3CL de picornavirus et un médicament permettant de prévenir et/ou de traiter une infection par picornavirus ; facultativement, le coronavirus est choisi parmi SARS-CoV, MERS-CoV, H229E-CoV, HKU1-CoV, NL63-CoV, OC43-CoV et SARS-CoV-2.

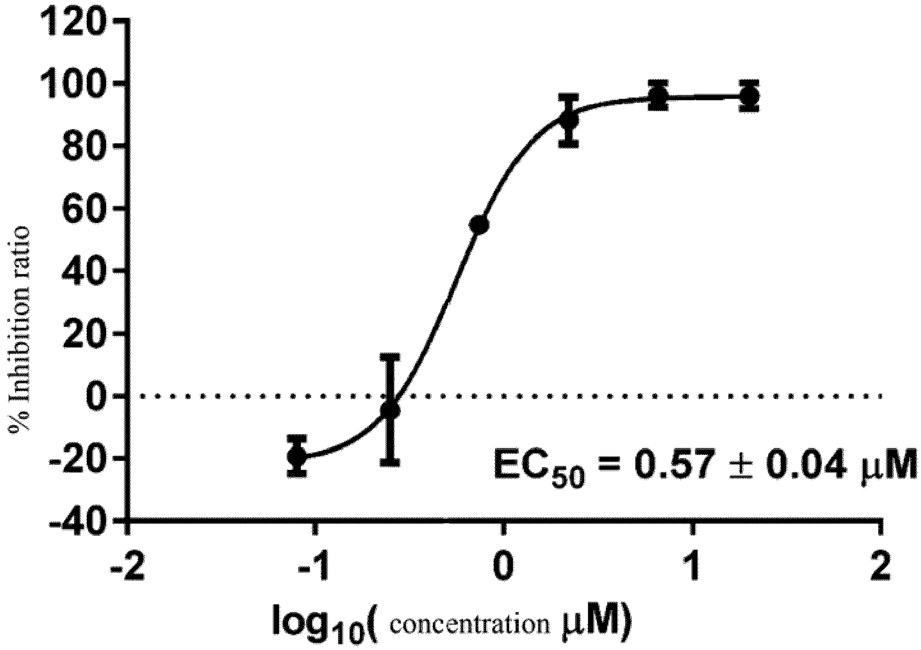
120
100
80
60
40
20
0
-20
-40
% Inhibition ratio
-2
-1
0
1
2
log10( concentration μM)
EC50 = 0.57 ± 0.04 μM

*FIG. 1*

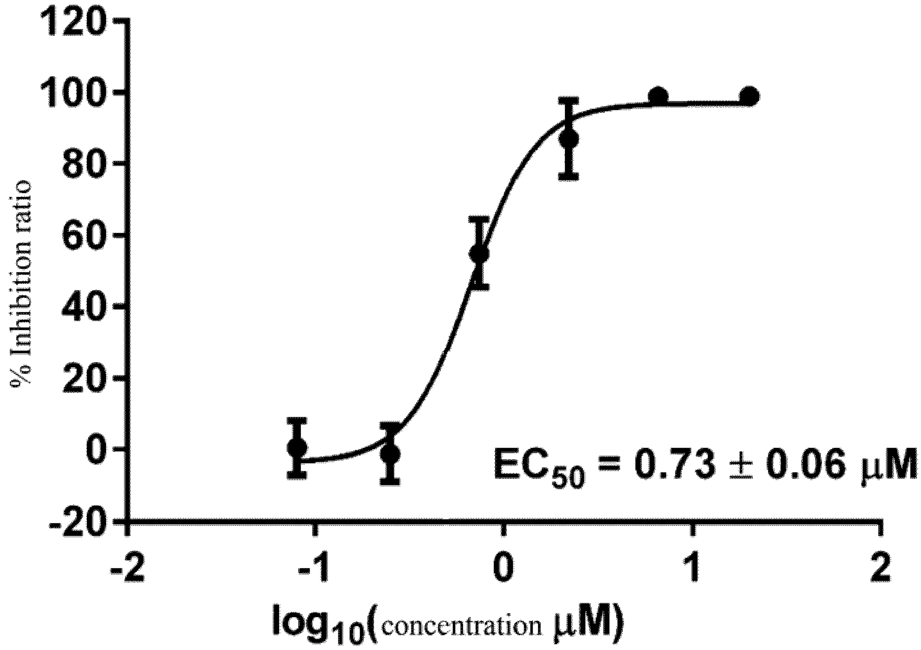
120
100
80
60
40
20
0
-20
% Inhibition ratio
-2
-1
0
1
2
log10(concentration μM)
EC50 = 0.73 ± 0.06 μM

*FIG. 2*

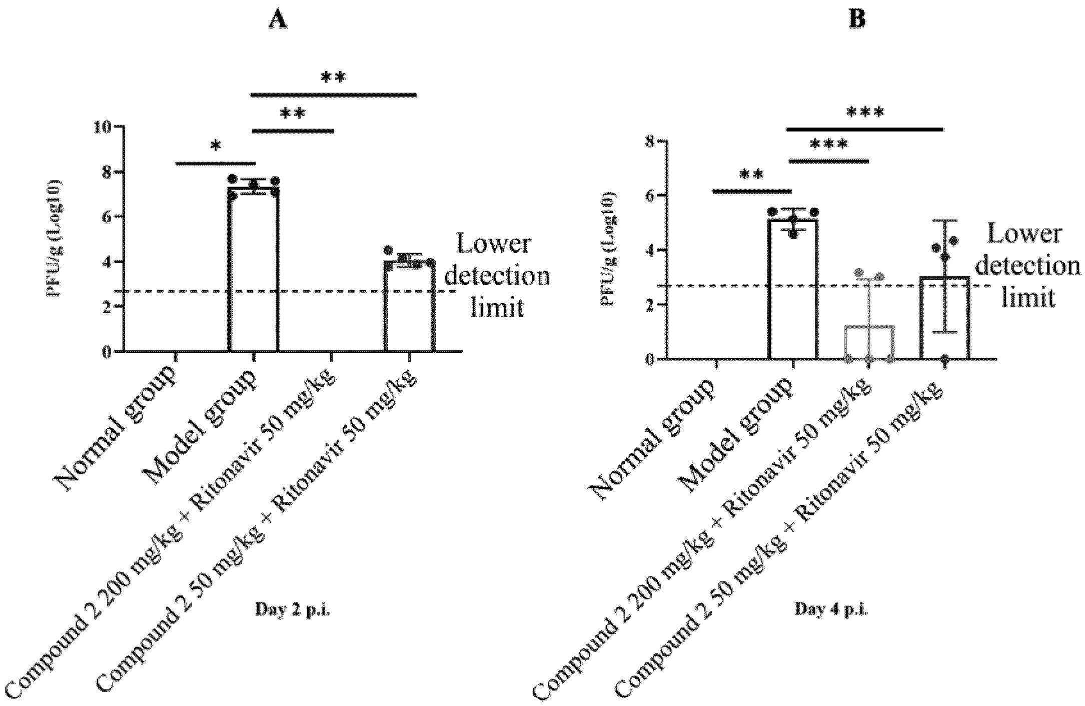
A
B
PFU/g (Log10)
Lower detection limit
Normal group
Model group
Compound 2 200 mg/kg + Ritonavir 50 mg/kg
Compound 2 50 mg/kg + Ritonavir 50 mg/kg
Day 2 p.i.
Day 4 p.i.

*FIG. 3*

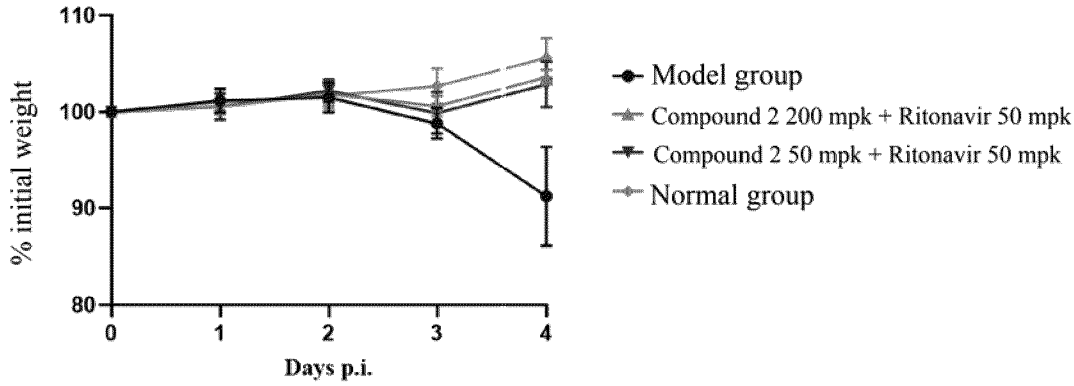
% initial weight
Days p.i.
Model group
Compound 2 200 mpk + Ritonavir 50 mpk
Compound 2 50 mpk + Ritonavir 50 mpk
Normal group

*FIG. 4*

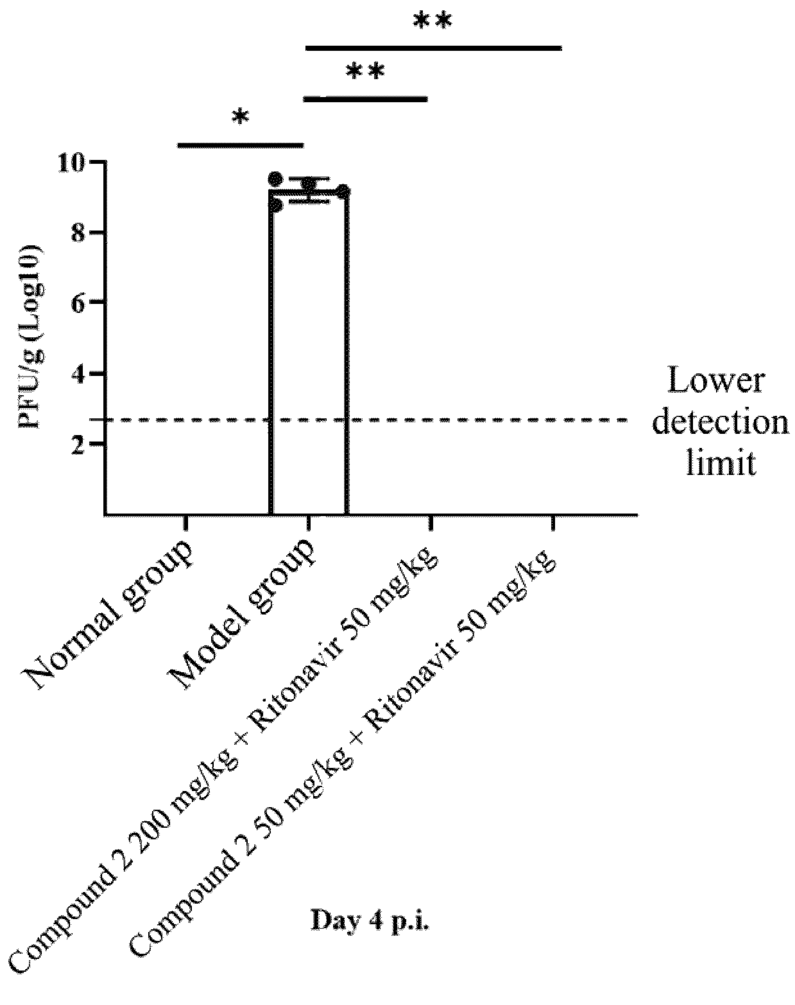
**
**
*
10
8
6
4
2
PFU/g (Log10)
Lower detection limit
Normal group
Model group
Compound 2 200 mg/kg + Ritonavir 50 mg/kg
Compound 2 50 mg/kg + Ritonavir 50 mg/kg
Day 4 p.i.

*FIG. 5*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111168232
- CN 202210973184